# EUROPEAN PATENT SPECIFICATION

(11) **EP 1 481 061 B1**
(45) Date of publication and mention of the grant of the patent: **15.06.2011**
(21) Application number: 02701532.0
(22) Date of filing: 05.03.2002
(51) Int. Cl.: C12N 15/29, C12N 15/82, C12N 15/87, C12N 5/04, C07H 21/02, C07H 21/04, A01H 1/00, A01H 5/00

(54) **DENATURANT STABLE AND/OR PROTEASE RESISTANT, CHAPERONE-LIKE OLIGOMERIC PROTEINS, POLYNUCLEOTIDES ENCODING SAME AND THEIR USES**
GEGEN DENATURIERUNGSMITTEL STABILE UND/ODER PROTEASERESISTENTE, CHAPERONÄHNLICHE OLIGOMERE PROTEINE, DIESE KODIERENDE POLYNUKLEOTIDE SOWIE IHRE VERWENDUNGEN
PROTEINES OLIGOMERES SEMBLABLES A UNE CHAPERONE, STABLES FACE AUX DENATURANTS ET/OU RESISTANT A LA PROTEASE, POLYNUCLEOTIDES CODANT LES MEMES PROTEINES ET UTILISATIONS CORRESPONDANTES

(30) Priority: 05.03.2001 US 272771 P
(43) Date of publication of application: 01.12.2004
(73) Proprietor: YISSUM RESEARCH DEVELOPMENT COMPANY OF THE HEBREW UNIVERSITY OF JERUSALEM, 91390 Jerusalem (IL)
(72) Inventor: WANG, Wangxia, 76301 Rechovot (IL); PELAH, Dan, 76281 Rechovot (IL); ALEGRAND, Tal, 70700 Gedera (IL); SHOSEYOV, Oded, 72910 Karme Yossef (IL); ALTMAN, Arie, 76452 Rechovot (IL)
(74) Representative: Machtalère, Georges
(86) International application number: PCT/IL2002/000174
(87) International publication number: WO 2002/070647

(56) References cited:
- EP-A2- 0 774 512
- PELAH D. ET AL.: "Characterization of BspA, a major boiling-stable, water-stress-responsive protein in aspen (Populus tremula)" TREE PHYSIOLOGY, vol. 15, no. 10, 1995, pages 673-678, XP009051172
- ALTMAN A. ET AL: "Molecular Biology of Drought Tolerance and Transformation of Populus and Pinus at the Hebrew University of Jerusalem" DENDROME, vol. 3, no. 2, December 1996 (1996-12), pages 1,5-7, XP002337629
- WANG W X ET AL: "Plant tolerance to water and salt stress: the expression pattern of a water stress responsive protein (BspA) in transgenic aspen plants" PLANT BIOTECHNOLOGY AND IN VITRO BIOLOGY IN THE 21ST CENTURY. PROCEEDINGS OF THE INTERNATIONAL CONGRESS OF THE INTERNATIONAL ASSOCIATION OF PLANT TISSUE CULTURE AND BIOTECHNOLOGY, 1999, pages 561-565, XP002904031
- SOTO A. ET AL: 'Heterologous Expression of a Plant Small Heat-Shock Protein Enhances Escherichiacoli Viability under Heat and Cold Stress' PLANT PHYSIOLOGY vol. 120, June 1999, pages 521 - 528, XP002979513
- LEE G.J. ET AL: 'Structure and in Vitro Molecular Chaperone Activity of Cytosolic Small Heat Shock Proteins from Pea' J. BIOLOGICAL CHEMISTRY vol. 270, no. 18, 05 May 1995, pages 10432 - 10438, XP002979514
- DATABASE GENBANK [Online] 30 November 2000 BRADSHAW H.D. ET AL: 'Populus x generosa pop3 peptide mRNA, complete cds', XP002979515 Retrieved from NCBI Database accession no. M18538

## Description

The present invention concerns a nucleic acid construct comprising a nucleic acid according to claim 1, a cell transformed with a nucleic acid construct according to claim 9, a non-human organism transformed with the nucleic acid construct according to claim 10, an antibody which specifically binds to an isolated boiling stable and detergent stable polypeptide according to claim 12, a method of stabilizing a protein against denaturing conditions according to claim 13, a fusion protein comprising a boiling stable and detergent stable polypeptide according to claim 14, a pharmaceutical composition according to claim 20 and claim 25, a method of protecting an enzyme preparation from reduction in enzymatic activity according to claim 21, a transgenic plant according to claim 22, a method of rendering a plant more tolerant to a biotic or abiotic stress according to claim 23, use of a boiling stable and detergent stable polypeptide according to claim 24, a method of increasing a binding avidity of a binding molecule according to claim 28, a hetero complex according to claim 32.

The present invention relates to denaturat (e.g., boiling, detergent, other denaturants) stable and/or protease resistant, chaperone-like oligomeric proteins, polynucleotides encoding same and uses thereof. More particularly, the present invention relates to novel denaturat-stable, protease resistant, homo-oligomeric proteins composed of homo-monomers, which proteins are referred to hereinbelow as stable proteins (SPs), methods of production and purification of SPs, nucleic acid constructs encoding SPs, antibodies recognizing SPs, the use of SPs for stabilizing, refolding and de-aggregating, in other words, chaperoning, macromolecules such as proteins, fusion proteins including SPs, nucleic acid constructs encoding these fusion proteins, their use in immunization and/or formation of homogenous or heterogeneous complex structures and other applications.

### Molecular chaperones:

Molecular chaperones are characterized by their remarkable ability to recognize selectively and bind unstable, non-natively organized (herein after non-native) proteins. The interactions of chaperones with such proteins addresses multiple (diverse) functions that are specific to different chaperones, and include: facilitating and promoting folding of nascent proteins to their final conformation, holding substrates in an unstructured form that is competent for membrane transport, maintaining proteins in specific conformations, preventing aggregation of unfolded proteins, and promoting renaturation of aggregated proteins. The last two functions are particularly important for cells experiencing high temperature and other stresses. It is therefore not surprising that many molecular chaperones were first identified as heat shock proteins (Hsps).

### Heat Shock Proteins (Hsps):

Hsps are a group of proteins found in all organisms exposed to stress temperatures. It has been clearly shown that many Hsps posses the activities of molecular chaperones that are involved in the proper folding of nascent polypeptides and help damaged proteins regain their biologically active conformation (Hartl, 1996). Small Hsps (sHsps) are Hsps having a molecular size ranging from 12-40 kDa in different organisms, and which are found abundantly in plants. Plant sHsp like other sHsp and alpha crystallins tend to form large oligomeric complexes that are believed to be their functional form (Chen et al., 1994; Lee et al., 1995; Collada et al., 1997). Suzuki et al. (1998) provided the evidence that chloroplast-localized Hsp21 from pea exists as a complex and does not dissociate during heat stress and recovery. In contrast to plant sHsps, mammalian cytosolic sHsps undergo complex dissociation to monomers by phosphorylation during heat stress (Rogalla et al., 1999). A recent paper by Haslbeck et al., (1999) demonstrated that the dissociation of Hsp26 complex from yeast is temperature-regulated and is a prerequisite for efficient chaperone activity. It has been shown that *in vitro*, sHsps bind to non-native proteins (Lee et al., 1995, Ehrnsperger et al., 1997, Veinger et al., 1998), therefore preventing the aggregation of non-native proteins, allowing subsequent refolding by chaperone network (Ehrnsperger et al., 1997, Veinger et al., 1998; Haslbeck et al. 1999).

In general, Hsps are stable at moderate temperatures but not at temperatures exceeding 80 °C. Accumulation of pea Hsp18.1 remains stable with a half life of 38 hours at 38 °C. At 55 °C, the effect of Hsp18.1 on preventing aggregation of heat denatured LDH was less than that at 45°C (Lee et al., 1995). Hsp25 oligomer was stable at 43 °C up to 60 minutes (Ehrnsperger et al., 1997). Exposure of Hsp21 to temperatures above 70 °C led to irreversible aggregation (Hrndahl et al. 1999). The only report of a highly heat stable Hsp is HSP 12 from yeast (Praekelt and Meacock, 1990). Based on its physico-chemical properties and similarity of amino acid composition, Mtwisha et al. (1998) suggested that HSP 12 is a LEA-like protein. It has not been reported that any oligomeric complex of sHsps is stable under SDS denaturation. All the reported sHsps are verified as monomer in SDS-PAGE, unless the protein has been cross-linked.

### Uses of Hsp and chiaperone-like molecules:

The unique ability of stress proteins to stabilize protein and peptide structures has been employed to modify the antigenicity of peptides, to protect cells from oxidative and thermal stress, to alter protein aggregation and to promote *in vitro* protein folding.

The ability of Hsps to effect the conformation of antigens has led to a number of proposed applications, including the incorporation of Hsp 70, Hsp 90 and gp 76 into vaccinations using non-antigenic tumor antigens (U.S. Patent No. 6,162,436 to Srivastava), for eliciting immunity to agents of infectious disease (U.S. Patent No. 6,139,841 to Srivastava) and the suppression of allograft and xenograft rejection through modulation of tissue graft immune response (U.S. Patent No. 5,891,653 to Attfield).

High level expression of cloned DNA sequences encoding Hsps has been employed to confer novel stress resistance in the transformed cells. Overexpressed human Hsp 27 protected transformed 1929 and 13.S. 1.24 cells from oxidative stress (Rogalla, T., et al. JBC (1999) 274, 18947-56). The plant-derived sHsp Cs Hsp 17.5 (from chestnut cotyledons), when overexpressed in transformed *E. coli,* protected the bacteria against extremes of cold (4 °C) and heat (50 °C) (Soto-A, et al. Plant Physiology (1999) 120, 521-528).

Alpha-beta lens crystallin is also considered a sHsp protein. When a DNA sequence encoding the crystallin protein was expressed in cells prone to amyloid aggregate formation, the sHsp prevented *in vitro* fibril formation. However, this de-aggregation increased rather than decrease the toxicity of the amyloid beta protein. (Stege, G. J. et al., Biochem. and Biophys. Res. Comm. (1999) 262 (1): 152- 6).

Scaffolding proteins have been successfully employed in the *in-vitro* assembly of viral capsid proteins (Newcomb, WW. et al., Journal of Virology (1999) 73, 4231-50); to promote accurate protein folding *in-vitro* and in heterologous expression systems (see, U.S. Patent No. 5,561,221 to Yoshida et al.) and to promote immunological response by displaying a plurality of antigens on the same particle (Gonzalo et al. J. Mol. Biol (2001) 305, 259-267.

None of the known Hsp or sHsp, however, is stable under harsh denaturing conditions such as boiling or exposure to high SDS concentration or is resistant to proteolytic cleavage.

### Boiling-stable proteins from plants:

Pelah et al. (1995) teaches an attempt of purifying a boiling stable protein from water-stressed aspen shoots. A boiling-stable proteins extract was separated on a 10 % SDS-PAGE, yielding a dominant band having a 66 kDa molecular mass. When micro-sequenced, the N-terminal sequence of the 66 kDa protein exhibited high homology with wheat germins GF-2.8 and GF-3.8. Germins and germin-like proteins are ubiquitous, water-soluble, homo-oligomeric extracellular glycoproteins, exhibiting extreme thermal-, pH- and detergent-stability and protease resistance, and having oxalate oxidase activity, however they lack any chaperone-like activity.

EP 774512 relates to an expression cassette which can express a soluble form of a desired protein in a bacterial cell, wherein the cassette comprises a sequence in which a gene encoding a molecular chaperon is operably linked to a first promoter and a site to which a gene encoding the desired protein can be inserted. Also, a method for expressing a desired protein in a soluble form is provided by the use of the expression cassette or co-transformation with a plasmid which can express a molecular chaperon and a plasmid which can express the desired protein.

Dendrome, Arie Altman, December 1996, volume 3, number 2 concerns molecular biology of drought tolerance and transformation of populus and pinus.

The nucleic acid construct comprising a nucleic acid of the present invention is defined in claim 1, the cell transformed with a nucleic acid construct of the present invention is defined in claim 9, the non-human organism transformed with the nucleic acid construct of the present invention is defined in claim 10, the antibody which specifically binds to an isolated boiling stable and detergent stable polypeptide of the present invention is defined in claim 12, the method of stabilizing a protein against denaturing conditions of the present invention is defined in claim 13, the fusion protein comprising a boiling stable and detergent stable polypeptide of the present invention is defined in claim 14, the pharmaceutical composition of the present invention is defined in claim 20 and claim 25, the method of protecting an enzyme preparation from reduction in enzymatic activity of the present invention is defined in claim 21, the transgenic plant of the present invention is defined in claim 22, the method of rendering a plant more tolerant to a biotic or abiotic stress of the present invention is defined in claim 23, the use of a boiling stable and detergent stable polypeptide of the present invention is defined in claim 24, the method of increasing a binding avidity of a binding molecule of the present invention is defined in claim 28, the hetero complex of the present invention is defined in claim 32.

According to one aspect of the present invention there is provided an isolated nucleic acid comprising a first polynucleotide encoding a denaturant (e.g., boiling and/or detergent) stable and/or protease resistant protein (herein, stable protein, SP), the stable protein having a chaperone-like activity; and a second polynucleotide including a promoter sequence being operably linked to the first polynucleotide for directing an expression of the stable protein.

According to a further feature in preferred embodiments described below, the promoter sequence is a eukaryote promoter.

According to a still further feature in the described preferred embodiments the eukaryote promoter is a constitutive promoter.

According to a yet further feature in the described preferred embodiments the promoter is a plant promoter, such as a constitutive plant promoter, a tissue specific plant promoter and an inducible plant promoter.

According to a yet further feature in the described preferred embodiments (i) the constitutive plant promoter is selected from the group consisting of CaMV35S plant promoter, CaMV19S plant promoter, FMV34S plant promoter, sugarcane bacilliform badnavirus plant promoter, CsVMV plant promoter, *Arabidopsis* ACT2/ACT8 actin plant promoter, *Arabidopsis* ubiquitin UBQ1 plant promoter, barley leaf thionin BTH6 plant promoter, and rice actin plant promoter; (ii) the tissue specific plant promoter is selected from the group consisting of bean phaseolin storage protein plant promoter, DLEC plant promoter, PHSβ plant promoter, zein storage protein plant promoter, conglutin gamma plant promoter from soybean, AT2S1 gene plant promoter, ACT11 actin plant promoter from *Arabidopsis,* napA plant promoter from *Brassica napus* and potato patatin gene plant promoter; and (iii) the inducible plant promoter is selected from the group consisting of a light-inducible plant promoter derived from the pea rbcS gene, a plant promoter from the alfalfa rbcS gene, DRE, MYC and MYB plant promoters which are active in drought; INT, INPS, prxEa, Ha hsp17.7G4 and RD21 plant promoters active in high salinity and osmotic stress, and hsr203J and str246C plant promoters active in pathogenic stress.

According to a yet further feature in preferred embodiments the promoter sequence is a prokaryotic promoter.

According to further features in preferred embodiments described below, the first polynucleotide has a sequence at least 60 % identical with SEQ ID NOs:1, 5, 6, 34, 39 or 40, as determined using the BestFit software of the Wisconsin sequence analysis package, utilizing the Smith and Waterman algorithm, where gap weight equals 50, length weight equals 3, average match equals 10 and average mismatch equals -9.

According to still further features in the described preferred embodiments the stable protein has a sequence at least 60 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

According to still further features in the described preferred embodiments the stable protein is natively an oligomer.

According to still further features in the described preferred embodiments the chaperone-like activity includes heat stabilization of proteins.

According to still further features in the described preferred embodiments the isolated nucleic acid further comprising a third polynucleotide encoding an additional protein, the third polynucleotide being adjacent and in frame, either at the 5' or the 3', to the first polynucleotide, the first and third polynucleotides encoding, in combination, a fusion protein of the stable protein and the additional protein, wherein the additional protein may be positioned C or N terminally to the stable protein and the fusion protein may also include a spacer peptide of, say 1-100 amino acids between the stable protein and the additional protein.

According to another aspect there is provided a nucleic acid construct comprising the nucleic acid described herein.

According to yet another aspect there is provided a cell or organism transformed with the nucleic acid described herein.

According to still another aspect there is provided a method of isolating a gene encoding a stable protein having chaperone-like activity from a biological source, the method comprising screening an expression library with the polynucleotide described herein or a portion thereof of at least 20, preferably at least 30, more preferably at least 50, still preferably at least 100 contiguous bases.

According to an additional aspect there is provided a denaturant (e.g., boiling and/or detergent) stable and/or protease resistant polypeptide having a chaperone-like activity effective, for example, in stabilizing proteins.

According to further features in preferred embodiments described below, the polypeptide is encoded by a polynucleotide as described herein.

According to still further features in the described preferred embodiments the polypeptide has a sequence at least 60 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

According to still further features in the described preferred embodiments the polypeptide is natively an oligomer, preferably a homo-oligomer of at least 10 subunits.

According to yet an additional aspect there is provided an antibody, either polyclonal or monoclonal antibody, recognizing at least one epitope of the polypeptide described herein.

According to still an additional aspect there is provided a method of preventing an aggregating protein from aggregating into an aggregate comprising causing an effective amount of the polypeptide described herein to become in contact with the aggregating protein.

According to a further aspect there is provided a method of de-aggregating aggregates of an aggregating protein comprising causing an effective amount of the polypeptide described herein to become in contact with the aggregate.

Hence, the present invention provides a method of treating a disease associated with protein aggregation of an aggregating protein, the method comprising administering to a subject in need thereof a denaturant stable and/or protease resistant protein, the denaturant stable and/or protease resistant protein having a chaperone-like activity, in an amount sufficient for de-aggregating and/or preventing aggregation of the aggregating protein, the aggregating protein is, for example, beta-amyloid or prion.

According to yet a further aspect there is provided a method of stabilizing a protein against denaturing conditions comprising causing an effective amount of the polypeptide described herein to become in contact with the protein.

According to still a further aspect there is provided a method of enriching or isolating a denaturant (e.g., boiling and/or detergent) stable and/or protease resistant protein having chaperone-like activity from a biological source, the method comprising (a) extracting total proteins from the biological source, so as to obtain a proteins extract; (b) boiling the proteins extract; (c) collecting soluble proteins; and optionally (d) assaying for chaperone-like activity of the soluble proteins and enriching or isolating the stable protein having the chaperone-like activity. Preferably, the method further comprises size fractionating the soluble proteins.

According to another aspect there is provided a method of isolating a gene encoding a denaturant (e.g., boiling and/or detergent) stable, and/or protease resistant protein having chaperone-like activity from a biological source, the method comprising screening an expression library with a polynucleotide encoding a polypeptide as herein described.

According to yet another aspect there is provided a method of isolating a gene encoding a denaturant (e.g., boiling and/or detergent) stable and/or protease resistant protein having chaperone-like activity from a biological source, the method comprising (a) extracting total proteins from the biological source, so as to obtain a proteins extract; (b) boiling the proteins extract; (c) collecting soluble proteins; (d) assaying for chaperone-like activity of the soluble proteins and isolating a stable protein having chaperone-like activity; (e) raising antibodies recognizing the stable protein having the chaperone-like activity; and (f) screening an expression library with the antibodies.

According to yet another aspect there is provided a method of isolating a gene encoding a denaturant (e.g., boiling and/or detergent) stable and/or protease resistant protein having chaperone-like activity from a biological source, the method comprising (a) extracting total proteins from the biological source, so as to obtain a proteins extract; (b) boiling the proteins extract; (c) collecting soluble proteins; (d) assaying for chaperone-like activity of the soluble proteins and enriching or isolating a stable protein having chaperone-like activity; (e) microsequencing the stable protein so as to obtain at least a partial amino acid sequence thereof; (f) designing an oligonucleotide corresponding to the amino acid sequence; and (g) screening a library with the oligonucleotide.

According to a further aspect there is provided a method of isolating a nucleic acid potentially encoding a denaturant (e.g., boiling and/or detergent) stable and/or protease resistant protein having chaperone-like activity, the method comprising screening a cDNA or genomic library with a polynucleotide of at least 17 bases at least 60 % identical to a contiguous portion of SEQ ID NOs:1, 5, 6, 34, 39 or 40.

According to yet a further aspect there is provided a method of identifying a nucleic acid potentially encoding a denaturant (e.g., boiling and/or detergent) stable and/or protease resistant protein having chaperone-like activity, the method comprising searching an electronic library containing a plurality of nucleic acid and/or amino acid sequences for sequences having a predetermined degree of identity or homology to any of SEQ ID NOs:1, 2, 5-35 or 39-40 or portions thereof of, or corresponding to, at least 15 bases.

According to still another aspect there is provided a method of isolating a nucleic acid potentially encoding a denaturant (e.g., boiling and/or detergent) stable and/or protease resistant protein having chaperone-like activity, the method comprising (a) providing at least one pair of oligonucleotides each being at least 15 bases in length, the at least one pair of oligonucleotides including at least one oligonucleotide corresponding to SEQ ID NOs:1, 2, 5-35 or 39-40, the at least one pair of oligonucleotides being selected for amplifying a nucleic acid having a degree of identity with, or encoding proteins homologous, to SEQ ID NOs:1, 2, 5-35 or 39-40; (b) contacting the at least one pair of oligonucleotides with a sample of nucleic acid and amplifying the nucleic acid having the degree of identity with, or encoding proteins homologous to, SEQ ID NOs:1, 2, 5-35 or 39-40; and (c) using the nucleic acid having the degree of identity with or encoding proteins homologous to SEQ ID NOs:1, 2, 5-35 or 39-40 for isolating a nucleic acid potentially encoding a denaturant (e.g., boiling and/or detergent) stable and/or protease resistant protein.

According to still another aspect there is provided a method of detergent-free isolation of a protease-resistant protein having chaperone-like activity from a biological source, the method comprising (a) extracting total proteins from the biological source, so as to obtain a proteins extract; (b) contacting the protein extract with a protease; (c) isolating a protease-resistant protein; and optionally (d) assaying the protease-resistant protein for chaperone-like activity.

According to another aspect there is provided a fusion protein comprising a denaturant (e.g., boiling and/or detergent) stable and/or protease resistant polypeptide having a chaperone-like activity fused to an additional polypeptide, preferably the fusion protein acquires an oligomeric form.

In one embodiment, the denaturant stable and/or protease resistant polypeptide having the chaperone-like activity is fused to the additional polypeptide via a peptide bond. In another embodiment, the denaturant stable and/or protease resistant polypeptide having the chaperone-like activity is fused to the additional polypeptide via a cross-linker.

According to yet an additional aspect there is provided a method of immunization comprising subjecting an immune system of a mammal to the fusion protein described herein.

According to another aspect there is provided a method of protecting an enzyme preparation from reduction in enzymatic activity, the method comprising adding to the enzyme preparation a denaturant stable and/or protease resistant protein, the denaturant stable and/or protease resistant protein having a chaperone-like activity, in an amount sufficient for protecting the enzyme preparation from reduction in enzymatic activity.

According to yet another aspect there is provided a method of repairing at least a portion of lost enzymatic activity of an enzyme preparation, the method comprising adding to the enzyme preparation a denaturant stable and/or protease resistant protein, the denaturant stable and/or protease resistant protein having a chaperone-like activity, in an amount sufficient for repairing at least the portion of the lost enzymatic activity of the enzyme preparation.

According to still another aspect there is provided a method of administering to an animal having an immune system a polypeptide, while reducing an immune response against the polypeptide, the method comprising administering the polypeptide to the animal, the polypeptide being fused to a denaturant stable and/or protease resistant protein, the denaturant stable and/or protease resistant protein having a chaperone-like activity, thereby reducing the immune response against the polypeptide, as compared to an immune response that develops by administering to the animal the polypeptide alone.

According to an additional aspect there is provided a transgenic plant expressing a denaturant stable and/or protease resistant protein, the denaturant stable and/or protease resistant protein having a chaperone-like activity above a natural amount of the denaturant stable and/or protease resistant protein having the chaperone-like activity in the plant.

According to yet an additional aspect there is provided a method of rendering a plant more tolerant to a biotic or abiotic stress, the method comprising engineering the plant to express a denaturant stable and/or protease resistant protein, the denaturant stable and/or protease resistant protein having a chaperone-like activity, above a natural amount of the denaturant stable and/or protease resistant protein having the chaperone-like activity in the plant.

According to still an additional aspect there is provided a method of rendering a plant more recoverable from a biotic or abiotic stress, the method comprising engineering the plant to express a denaturant stable and/or protease resistant protein, the denaturant stable and/or protease resistant protein having a chaperone-like activity, above a natural amount of the denaturant stable and/or protease resistant protein having the chaperone-like activity in the plant.

According to a further aspect there is provided a method of increasing cell migration, the method comprising exposing the cells to an amount of a denaturant stable and/or protease resistant protein, the denaturant stable and/or protease resistant protein having a chaperone-like activity, sufficient for increasing cell migration.

According to yet a further aspect there is provided a method of accelerating wound healing, the method comprising administering onto a wound an amount of a denaturant stable and/or protease resistant protein, the denaturant stable and/or protease resistant protein having a chaperone-like activity, sufficient for accelerating wound healing.

According to still a further aspect there is provided a method of inducing wound healing, the method comprising administering onto a wound an amount of a denaturant stable and/or protease resistant protein, the denaturant stable and/or protease resistant protein having a chaperone-like activity, sufficient for inducing wound healing.

According to another aspect there is provided a method of strengthening and/or grooming hair, nail or skin, the method comprising administering onto the hair, nail or skin an amount of a denaturant stable and/or protease resistant protein, the denaturant stable and/or protease resistant protein having a chaperone-like activity, sufficient for strengthening and/or grooming the hair, nail or skin.

According to yet another aspect there is provided a pharmaceutical composition, comprising, as an active ingredient, a denaturant stable and/or protease resistant protein, the denaturant stable and/or protease resistant protein having a chaperone-like activity, and a pharmaceutically acceptable carrier.

According to further features in preferred embodiments described below, the pharmaceutical composition is packaged in a package and identified in print for use in a wound healing application.

According to still further features in the described preferred embodiments the pharmaceutical composition is packaged in a package and identified in print for use in a strengthening and/or grooming hair, nail or skin application.

According to still another aspect there is provided a method of isolating a boiling stable protein having chaperone-like activity from a biological source, the method comprising: (a) extracting total proteins from the biological source, so as to obtain a proteins extract; (b) boiling the protein extract; (c) recovering soluble protein fraction; and optionally; (d) assaying the protease-resistant protein for chaperone-like activity.

According to further features in preferred embodiments described below, the method further comprising digesting the protein extract with a protease.

According to another aspect there is provided a method of increasing a binding avidity of a binding molecule, the method comprising displaying multiple copies of the binding molecule on a surface of an oligomer of a denaturant stable and/or protease resistant protein, the denaturant stable and/or protease resistant protein having a chaperone-like activity. The binding molecule, can be, for example, a receptor, a ligand, an enzyme, a substrate, an inhibitor, an antibody and an antigen. In cases where the binding molecule is a binding protein, the binding protein can be fused to the oligomer units via either genetic engeneering techniques or chemical cross linking. In cases where the binding molecule is not a protein, the binding molecule can be fused or linked to the oligomer units via chemical cross linking techniques.

The present invention also provides a hetero complex comprising an oligomer including a plurality of a denaturant stable and/or protease resistant protein, the denaturant stable and/or protease resistant protein having a chaperone-like activity, and at least two different molecules which are fused to the oligomer. The at least two different molecules may comprise at least a first enzyme and a second enzyme. The first enzyme and the second enzyme may catalyze sequential reactions in a synthesis or degradation pathway. The first enzyme and the second enzyme may catalyze different reactions in a synthesis or degradation pathway. In another embodiment, the at least two different molecules comprise at least a binding molecule and a reporter molecule.

The present invention successfully addresses the shortcomings of the presently known configurations by providing a denaturant (e.g., boiling and/or detergent) stable and/or protease resistant protein and describing its uses.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

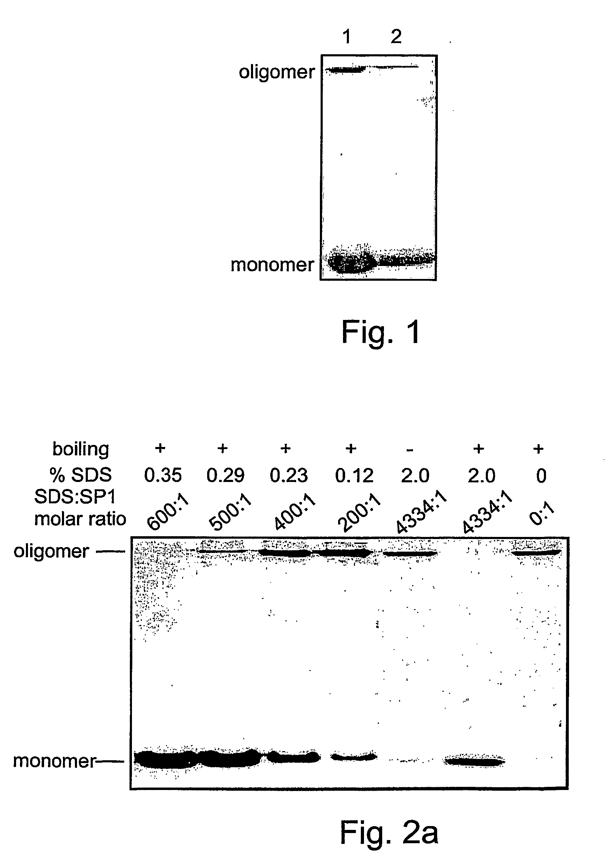

FIG. 1 demonstrates the separation on SDS-tricine PAGE of the purified 12.4 kDa SP1 monomer and the 116 kDa oligomer fractions. 12.4 and 116 kDa fractions of SP1 proteins were excised from corresponding bands in 17 % SDS-tricine-PAGE of boiling-stable aspen extracts. The proteins were then electro-eluted. Samples of the electro-eluted proteins were prepared in 2 X SDS sample buffer, boiled for 5 minutes and loaded on a 17 % polyacrylamide SDS-tricine gel. Lane 1: 12.4 kDa SP1 fraction. Lane 2: 116 kDa SP1 fraction.

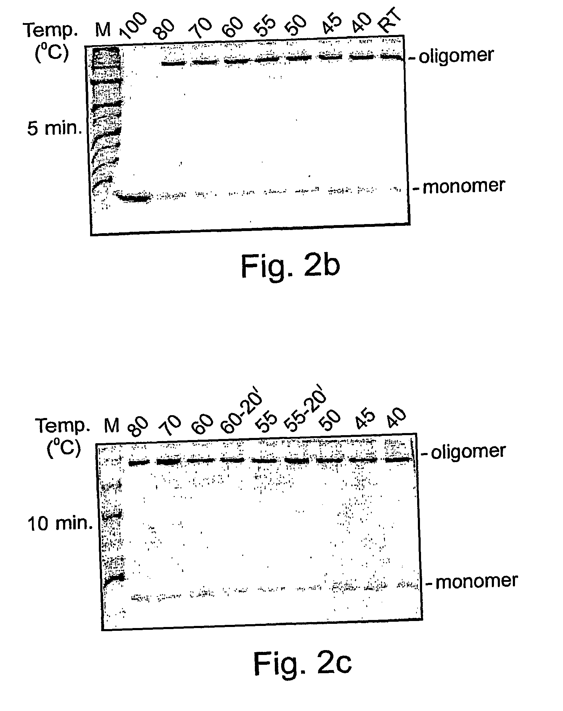

FIGs. 2a-c demonstrate the stability of the SP1 oligomer to SDS- and heat treatment. Figure 2a shows SDS stability of the SP1 oligomer. Electro-eluted SP1 (116 kDa) was prepared in SDS sample buffer at a range of molar ratios, and boiled (+) or not boiled (-) before separation on 17 % polyacrylamide SDS-tricine gels. Figures 2b-c show the heat stability of the SP1 oligomer: Electro-eluted SP1 oligomer (116 kDa) was prepared in SDS sample buffer at a final molar ratio of 1:900 SDS: SP1 monomer and heated for 5, 10 or 20 minutes at the indicated temperatures before separation on 17 % polyacrylamide SDS-tricine gels. RT: room temperature. M: Molecular size markers.

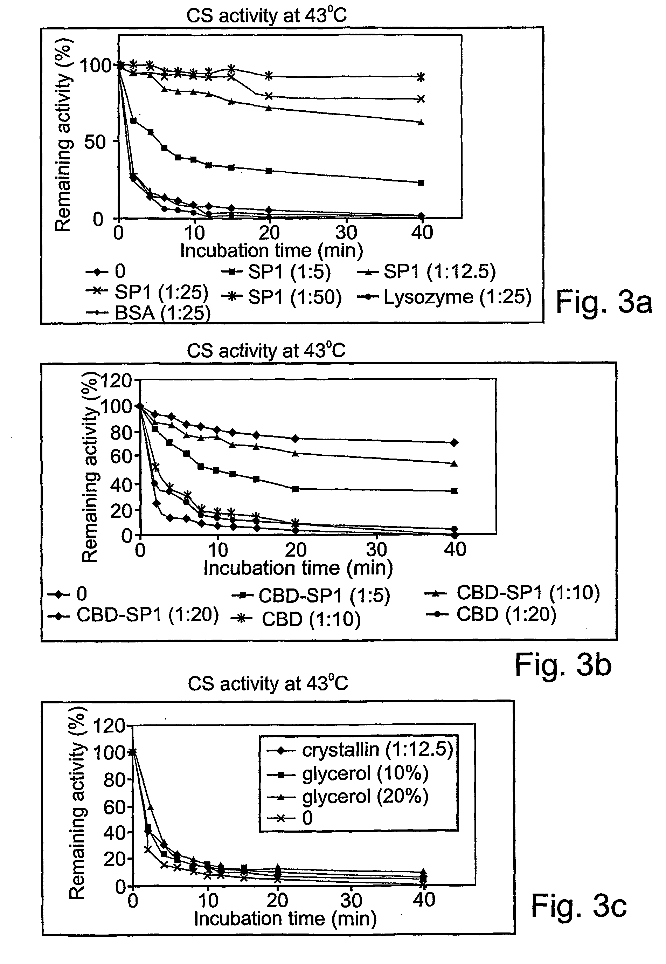

FIGs. 3a-c demonstrate the protection of Citrate Synthase (CS) from heat inactivation by addition of native or recombinant SP1. Enzymatic activity of CS at 43 ° C was assayed at successive intervals (as described in the Examples section that follows). Figure 3a: CS was assayed in the absence (0) or presence of SP1, in the following monomeric molar ratios (CS:SP1): 1:5, 1:12.5, :25, 1:50, or in the presence of BSA and lysozyme (1:25 CS monomer: BSA or lysozyme molar ratio). Figure 3b: CS was assayed in the absence (0) or presence of recombinant CBD-SP1 in the following monomeric molar ratios (CS monomer : CBD-SP1 monomer) :1:5, 1:10 and 1:20, or in the presence of CBD protein at a molar ratio (CS monomer : CBD) of 1:10 or 1:20. Figure 3c: CS was assayed in the absence (0) or presence of alpha crystallin at a monomeric molar ratio (CS:crystallin) of 1:12.5, or in the presence of glycerol at final concentrations of 10 % or 20 %.

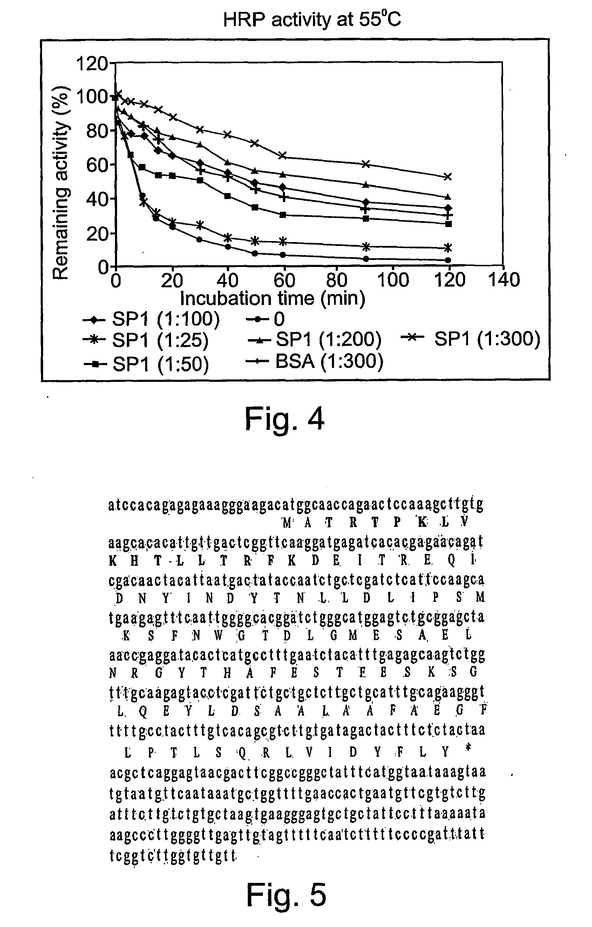

FIG. 4 demonstrates the protection of Horseradish Peroxidase (HRP) from heat inactivation by addition of SP1. The activity of HARP at 55 °C was assayed (as described in the examples section that follows) at successive intervals in the absence (0) or presence of native SP1 at monomeric molar ratio (HRP:SP1) of 1:25, 1:50, 1:100, 1:200 or 1:300; or in the presence of BSA at a final ratio (HRP:BSA) of 1:300.
FIG. 5 depicts the SP1 cDNA nucleotide sequence (SEQ ID NO:1) and deduced protein sequence (SEQ ID NO:2) of SP1. SP1 cDNA was submitted to EMBL under the Acession Number AJ276517).

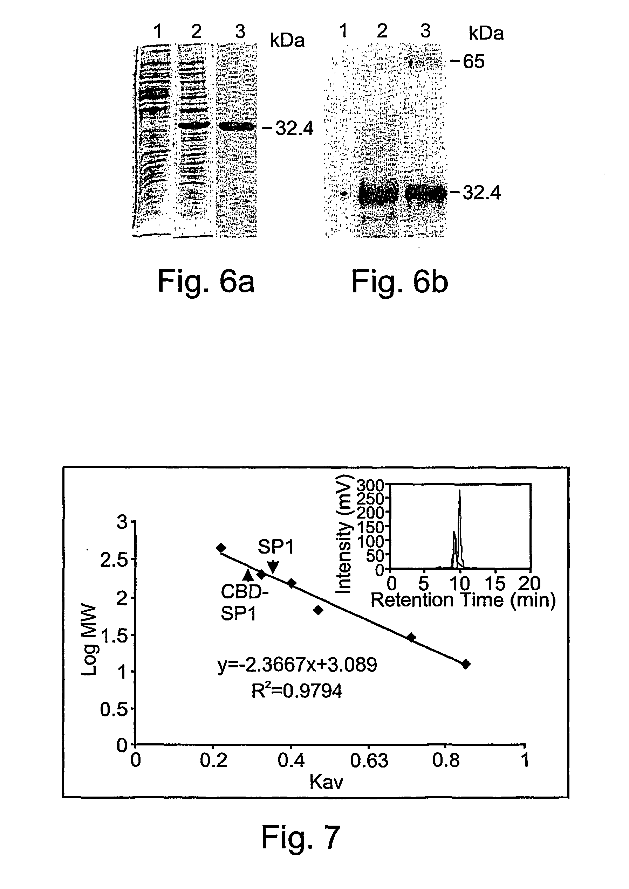

FIGs. 6a-b demonstrate the PAGE analysis and immunodetection of recombinant CBD-SP1 fusion protein (32.4 kDa) from transformed *E. coli* cells. Proteins were separated on 15 % trince-SDS-polyacrylamide mini-gels. Lane 1: Total bacterial proteins (*E. coli* containing no cDNA insert). Lane 2: Total transformed bacterial proteins (*E. coli* containing cbd-SP1 insert). Lane 3: Cellulose- purified CBD-SP1 fusion protein.
FIG. 6a presents a Coomassie blue stained gel of the bacterial proteins.
FIG. 6b demonstrates the immunodetection of SP1 proteins by Western blotting of the bacterial proteins onto nitrocellulose and reaction with polyclonal anti-SP1 antibodies (1:2,500).
FIG. 7 demonstrates the gel filtration HPLC molecular weight analysis of native SP1 and recombinant CBD-SP1. Purified SP1 and CBD-SP1 eluted from the TSK-3000 column as single peaks with retention times of 9.8 (Kav = 0.36025) and 9.2 (Kav = 0.2775) minutes respectively. The calibration curve was obtained by plotting the logarithms of the molecular weights of standard proteins (see Materials and Methods) against their respective elution parameters (Kav). R² volume was calculated by the method of least square and is shown in the figure.
   Insert: Chromatogram of the eluted peaks of SP1 and CBD-SP1.

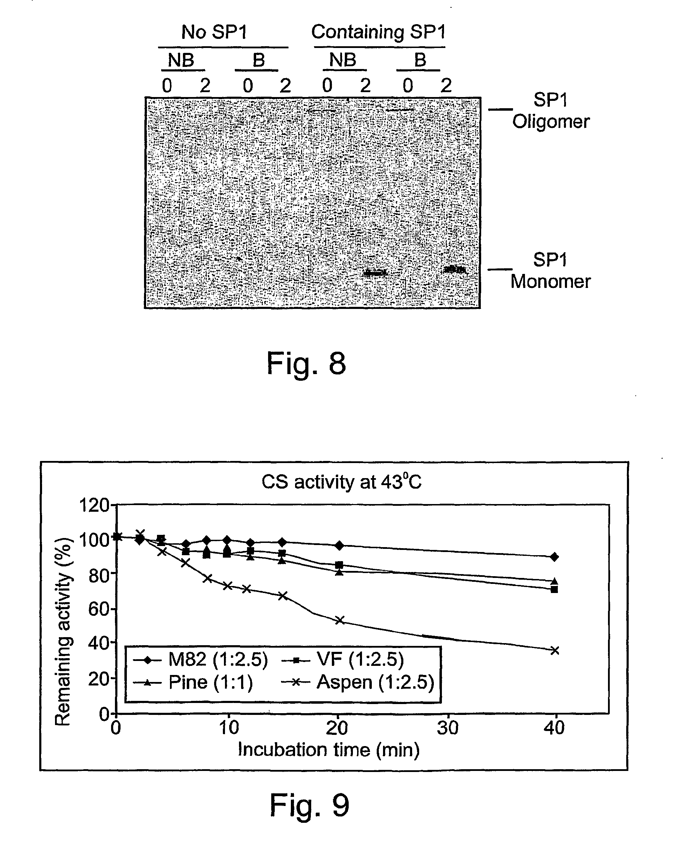

FIG. 8 demonstrates the boiling-stable properties of recombinant SP1 expressed in *Pichia pastoris.* Culture medium of control and SP1-transformed *Pichia pastoris* cells was either boiled (B) or not boiled (NB) for 10 minutes and then centrifuged for 10 minutes at 10,000 g. Supernatant samples were prepared in either full strength (2%) SDS sample buffer (lane 2) or native (0% SDS) sample buffer (lane 0), boiled for 5 minutes, and separated on 17% polyacrylamide SDS-tricine gel. No SP 1: Culture medium from *Pichia pastoris* without SP1 sequences. Containing SP1: Culture medium from *Pichia pastoris* secreting recombinant SP 1.
FIG. 9 demonstrates the prevention of heat inactivation of CS by boiling-stable protein fractions from plants. CS activity at 43°C was assayed (as described in the Examples section that follows) at successive intervals in the presence of boiling-stable proteins extracts of tomato M82, tomato VF36 or aspen (CS: protein equals 1:2.5 µgram per milliliter) or BSA (HRP:BSA molar ratio 1:300).

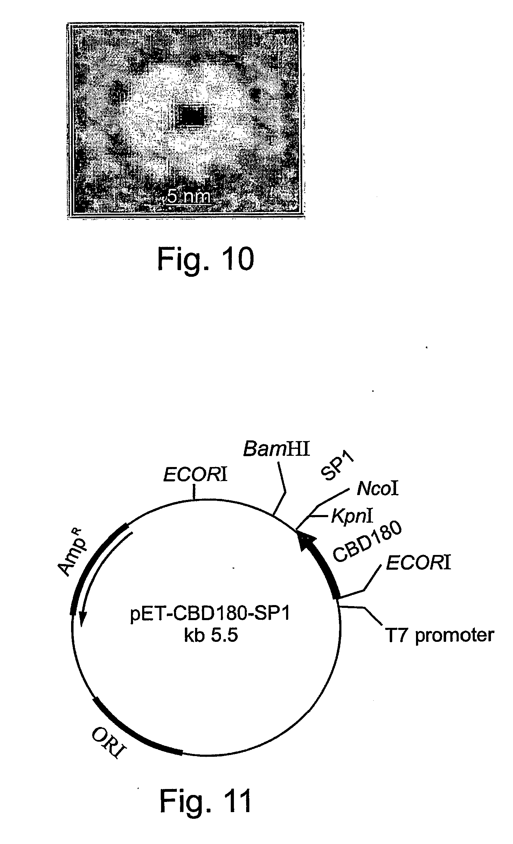

FIG. 10 depicts a transmission electron microscopic (TEM) image of SP1 molecular structure. The image represents the average of 51 particles made by rotational and translational alignment.
FIG. 11 depicts the plasmid construct pET-CBD180-SP1, containing the SP1 cDNA sequence (SEQ ID NO:1) inserted downstream of the CBD-180 element, between the *NcoI and BamHI* restriction sites.

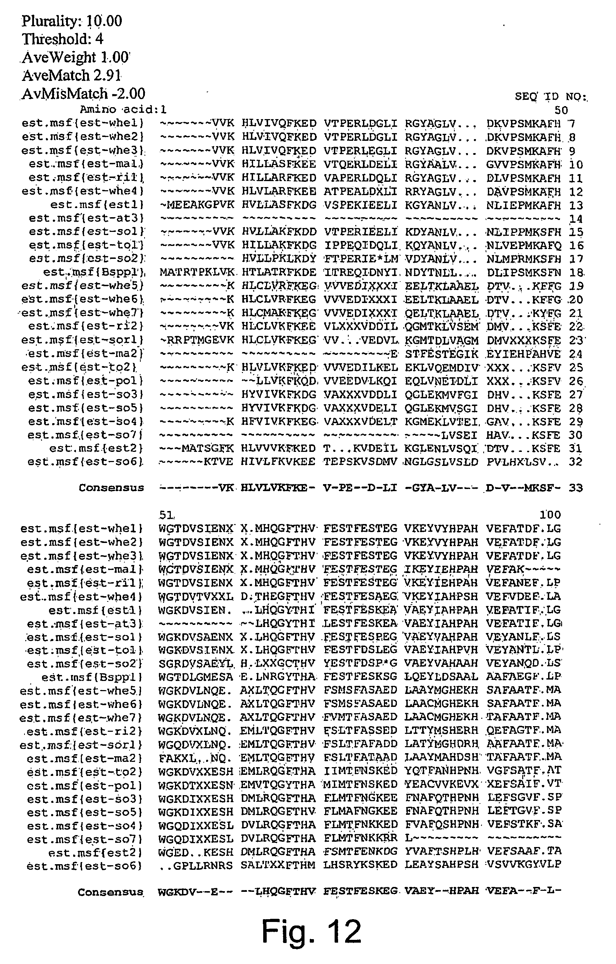

FIG. 12 depicts the comparison of sequence homology between the putative SP1 polypeptide (SEQ ID NO:2) and the putative peptide sequences from homologous ESTs from various related and non-related plant species (SEQ ID NOs:7-32; Plurality = 10.00; threshold = 4.00; average weight = 1.00; average match = 2.91; average mismatch = minus 2.00). Consensus sequences (SEQ ID NO:33) are indicated for each 50 residue grouping.

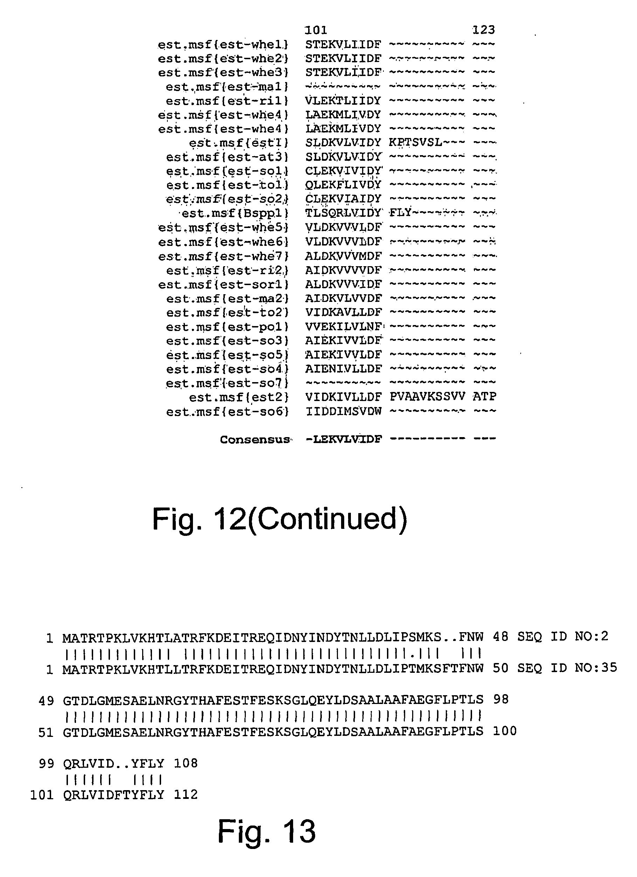

FIG. 13 depicts the comparison of sequence homology between the putative SP1 polypeptide (SEQ ID NO:2) and the putative peptide encoded by a certain pop3 mRNA (SEQ ID NO:34).

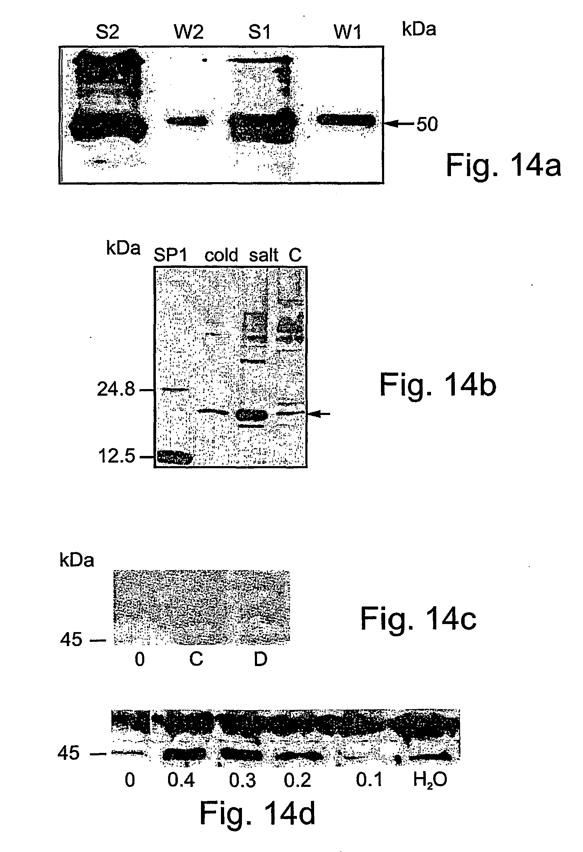

FIGs. 14a-d depict the immunodetection of SPs from pine and tomato having antigenic cross-reactivity to SP 1. Total boiling stable proteins from pine (Figures 14a and 14b) and tomato (Figures 14c and 14d) were prepared (as described in the Examples section that follows), separated on PAGE, blotted onto nitrocellulose and the cross-reactive proteins immune-detected with anti-SP1 or anti-recombinant SP1 antibodies. Figure 14a depicts the immune cross-reactivity of boiling stable proteins from pine (*Pinus halepensis*) needles in rainy (lanes W1 and W2) or dry (lanes S1 and S2) seasons after separation on 12.5 % SDS-glycine PAGE, blotting onto nitrocellulose and reaction with anti recombinant SP1 (anti-CBD-SP1) antibodies. Figure 14b depicts the immune cross-reactivity of boiling stable proteins from 36 hours cold treated (cold), 3 days salt treated (salt) or untreated (C) pine seedlings with purified aspen SP1 (SP1) after separation on 17 % SDS-tricine PAGE, blotting onto nitrocellulose and reaction with anti native (oligomeric) SP1 antibodies. Figures 14c and 14d depict the immune cross-reactivity of boiling stable proteins from tomato (*Lycopersicum esculentum*) leaves. Extracts from leaves subjected to detachment with drought stress (Figure 14c) (lane D), without drought stress (C) or detachment alone (0); or detachment with (lanes 0, 0.1, 0.2, 0.3 and 0.4 M NaCl) or without (H₂O) salt stress (Figure 14d) were separated on 17 % SDS-tricine PAGE, blotted onto nitrocellulose and reacted with anti recombinant (anti-CBD-SP1) antibodies.

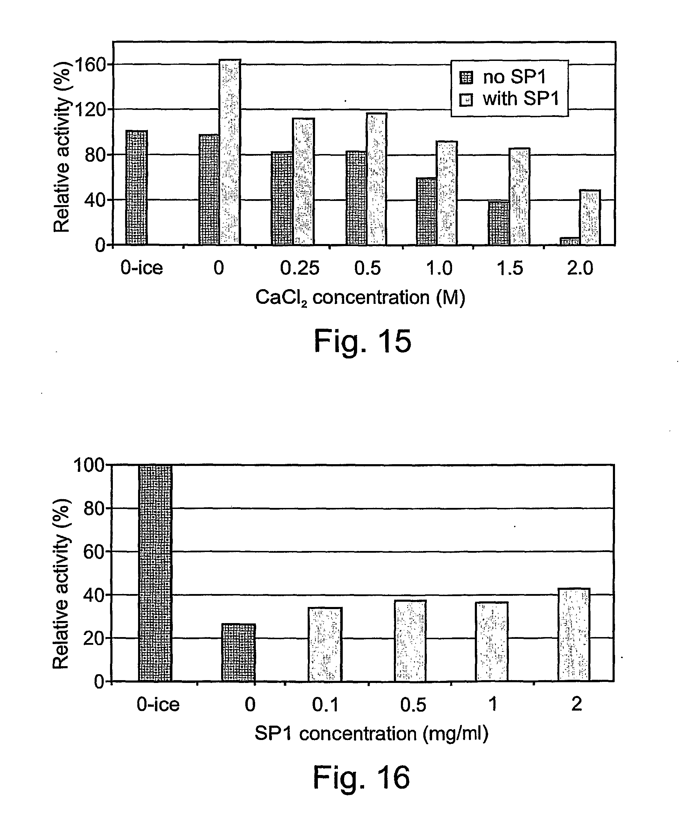

FIG. 15 is a bar graph demonstrating that SP1 protects α-amylase from CaCl₂ induced inactivation. α-Amylase (Sigma, A 6380, dissolved in 20 mM Tris-HCl buffer pH 7.0, containing 6 mM NaCl, 0.2 mg/ml) was incubated in the presence of increasing CaCl₂ concentration at room temperature (25 °C) for 2 HOURS in the absence or presence of SP1 (0.7 µM, of the 12-mer complex) (α-amylase:SP1 molar ratio 6:1). α-Amylase activity was measured using 'SIGMA DIAGNOSTICS AMYLASE' (Sigma, Cat No. 577-3) and was expressed as percentage of the untreated enzyme (0-ice).
FIG. 16 is a bar graph demonstrating that SP1 protects α-amylase activity during incubation at room temperature. α-Amylase (Sigma, A 6380, dissolved in 20 mM Tris-HCl buffer pH 7.0, containing 6 mM NaCl, 0.2 mg/ml) was incubated at room temperature (25 °C) for one week in the absence (0) or presence of SP1 at various concentrations. α-Amylase activity was tested by measuring the amount of starch remained after being hydrolyzed by α -amylase. The activity is defined as milligrams starch that was hydrolyzed by one milligram of α-amylase per minute at 37 °C. The relative activity in this Figure is expressed as percentage of untreated enzyme (0-cold).

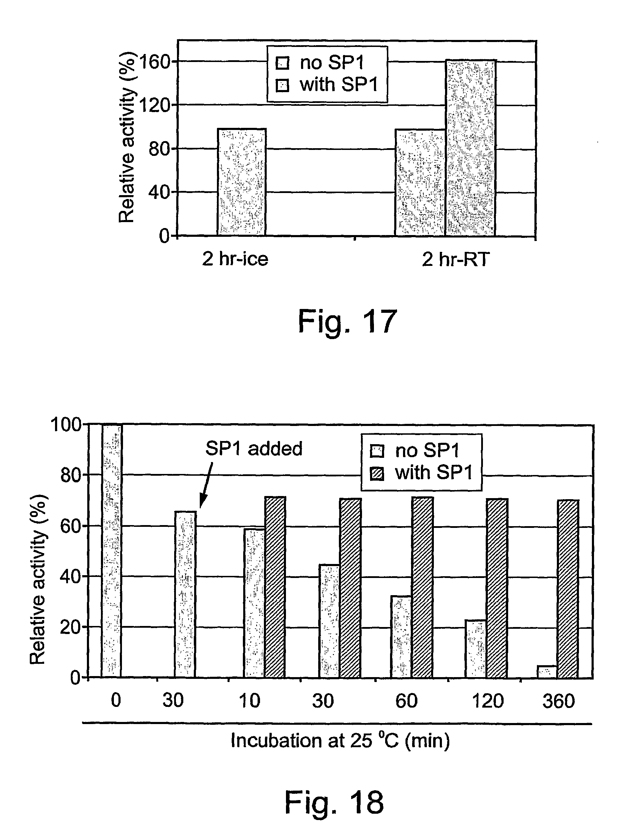

FIG. 17 is a bar graph demonstrating that SP1 repairs α-amylase activity. α-amylase (Sigma, A 6380, dissolved in 20 mM Tris-HCl buffer pH 7.0, containing 6 mM NaCl, 0.2 mg/ml) was incubated with 0.1 mg/ml (0.7 µM) SP1 at room temperature (25 °C). α-Amylase activity was tested 2 hours later, using 'SIGMA DIAGNOSTICS AMYLASE' (Sigma, Cat. No. 577-3). The amylase:SP1 monomeric molar ratio was 6:1.
FIG. 18 is a bar graph demonstrating that SP1 repairs damaged HRP. Diluted HRP solution (5 nM in 40 mM HEPES buffer, pH 7.5) was incubated at room temperature (25 °C) for 30 minutes, followed by SP1 or buffer addition (170 nM, corresponding to a 12-mer complex molar ratio of HRP:SP1 of 1:17). HRP activity was measured at different time points as indicated. The relative activity was calculated as the percentage of non-treated enzyme (0 time).

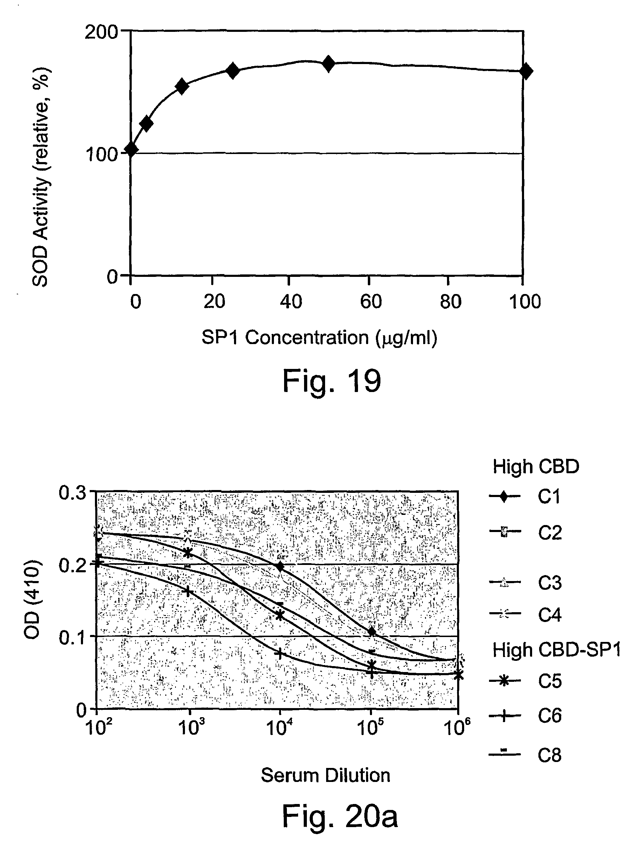

FIG. 19 is a plot demonstrating that SP1 repairs SOD activity. Cosmetic grade SOD ("dismutin", Pentapharm Ltd.) was 1000-fold diluted in 50 mM Acetate/Tris buffer, pH 5.5, 1.0 mM EDTA (final SOD concentration 10 Units/ml, wherein SOD unit is defined as the amount of enzyme which, under specified conditions of the assay, cause a 50% inhibition in the rate of reduction of pyrogallol) and was incubated at 37 °C in the absence or presence of SP1 in the indicated concentrations. SOD reaction conditions was determined in disposable plastic 1 ml cuvett at 25 °C , as follows: 100 µl of SOD solution (10 U/ml) was mixed with 800 ml of Tris/Acetate buffer, pH 8.3, 1.0 mM EDTA and 100 µl of freshly made solution of 2 mM pyrogaloll which was dissolved in the same buffer (final concentration of 0.2 mM). The final pH in the reaction buffer was 8.0. Optical density was recorded after 60 minutes at 420 nm at room temperature.

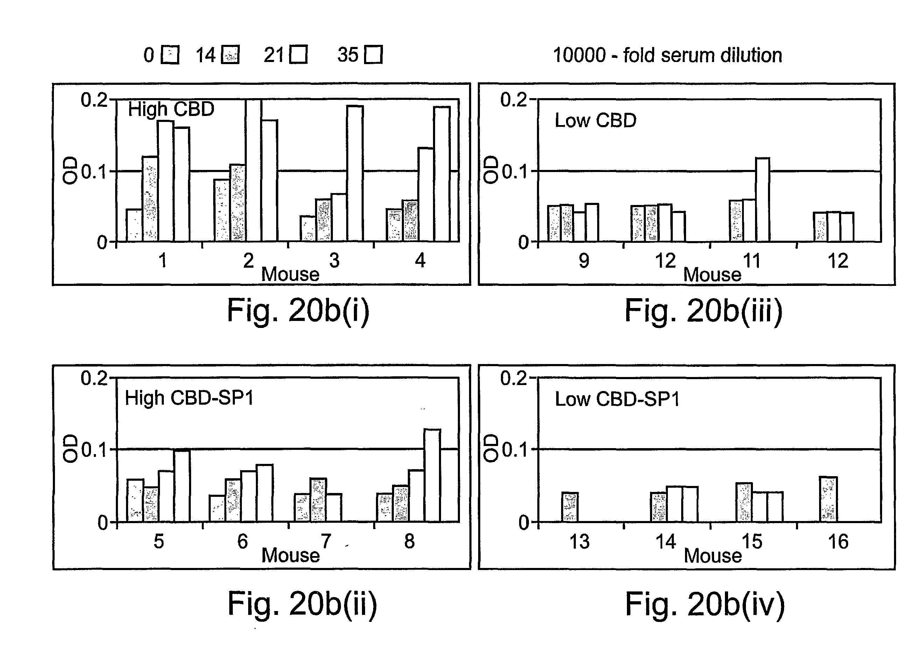

FIGs. 20a and 20b(i)-(iv) are graphs demonstrating that the immune response against CBD of mice injected with CBD is far higher than mice injected with CBD-SP1 fusion. 16 mice (C57BL/6) were injected (100 µl) with either CBD {5 µM (micel-4), 0.05 µM (mice 9-12)} or CBD- SP1 fusion protein} 5 µM (mice 5-8), 0.05 µM (mice 12-16)}. Two injections were given to each mice at day 0 and day 21. Mice were bled just before the first immunization (NIS) 14, 21 and 35 days after the first immunization. Antibody activity in the serum was tested using ELISA with CBD as antigen and HRP conjugated anti mouse antibody for detecting amount of bound antibody. To this end, plates were coated with CBD (1.0 µg/ml PBS, 120 µg/well for 2 hours, at 37°C). Blocking was performed using 1 % BSA in PBS for 1 hour at 37 °C. First antibody reaction was conducted by diluting sera in 1 % BSA in PBS for 1 hour at 37 °C. All washing steps were conducted five times with PBS supplemented with 0.1 % TWEEN-20. Second antibody reaction was conducted with HRP conjugated anti mouse IgG (Sigma, diluted 1/10000, v/v). Color development was stopped by 1 M sulfuric acid after 5 minutes incubation with TMB substrate (3,3',5,5'-tetramethylbenzidiine, PIERCE).

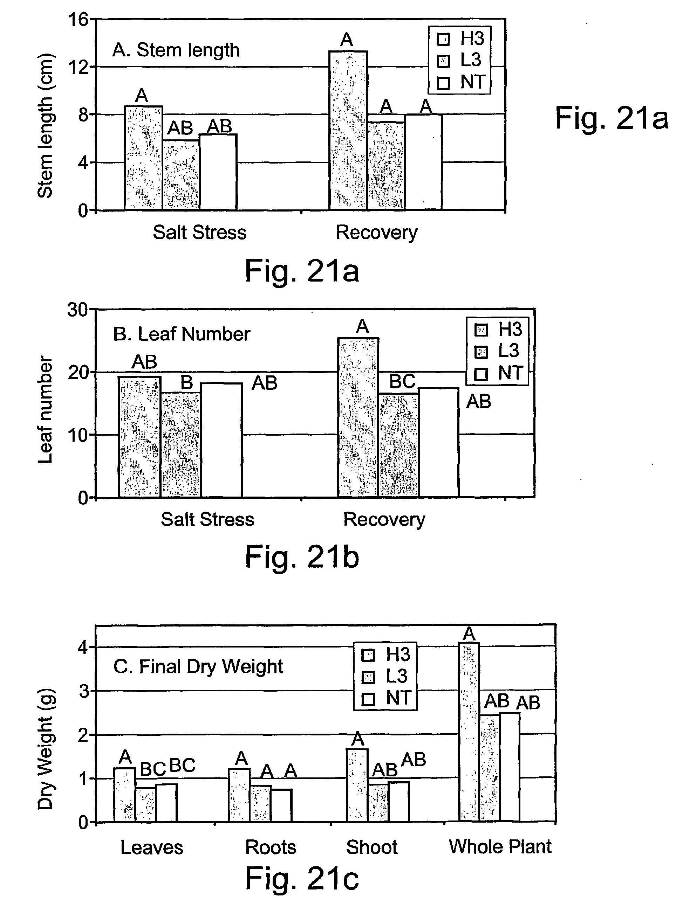

FIGs. 21a-c are bar graphs demonstrating stem elongation, leaf retention and dry weight of aspen plants following salt stress and recovery from stress. Aspen plants were transformed with a vector harboring the sp1 gene under the control of the constitutive promoter CaMV 35S. Two selected spl-transgenic aspen lines, showing different level of SP1 expression, as well as the non-transformed plant (NT), were tested for salt tolerance. In vitro cloned aspen plants were acclimated in greenhouse and hardened in half-liter-pots containing 1:1 vermiculite:garden medium. A block experiment, with groups of six plants for each line and each treatment was designed in a controlled greenhouse (26 °C in day time and 20 °C at night). Control plants were irrigated daily with tap water (250 ml per pot) containing 200 ppm of fertilizer (7/3/7, NPK). Salt-stressed plants were irrigated as the control, but supplemented with 150 mM NaCl and CaCl2 (in a molar ratio of 6:1), for 3 weeks, i.e., "stress period". At the end of the salt treatment, all plants were irrigated for additional 3 weeks without salt, i.e., "recovery period". Stem length and number of leaves, as well as leaf samples for measuring osmotic potential, were taken every week during the stress and recovery periods. The fresh and dry weights were taken at the end of the experiment.

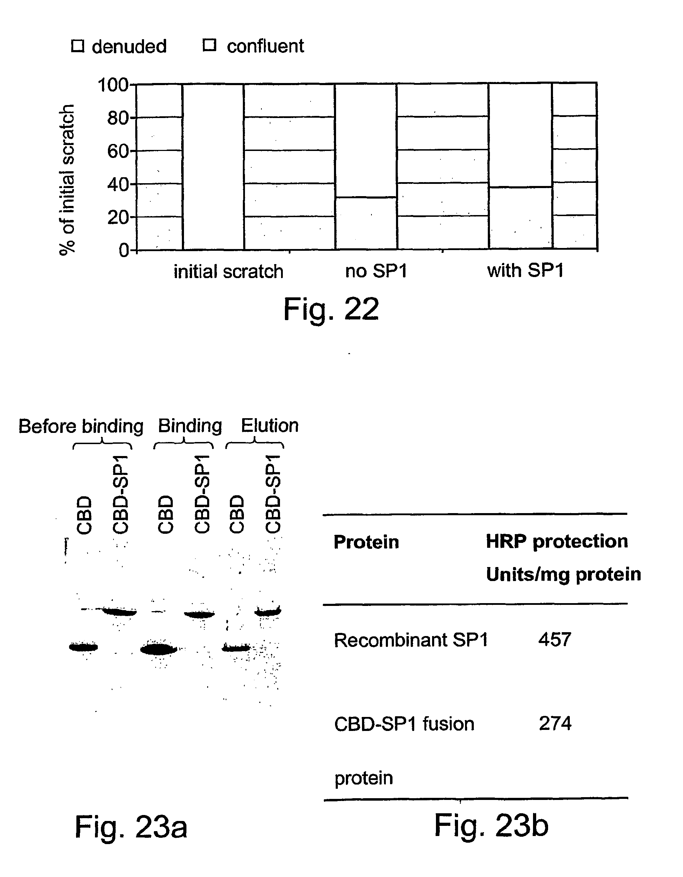

FIG. 22 is a bar graph demonstrating SP1 effect on wound healing. Human fibroblast cells were seeded at a density of 200,000/petri-dish (4 cm in diameter) in a growth medium (2 ml of DEME medium containing 10 % FCS, 2 % glutamine and antibiotics). The medium was changed every two days until cells reached confluecy. A scratch was made with a micropipette tip (1 ml tip) and the dish was washed twice in PBS, in order to remove loosened debris. Four randomly selected spots along the scratch were marked using a marker pen on the bottom of the dish. Fresh medium without or with 5 µg/ml of plant derived SP1 protein was added to the medium. The cells were incubated at 37 °C for 28 hours. The scratch in each plate was recorded at time 0 and 28 hours by taking the pictures of the four-marked spots along the scratch, using a video-camera connected to a inverted microscope. All images were taken in the same magnification. The distance of the scratch was measure on these pictures. The data were averaged for 12 images of 3 replica petri-dishes. In the Figure, confluent was calculated as percentage of initial scratch.
FIGs. 23a-b are a photograph of a gel and a Table demonstrating that CBD-SP1 binds to cellulose as CBD protein does and protects HRP as SP1 does. Equal amount of CBD and CBD-SP1 proteins (15 pmol, calculated based on CBD molecular weight) were applied to 30 mg of cellulose (Sigmacell type 20). The protein samples were collected before applied to the cellulose (Before binding), before elution from cellulose (Binding) and after elution from cellulose (Elution). Protein samples from each stage were mixed with SDS-sample buffer, boiled for 5 min, and separated in 15 % tricine-SDS-PAGE. For HRP protection assay, a 100 µl aliquot of HRP (Sigma, 5 nM in 40 mM HEPES buffer pH 7.5) was incubated at 25 °C in the presence of SP1 or SP1-CBD fusion protein at different concentrations. Aliquots were removed after 16 hours to determine remaining enzymatic activity. HRP reaction conditions were determined as follows: 5 µl of 5 nM HRP and 100 µl of TMB substrate (3,3',5,5'-tetramethylbenzidiine; PIERCE) were incubated at 25 °C. The reaction was stopped after 10 min by addition of 1 M sulfuric acid and was recorded by a microplate reader at 435 nm. Colorimetric reaction of HRP as well as HRP substrate concentration was determined to be in the linear range. The protection units are the dilution factor of an SP1 solution at a concentration of 1 mg/ml that confers 50 % protection of HRP activity under the above conditions.

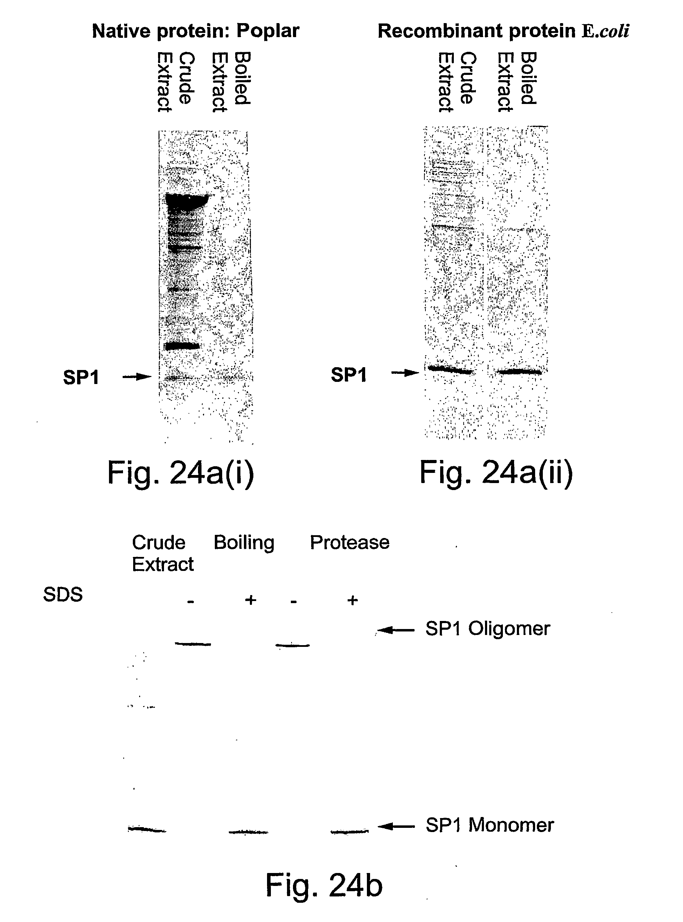

FIGs. 24a(i)-(ii) and 24b are photographs demonstrating SP1 protein production from plants and recombinant bacteria. *E. coli* strain BL21(DE3) was transformed with a plasmid carrying SP1 gene (pET29a, kanamycin resistance conferred) and was grown in M9 minimal medium (Sambrook et al., 1989) containing kanamycin A (Sigma K4000; 50 µg/ml) to (O.D.₍₆₀₀ₙₘ₎) = 0.05. This procedure was repeated for a total of five successive dilutions and regrowths. Sterile glycerol was added to a final concentration of 15 % (v/v). 40 µL aliquots were dispensed aseptically into sterile tubes and stored in -80 °C. Growing of bacteria carrying recombinant SP1 gene: An aliquot of bacterial glycerol stock (40 µl) was aseptically diluted 250 times into 12 ml of M9 minimal medium (Sambrook et al., 1989) containing kanamycin A and grown to O.D.₍₆₀₀ₙₘ₎ of 1-3. This culture was diluted 120 times into complex medium and grown to O.D.₍₆₀₀ₙₘ₎ of 0.8. At this point, the culture was induced by addition of isopropyl-β-D-thiogalactopyranoside (IPTG, 0.5 mM), and culture was allowed to grow for another four hours. Cell were harvested by centrifugation (10000 g, 15 minutes, 4 °C), and cells pellet was stored frozen at -80°C. Extraction and Purification of recombinant SP1 from bacteria Preparation of bacterial storage cultures: Bacteria cells pellet was suspended in Tris·HCl buffer (30 mM; pH = 10.5; 180g/liter; OD_{(600 nm)} =100-120) sonicated on ice (1 hour, pulses of 40 % of full capacity) until turbidity declined four fold. Triton-X-100 and lysozyme were added (0.1% and 10 µg/mL, respectively), incubated with gentle stirring (1 hour at 37 °C), followed by centrifugation (15000 g, 15 minutes, 4 °C). Pellet was discarded, NaCl and MgCl₂ were added to supernatant (150 mM and 2.5 mM, respectively) and the pH was adjusted to 8.3 with HCl. To digest nucleic acids, solution was incubated with Benzonase (Sigma, Cat No. E1014; 0.3 unit/ml) with gentle stirring (12 hours at 37 °C). To digest protein, NH₄HCO₃ and Subtilisin (Sigma, Cat. No. P5380) were added to the extract (0.1 M and 1 µg/ml, respectively) and the solution was incubated with gentle stirring (12 hours at 37 °C). To remove boiling sensitive proteins extract was boiled (10 minutes), cooled on ice and centrifuged (10000 g, 15 minutes, 4 °C). Supernatant was filtered through filter paper (Whatman number 42) and membrane filters (Schleicher & Schull ME-28, 1.2 µm pore size and ME-25, 0.45 µm pore size). Filtrate was concentrated and washed with Tris buffered saline or with phosphate buffered saline until filtrate became colorless. Protein concentration was measured using the Bradford method and SP1 as standard, and adjusted to 10 mg/ml. Thimerosal (Sigma, Cat No. T8784) was added (50 ppm). Solution was dispensed to screw capped amber vials as 1 ml aliquots and stored in dark at 4 °C. Figure 24a(i) (from left to right) Lane 1 - Crude extract of poplar leaves. The sample was mixed with Tricine application buffer and boiled for 10 min prior to application on the gel; Lane 2 - Crude extract of poplar leaves following boiling for 10 minutes and centrifugation. The sample was mixed with Tricine application buffer and boiled for 10 minutes prior to application on the gel. Figure 24a(ii) (from left to right) Lane 1 - Crude extract of recombinant protein expressed in *E. coli.* The sample was mixed with Tricine application buffer and boiled for 10 minutes prior to application on the gel; Lane 2 - Crude extract of recombinant protein expressed in *E. coli* following boiling for 10 minutes and centrifugation. The sample was mixed with Tricine application buffer and boiled for 10 min prior to application on the gel. Figure 24b - Recombinant SP-1 is resistant to boiling and proteolysis (From left to right): Lane 1, Crude extract: solubilized bacteria (see text above); Lane 2 - Supernatant of Crude extract following boiling for 10 minutes and centrifugation. The sample was mixed with Tricine application buffer and boiled for 10 min prior to application on the gel. Lane 3 - Supernatant of Crude extract following boiling for 10 minutes and centrifugation. The sampel was mixed with Tricine application buffer but was not boiled prior to application on the gel. Lane 4 - Crude extract following Proteinase K treatment. The sample was mixed with Tricine application buffer and boiled for 10 minutes prior to application on the gel. Lane 5 - Crude extract following Proteinase K treatment. The sample was mixed with Tricine application buffer and but was not boiled prior to application on the gel.

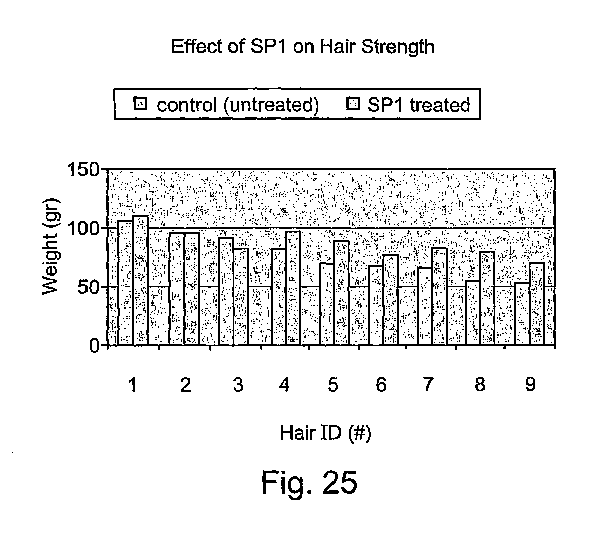

FIG. 25 is a bar graph demonstrating the effect of SP1 on human hair. Human hair, about 20 cm in length, was taken from one individual. Each hair was cut into two fragments, each about 10 cm in length. One fragment was incubated for 10 minutes at room temperature in Tris buffer solution {(100 mM; pH 8.0), control} and the other was incubated in the same buffer containing SP1 (50 µg/ml; SP1 treated hair). The hair was dried in air for 10 minutes, and the strength of each individual fragment was compared with the strength of the other. Each hair fragment was taped using masking tape to a metal rod and on the other end to the handle of a plastic cup. The cup was hanged from the metal rod through the hair. Weigh was increased gradually by adding water to the cup until the hair was torn. Strength was defined as the weight above which the hair was torn. The probability that the strength of the SP1 treated hair is higher than those of the control hair is 97 %, as determined by paired Student t-test.

### DESCRIPTION OF THE PREFERRED EMBODIMENTS

The present invention is of (i) a novel denaturant (e.g., boiling and/or detergent) stable and/or protease resistant, homo-oligomeric proteins, also referred to herein as stable proteins (SPs), having chaperone-like activity; (ii) methods of production and purification of SPs; (iii) nucleic acids encoding SPs; (iv) methods of isolating nucleic acids encoding SPs; (v) antibodies recognizing SPs; (vi) the use of SPs for stabilizing, refolding, activating, preventing aggregation and/or de-aggregating macromolecules, proteins in particular; (vii) fusion proteins including SPs; (viii) nucleic acid constructs encoding the fusion proteins; and (ix) their use for immunization. Additional aspects and applications of the invention are further discussed below.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

While reducing the present invention to practice a novel, major boiling stable protein was purified from water-stressed aspen plants (referred to herein as SP1). In the native state, as demonstrated by SDS-PAGE, SP1 exists as a high-molecular weight oligomeric complex of 116 kDa, which is stable at temperatures in excess of 80 °C, and in the presence of high concentrations (up to 600:1 molar ratio SDS:SP1 monomer) of SDS. The oligomeric SP1 complex is convertible to its monomeric form (12.4 kDa) only when subjected to a combination of extreme temperature (100 °C) and high (greater than, or equal to, 600:1 molar ratio SDS:SP1 monomer) SDS concentrations.

Further, while reducing the present invention to practice, it was shown that native SP1 from crude proteins extract of water-stressed aspen demonstrated resistance to proteolytic digestion with proteinase K. When extracted from liquid nitrogen-homogenized aspen plant material, the predominant soluble protein remaining following 60 minutes digestion with proteinase K was identified as SP1. Furthermore, protease-purified SP1 protein maintains its oligomeric nature. Thus, SP1 exhibits resistance to proteolytic digestion and may be isolated and purified without detergent from plant material.

Further, while reducing the present invention to practice, gel-purified native SP1 was tested for its ability to stabilize and repair heat-labile proteins against thermal inactivation/aggregation. Citrate Synthase enzyme activity declines when incubated at 43 °C for 15 minutes, due to aggregation of the enzyme protein. Some sHsps (such as alpha-crystallin) are capable of preventing aggregation of the dimeric CS protein, but not reversing the inactivation of CS catalytic activity. The addition of SP1 protein (1:50 CS to SP1 monomer) conferred nearly complete thermal protection (93 %) of CS enzyme activity for 40 minutes at 43°C. Lower concentrations of SP1 conferred significant, but proportionally less thermal stabilization. BSA, alpha-crystallin and glycerol were ineffective in protecting CS from thermal inactivation. Similar concentrations of native, purified oligomeric SP1 were also effective in protecting the monomeric enzyme, Horseradish Peroxidase (SP1 to HRP molar ratio of 300:1) from thermal inactivation of catalytic activity by prolonged exposure to 55 °C (53 % activity remaining after 2 hours). In addition, native, purified oligomeric SP1 was effective in repairing the activity of Horseradish Peroxidase following thermal inactivation. Thus, SP1 exhibits chaperone-like ability to stabilize and repair monomeric and polymeric proteins during exposure to denaturing conditions, without inhibition of biological activity under non-denaturing conditions.

Further, while reducing the present invention to practice, poly-adenylated RNA from water-stressed aspen shoots was used to prepare cDNA for a lambda expression library in *E. coli.* A clone expressing a SP1 polypeptide sequence was identified by reactivity with polyclonal anti-SP1 antibodies raised against gel purified native SP1. When amplified and sequenced, the 567 nucleotide SP1 DNA insert (SEQ ID NO:1) was found to contain an open reading frame representing the full-length coding sequence of the SP1 polypeptide monomer (SEQ ID NO:2). Analysis of the coding sequence indicated a highly hydrophilic protein, rich in Threonine, Alanine, Leucine, Glutamic and Serine residues, low in Tryptophan, and lacking Cysteines. No homology with other reported protein sequences was detected, but proteins exhibiting sequence homology with SP1 from various evolutionary distant (phylogenetically remote) plant species were identified using EST databases. Twenty five sequences with significant homology were identified (3 in *Arabidopsis,* 2 in maize, 1 in potato, 2 in rice, 1 in sorghum, 7 in soybean, 2 in tomato and 7 in wheat, SEQ ID NOs:7-32). 96.6 % homology is found between SP1 and a *Populus trichocarpa* x *Populus deltoides* pop3 mRNA sequence (SEQ ID NOs:34 and 35 for nucleic acid and amino acid sequences, respectively). The putative peptide sequences were aligned and compared with the peptide sequence of SP1 (SEQ ID NO:2), revealing a few conserved consensus sequences: "HAFESTFES" (61-75, SEQ ID NO:36), "VKH" (9-11, SEQ ID NO:37) and "KSF" (47-49, SEQ ID NO:38), for example, indicating that SP1 is a member of a family of protein genes with wide representation in both monocotyledonous and dicotyledenous plant genomes. However, except for SP1 no function has been discovered or ascribed for any of the proteins in this family.

Further, while reducing the present invention to practice, when the open reading frame of SP1 cDNA was inserted in the proper orientation downstream of the CBD element of the pET-CBD-180 CBD expression vector, a nucleotide sequence coding for a CBD-SP1 fusion protein was obtained. The recombinant CBD-SP1 fusion protein was detected in inclusion bodies of transformed bacteria. On SDS-PAGE, the fusion protein migrated at 32:4 kDa, and was highly immunoreactive as was determined by Western blot analysis with polyclonal anti-SP1 antibodies. Thus, the fusion protein and the native SP1 have common epitopes. This antigenic identity was further demonstrated upon generation of polyclonal anti-CBD-SP1 antibodies. Taking advantage of the CBD element's affinity for cellulose, recombinant CBD-SP1 protein was purified on cellulose beads. A polyclonal antibody was raised against the highly purified fusion protein. The anti-CBD-SP1 antibody and the anti-native SP1 antibodies both recognized the same SP1 and CBD-SP1 fusion protein on SDS-PAGE of gel-filtration HPLC-purified native (172.5 ± 1.25 kDa) and recombinant (267.5 ± 2.5 kDa) proteins. Thus, recombinant SP1 sequences retain the antigenic and oligomeric-forming properties of the native protein.

Further, while reducing the present invention to practice, secretion of a non-fused, recombinant SP1 was achieved by cloning a portion of the SP1 coding sequence in-frame into a secretory *P. pastoris* expression vector pPIC9K and transforming host plant cells with verified in-frame constructs. Screening and induction of high-level expression revealed that recombinant SP1 is secreted into the culture medium. The non-fused, recombinant SP1 secreted by *P. pastoris* was found to be antigenically cross-reactive with anti-native SP1 and anti-recombinant CBD-SP1 polyclonal antibodies.

Further, while reducing the present invention to practice, the recombinant SP1 recovered from the *P. pastoris* culture medium was subjected to extreme denaturing conditions, and visualized on SDS-PAGE. Like the native protein, recombinant SP1 remained as an oligomeric complex following exposure to high detergent concentrations (e.g., 2 % SDS) or high temperatures (e.g., boiling). Only the combination of the two extremes, high SDS concentration and boiling, caused the protein to migrate as the monomeric form on SDS-PAGE. Thus, recombinant SP1 also retains the antigenic and boiling-and detergent-stable oligomeric character of the native SP1 protein.

The recombinant SP1 polypeptides have chaperone-like activity similar to the native SP1. At a relatively low monomeric molar ratio (20:1 CBD-SP1:CS ratio), the purified CBD-SP1 fusion protein conferred significant (73 %) protection against thermal inactivation of Citrate Synthase (CS) activity. A lower CBD-SP1 to CS ratio (5:1) led to proportionally less protection (33 %), while incubation of the CS with higher concentrations of lysozyme or alpha-crystallin had no stabilizing effect on CS enzyme activity. Thus, the portion of the SP1 protein encoded by the cloned SP1 sequence retains both the antigenic and thermal stabilizing properties of the native protein.

The oligomeric nature of SP1 was examined via transmission electron microscopic (TEM) imaging. The images revealed an oligomer of 12 subunits, arranged in a ring-like arrangement having an external diameter of about 11 nm.

A major boiling-stable protein that was found to protect the catalytic activity of CS was detected in other phylogenetically remote, plants - tomato and pine. When separated on SDS PAGE, blotted onto nitrocellulose and immune-detected with anti-SP1 antibodies, the boiling stable proteins extracts from these remote species were found to contain cross-reactive proteins correlating to both monomeric and oligomeric structure of SP1. Furthermore, these cross reactive proteins from tomato and pine appeared to be drought, cold and salt-stress responsive. Thus, SPs from phylogenetically remote species and which exhibit immune cross-reactivity with SP1 also have chaperone-like activity.

Further while reducing the present invention to practice it was found that SP can be used for protecting an enzyme preparation from reduction in enzymatic activity, for repairing at least a portion of lost enzymatic activity of an enzyme preparation. It was further found that SP can be used for administering to an animal having an immune system a polypeptide, while reducing an immune response against the polypeptide. It was still further found that a transgenic plant expressing SP above a natural amount of SP is more tolerant to and more recoverable from abiotic stress. Similar behavior with respect to biotic stress, such as parasite infection, is anticipated. It was yet further found that SP can be used to increase cell migration and hence can be used for acceleration and/or induction of wound healing. It was also found that SP can be used to increase the strength of hair. It is anticipated that SP could be used to increase the strength of nails and skin as well.

Thus, according to one aspect of the present invention there is provided an isolated nucleic acid comprising a first polynucleotide encoding a denaturant (e.g., boiling and/or detergent) stable and/or protease resistant protein. The denaturant (e.g., boiling and/or detergent) stable and/or protease resistant protein encoded by the polynucleotide of this aspect of the present invention has a chaperone-like activity, which is assayable as is further described herein.

As used herein the phrase "denaturant-stable" refers to major (above 50 %) structural oligomeric stability following a denaturation treatment in aqueous solution. A denaturation treatment can include boiling and exposure to a chemical denaturant, such as, a detergent (e.g., SDS), urea, or guanidin-HCl.

As used herein in the specification and in the claims section that follows, the phrase "boiling stable" refers to major (above 50 %) structural oligomeric stability following treatment at substantially 100 °C in aqueous solution for at least 10 minutes, as determined by a size fractionation assay.

As used herein in the specification and in the claims section that follows, the phrase "detergent stable" refers to major (above 50 %) structural oligomeric stability of an oligomeric protein following treatment in aqueous solution containing 1/2,000 molar ratio (monomer:SDS), as determined by a size fractionation assay.

As used herein in the specification and in the claims section that follows, the phrase "protease resistant" refers to major (above 50%) stability following treatment in aqueous solution containing 50 µg per ml proteinase K for at least 60 minutes at 37 °C.

As used herein in the specification and in the claims section that follows, the phrase "chaperone-like activity" refers to the ability to mediate native folding and native oligomerization of proteins, to prevent the formation of incorrect protein structures, to unscramble existing incorrect protein structures and to limit stress-related damage by inhibiting incorrect interactions that could occur between partially denatured proteins or their domains. One such incorrect interaction could, for example, lead to the irreversible denaturation of enzyme proteins, as in Citrate Synthase, and significant loss of catalytic activity resulting from thermal extremes. Another incorrect interaction could cause aggregation of non-natively folded proteins, as a result of stress or in heterologous gene expression in transformed cells. By preventing such incorrect interactions, molecules having "chaperone-like activity" could confer thermal- and other stress-resistance to biologically active molecules, and prevent or even reverse aggregation of proteins.

The polynucleotide of the invention has a sequence which is at least 50 %, preferably at least 60 %, still preferably at least 65 %, more preferably at least 70 %, still more preferably at least 75 %, preferably at least 80 %, yet preferably at least 85 %, preferably at least 90 %, most preferably at least 95 %, identical with SEQ ID NOs:1, 5, 6, 34, 39 or 40 or a portion thereof of at least 100, at least 150, at least 200 or at least 250 contiguous bases, as determined using the BestFit software of the Wisconsin sequence analysis package, utilizing the Smith and Waterman algorithm, where gap weight equals 50, length weight equals 3, average match equals 10 and average mismatch equals -9.

SEQ ID NO:1 is a cDNA encoding a stable protein (SP) from Aspen (SP1) which was cloned using an expression library and an anti-SP1 antibody raised against a major band of a heat-stable protein fraction. SEQ ID NO:34 is a homologous sequence. Using these or other homologous sequences, and conventional nucleic acid hybridization, reverse-transcription PCR or other techniques, or alternatively, using the anti-SP1 antibodies one of ordinary skills in the art can isolate (i) the genomic clone encoding for SP1; and (ii) cDNA and genomic clones of stable proteins from other species. It should further be emphasized in this context that SP1 is the major protein in a boiling stable protein fraction from water stressed Aspen, it is present in other plant species and it is therefore expected to be the most abundant protein in a boiling stable protein fraction of any plant, especially under water stress conditions, which renders the isolation thereof and polynucleotides encoding same rather simple by, for example, preparative gel electrophoresis, peptide isolation and microsequencing, followed by screening of appropriate or genomic libraries using synthetic oligonucleotides or by RT-PCR.

Thus, according to another aspect of the present invention there is provided a method of isolating a gene encoding a stable protein having chaperone-like activity from a biological source, the method comprising screening an expression library with the polynucleotide described herein or a portion thereof. Additional gene isolation methods are discussed hereinbelow.

As used herein the phrase "complementary polynucleotide" or "cDNA" includes sequences which originally result from reverse transcription of messenger RNA using a reverse transcriptase or any other RNA dependent DNA polymerase. Such sequences can be subsequently amplified *in vivo* or *in vitro* using a DNA dependent DNA polymerase.

As used herein the phrase "genomic polynucleotide" includes sequences which originally derive from a chromosome and reflect a contiguous portion of a chromosome.

Preferably, the stable protein encoded by the polynucleotide of this aspect of the invention has a sequence at least 50 %, preferably at least 60 %, more preferably at least 65 %, still more preferably at least 70 %, still preferably at least 75 %, preferably at least 80 %, yet preferably at least 85 %, preferably at least 90 %, most preferably at least 95 %, homologous (identical + similar amino acids) to SEQ ID NOs:2 or 35, as determined using the BestFit software of the Wisconsin sequence analysis package, utilizing the Smith and Waterman algorithm, where gap creation penalty equals 8 and gap extension penalty equals 2.

Alternatively or additionally, the polynucleotide according to this aspect of the present invention is preferably hybridizable with SEQ ID NOs:1, 5, 6, 34, 39 or 40, or with the nucleic acids encoding SEQ ID NOs:7-33, or portions thereof of at least 100, at least 150, at least 200 or at least 250 consecutive bases.

Hybridization for long nucleic acids (e.g., above 100 bp in length) is effected according to preferred embodiments of the present invention by stringent or moderate hybridization, wherein stringent hybridization is effected by a hybridization solution containing 10 % dextrane sulfate, 1 M NaCl, 1 % SDS and 5 x 10⁶ cpm ³²p labeled probe, at 65 °C, with a final wash solution of 0.2 x SSC and 0.1 % SDS and final wash at 65°C; whereas moderate hybridization is effected by a hybridization solution containing 10 % dextrane sulfate, 1 M NaCl, 1 % SDS and 5 x 10⁶ cpm ³²p labeled probe, at 65 °C, with a final wash solution of 1 x SSC and 0.1 % SDS and final wash at 50 °C.

Thus, this aspect of the present invention encompasses (i) polynucleotides as set forth in SEQ ID NO:1 and 34 (ii) fragments thereof; (iii) sequences hybridizable therewith; (iv) sequences homologous thereto; (v) sequences encoding similar polypeptides with different codon usage; (vi) altered sequences characterized by mutations, such as deletion, insertion or substitution of one or more nucleotides, either naturally occurring or man induced, either randomly or in a targeted fashion. Each such sequence can be expressed using an expression system and the protein encoded thereby tested for stability and chaperone-like activity as is further described an exemplified herein in the Examples section that follows.

As used herein, the phrase "sequences with different codon usage" refers to polynucleotide sequence encoding polypeptides of identical amino acid residue sequence and number, differing in the base composition of one or more of the triplet codons specifying the amino acids. Such different codon usage is a function of the plurality of triplets encoding individual amino acid residues, and has been demonstrated for genes of homologous proteins in remote species such as mammals and protozoa, and for tissue-specific proteins of multi-copy gene families.

According to a preferred embodiment of the invention the isolated nucleic acid according to this aspect of the present invention further comprising a second polynucleotide harboring a promoter sequence for regulating the expression of the first polynucleotide in a sense orientation. Such promoters are known to be cis-acting sequence elements required for transcription as they serve to bind DNA dependent RNA polymerase which transcribes sequences present downstream thereof.

While the first polynucleotide described herein is an essential element of the invention, it is modular and can be used in different contexts. The promoter of choice that is used in conjunction with the polynucleotide of the invention is of secondary importance *per se*, and will comprise any suitable promoter. It will be appreciated by one skilled in the art, however, that it is necessary to make sure that the transcription start site(s) will be located upstream of an open reading frame. In a preferred embodiment of the present invention, the promoter that is selected comprises an element that is active in the particular host cells of interest, be it a bacteria, yeast or a higher cell of a plant or animal, including insect and mammal derived cells.

As used herein a "eukaryote promoter" refers to a promoter that can direct gene expression in eukaryotic cells. It can be derived from a eukaryote genome or from a viral genome capable of infecting a eukaryote cell.

As used herein a "prokaryote promoter" refers to a promoter that can direct gene expression in a prokaryote. It can be derived from a prokaryote genome or plasmid or from a viral genome capable of infecting a prokaryote cell.

As used herein in the specification and in the claims section that follows the phrase "plant promoter" includes a promoter which can direct gene expression in plant cells. Such a promoter can be derived from a plant, viral, fungal or animal origin. Such a promoter can be constitutive, i.e., capable of directing high level of gene expression in a plurality of plant tissues, tissue specific, i.e., capable of directing gene expression in a particular plant tissue or tissues, inducible, i.e., capable of directing gene expression under a stimulus, or chimeric.

Promoters that can direct gene expression in subcellular organelles such as chloroplasts, chloroplastids or mitochondria, are also within the scope of the present invention. Such promoters may be operative also in prokaryotes.

The plant promoter employed can be a constitutive promoter, a tissue specific promoter, an inducible promoter or a chimeric promoter.

Examples of constitutive plant promoters include, without being limited to, CaMV35S and CaMV19S promoters, FMV34S promoter, sugarcane bacilliform badnavirus promoter, CsVMV promoter, *Arabidopsis* ACT2/ACT8 actin promoter, *Arabidopsis* ubiquitin UBQ promoter, barley leaf thionin BTH6 promoter, and rice actin promoter.

Examples of tissue specific promoters include, without being limited to, bean phaseolin storage protein promoter, DLEC promoter, PHSβ promoter, zein storage protein promoter, conglutin gamma promoter from soybean, AT2S1 gene promoter, ACT11 actin promoter from *Arabidopsis,* napA promoter from *Brassica napus* and potato patatin gene promoter.

The inducible promoter is a promoter induced by a specific stimuli such as stress conditions comprising, for example, light, temperature, chemicals, drought, high salinity, osmotic shock, oxidant conditions or in case of pathogenicity and include, without being limited to, the light-inducible promoter derived from the pea rbcS gene, the promoter from the alfalfa rbcS gene, the promoters DRE, MYC and MYB active in drought; the promoters INT, INPS, prxEa, Ha hsp17.7G4 and RD21 active in high salinity and osmotic stress, and the promoters hsr203J and str246C active in pathogenic stress.

The first (coding region) and second (promoter sequence) polynucleotides herein described preferably form a part of a nucleic acid construct which preferably has additional genetic elements as is further described below.

Thus, a construct according to the present invention preferably further includes an appropriate selectable marker. In a more preferred embodiment according to the present invention the construct further includes an origin of replication. In another most preferred embodiment according to the present invention the construct is a shuttle vector, which can propagate both in *E. coli* (wherein the construct comprises an appropriate selectable marker and origin of replication) and be compatible for propagation in cells, or integration in the genome, of an organism of choice. The construct according to this aspect of the present invention can be, for example, a plasmid, a bacmid, a phagemid, a cosmid, a phage, a virus or an artificial chromosome.

The construct of the present invention can be used to express the polypeptide encoded thereby in a variety of species ranging from bacteria such as *E. coli,* yeast cells or higher cells such as the cells of a plant. Expression can be selected stable or transient, as is further detailed hereinunder. Plants overexpressing a stable protein which has chaperone-like activity of the invention are expected to become stress adapted or tolerant, since the endogenous expression of SP1 and SP1-like proteins in plants correlates with stress induction (see Figure 14 and the examples section).

Several nucleic acid transformation methods can be used to implement a method of generating stress tolerant plants according to the present invention.

Thus, there are various methods of introducing nucleic acid constructs into both monocotyledonous and dicotyledenous plants (Potrykus, I., Annu. Rev. Plant. Physiol., Plant. Mol. Biol. (1991) 42:205-225; Shimamoto et al., Nature (1989) 338:274-276). Such methods rely on either stable integration of the nucleic acid construct or a portion thereof into the genome of the plant, or on transient expression of the nucleic acid construct in which case these sequences are not inherited by a progeny of the plant.

There are two principle methods of effecting stable genomic integration of exogenous sequences such as those included within the nucleic acid constructs of the present invention into plant genomes:
(i) Agrobacterium-mediated gene transfer: Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Arntzen, C. J., Butterworth Publishers, Boston, Mass. (1989) p. 93-112.
(ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The *Agrobacterium* system includes the use of plasmid vectors that contain defined DNA segments that integrate into the plant genomic DNA. Methods of inoculation of the plant tissue vary depending upon the plant species and the *Agrobacterium* delivery system. A widely used approach is the leaf disc procedure which can be performed with any tissue explant that provides a good source for initiation of whole plant differentiation. Horsch et al. in Plant Molecular Biology Manual A5, Kluwer Academic Publishers, Dordrecht (1988) p. 1-9. A supplementary approach employs the *Agrobacterium* delivery system in combination with vacuum infiltration. The *Agrobacterium* system is especially viable in the creation of transgenic dicotyledenous plants.

There are various methods of direct DNA transfer into plant cells. In electroporation, protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals, tungsten particles or gold particles, and the microprojectiles are physically accelerated into cells or plant tissues.

Following transformation plant propagation is exercised. The most common method of plant propagation is by seed. Regeneration by seed propagation, however, has the deficiency that due to heterthere is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Transient expression methods which can be utilized for transiently expressing the isolated nucleic acid included within the nucleic acid construct of the present invention include, but are not to, microinjection and bombardment as described above but under conditions which favor transient expression, and viral mediated expression wherein a packaged or unpackaged recombinant virus vector including the nucleic acid construct is utilized to infect plant tissues or cells such that a propagating recombinant virus established therein expresses the non-viral nucleic acid sequence.

Viruses that have been shown to be useful for the transformation of plant hosts include CaMV, TMV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Construction of plant RNA viruses for the introduction and expression of non-viral exogenous nucleic acid sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; and Takamatsu et al. FEBS Letters (1990) 269:73-76.

When the virus is a DNA virus, the constructions can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous nucleic acid sequences such as those included in the construct of the present invention is demonstrated by the above references as well as in U.S. Pat. No. 5,316,931.

In one embodiment, a plant viral nucleic acid is provided in which the native coat protein coding sequence has been deleted from a viral nucleic acid, a non-native plant viral coat protein coding sequence and a non-native promoter, preferably the subgenomic promoter of the non-native coat protein coding sequence, capable of expression in the plant host, packaging of the recombinant plant viral nucleic acid, and ensuring a systemic infection of the host by the recombinant plant viral nucleic acid, has been inserted. Alternatively, the coat protein gene may be inactivated by insertion of the non-native nucleic acid sequence within it, such that a protein is produced. The recombinant plant viral nucleic acid may contain one or more additional non-native subgenomic promoters. Each non-native subgenomic promoter is capable of transcribing or expressing adjacent genes or nucleic acid sequences in the plant host and incapable of recombination with each other and with native subgenomic promoters. Non-native (foreign) nucleic acid sequences may be inserted adjacent the native plant viral subgenomic promoter or the native and a non-native plant viral subgenomic promoters if more than one nucleic acid sequence is included. The non-native nucleic acid sequences are transcribed or expressed in the host plant under control of the subgenomic promoter to produce the desired products.

In a second embodiment, a recombinant plant viral nucleic acid is provided as in the first embodiment except that the native coat protein coding sequence is placed adjacent one of the non-native coat protein subgenomic promoters instead of a non-native coat protein coding sequence.

In a third embodiment, a recombinant plant viral nucleic acid is provided in which the native coat protein gene is adjacent its subgenomic promoter and one or more non-native subgenomic promoters have been inserted into the viral nucleic acid. The inserted non-native subgenomic promoters are capable of transcribing or expressing adjacent genes in a plant host and are incapable of recombination with each other and with native subgenomic promoters. Non-native nucleic acid sequences may be inserted adjacent the non-native subgenomic plant viral promoters such that the sequences are transcribed or expressed in the host plant under control of the subgenomic promoters to produce the desired product.

In a fourth embodiment, a recombinant plant viral nucleic acid is provided as in the third embodiment except that the native coat protein coding sequence is replaced by a non-native coat protein coding sequence.

The viral vectors are encapsidated by the coat proteins encoded by the recombinant plant viral nucleic acid to produce a recombinant plant virus. The recombinant plant viral nucleic acid or recombinant plant virus is used to infect appropriate host plants. The recombinant plant viral nucleic acid is capable of replication in the host, systemic spread in the host, and transcription or expression of foreign gene(s) (isolated nucleic acid) in the host to produce the desired protein.

Alternatively, the nucleic acid construct according to this aspect further includes a positive and a negative selection markers and may therefore be employed for selecting for homologous recombination events, including, but not limited to, homologous recombination employed in knock-in and knock-out procedures. One ordinarily skilled in the art can readily design a knock-out or knock-in constructs including both positive and negative selection genes for efficiently selecting transfected embryonic stem cells that underwent a homologous recombination event with the construct. Further detail relating to the construction and use of knock-out and knock-in constructs is provided in, for example, Fukushige, S. and Ikeda, J.E.: Trapping of mammalian promoters by Cre-lox site-specific recombination. DNA Res 3 (1996) 73-80; Bedell, M.A., Jenkins, N.A. and Copeland, N.G.: Mouse models of human disease. Part I: Techniques and resources for genetic analysis in mice. Genes and Development 11 (1997) 1-11; Bermingham, J.J., Scherer, S.S., O'Connell, S., Arroyo, E:, Kalla, K.A., Powell, F.L. and Rosenfeld, M.G.: Tst-1/Oct-6/SCIP regulates a unique step in peripheral myelination and is required for normal respiration. Genes Dev 10 (1996) 1751-62.

According to another aspect there is provided a transgenic plant expressing a denaturant stable and/or protease resistant protein, the denaturant stable and/or protease resistant protein having a chaperone-like activity above a natural amount of the denaturant stable and/or protease resistant protein having the chaperone-like activity in the plant.

Elevated native SP expression in plants is positively correlated to stress conditions. Overexpression of SP1 in plants resulted in (i) rendering the plant more tolerant to, and more recoverable following, a biotic stress.

Hence, there is provided a method of rendering a plant more tolerant to a biotic or abiotic stress. The method according to this aspect is effected by engineering the plant to express a denaturant stable and/or protease resistant protein, the denaturant stable and/or protease resistant protein having a chaperone-like activity, above a natural amount of the denaturant stable and/or protease resistant protein having the chaperone-like activity in the plant.

According to another aspect there is provided a method of rendering a plant more recoverable from a biotic or abiotic stress. The method according to this aspect of the invention is effected by engineering the plant to express a denaturant stable and/or protease resistant protein, the denaturant stable and/or protease resistant protein having a chaperone-like activity, above a natural amount of the denaturant stable and/or protease resistant protein having the chaperone-like activity in the plant.

According to still another aspect there is provided an oligonucleotide of at least 17, at least 18, at least 19, at least 20, at least 22, at least 25, at least 30 or at least 40, bases specifically hybridizable with any of the polynucleotides described herein encoding a stable protein.

Hybridization of shorter nucleic acids (below 100 bases in length, e.g., 17-40 bases in length) is effected by stringent, moderate or mild hybridization, wherein stringent hybridization is effected by a hybridization solution of 6x SSC and 1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 1 - 1.5 °C below the Tₘ, final wash solution of 3 M TMACI, 0.01 M sodium phosphate (pH 6.8), 1 mM EDTA (pH 7.6), 0.5 % SDS at 1 - 1.5 °C below the Tₘ; moderate hybridization is effected by a hybridization solution of 6 x SSC and 0.1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 2 - 2.5 °C below the Tₘ, final wash solution of 3 M TMACI, 0.01 M sodium phosphate (pH 6.8), 1 mM EDTA (pH 7.6), 0.5 % at 1 - 1.5 °C below the Tₘ, final wash solution of 6 x SSC, and final wash at 22 ° C; whereas mild hybridization is effected by a hybridization solution of 6 x SSC and 1 % SDS or 3 M TMACI, 0.01 M sodium phosphate (pH 6.8), 1 mM EDTA (pH 7.6), 0.5 % SDS, 100 µg/ml denatured salmon sperm DNA and 0.1 % nonfat dried milk, hybridization temperature of 37 °C, final wash solution of 6 x SSC and final wash at 22°C.

According to an additional aspect there is provided a pair of oligonucleotides each independently of at least 17, at least 18, at least 19, at least 20, at least 22, at least 25, at least 30 or at least 40 bases specifically hybridizable with the isolated nucleic acid described herein in an opposite orientation so as to direct exponential amplification of a portion thereof in a nucleic acid amplification reaction, such as a polymerase chain reaction (PCR). The polymerase chain reaction and other nucleic acid amplification reactions are well known in the art and require no further description herein. The pair of oligonucleotides according to this aspect of the present invention are preferably selected to have compatible melting temperatures (Tm), e.g., melting temperatures which differ by less than that 7 °C, preferably less than 5 °C, more preferably less than 4°C, most preferably less than 3 °C, ideally between 3 °C and zero °C. Suitable oligonucleotide pairs can be selected using the OLIGO software.

Consequently, according to yet an additional aspect there is provided a nucleic acid amplification product obtained using the pair of primers described herein. Such a nucleic acid amplification product can be isolated by gel electrophoresis or any other size based separation technique. Alternatively, such a nucleic acid amplification product can be isolated by affinity separation, either stranded affinity or sequence affinity. In addition, once isolated, such a product can be further genetically manipulated by restriction, ligation and the like, or it can be labeled, as required for further use.

According to a presently preferred embodiment the denaturant (e.g., boiling and/or detergent) stable and/or protease resistant protein encoded by the polynucleotide of the invention is natively a homo-oligomer of, for example, at least 10 subunits, optionally 12 or 14 subunits, arranged, for example, in a concentric arrangement, which homo-oligomer is denaturant (e.g., boiling and/or detergent) stable and/or protease resistant as these terms are herein defined. It will, however, be appreciated that the process of homo-oligomer formation of stable proteins may result in homo-oligomers of less subunits, as, at least for short time periods, partially assembled homo-oligomers of 2 or more subunits are expected.

According to another aspect there is provided a method of isolating a gene encoding a denaturant (e.g., boiling and/or detergent) stable and/or protease resistant protein having chaperone-like activity from a biological source, the method comprising (a) extracting total proteins from the biological source, so as to obtain a proteins extract; (b) boiling the proteins extract; (c) collecting soluble proteins; (d) obtaining a purified boiling stable protein having chaperone-like activity; (e) raising antibodies recognizing the boiling stable protein having the chaperone-like activity; and (f) screening an expression library with the antibodies.

According to yet another aspect there is provided a method of isolating a gene encoding a denaturant (e.g., boiling and/or detergent) stable and/or protease resistant protein having chaperone-like activity from a biological source, the method comprising (a) extracting total proteins from the biological source, so as to obtain a proteins extract; (b) boiling the proteins extract; and (c) collecting soluble proteins; (d) obtaining a purified boiling stable protein having the chaperone-like activity, by, for example, assaying the soluble proteins for chaperone-like activity and enriching or isolating a stable protein having chaperone-like activity; (e) microsequencing the stable protein having the chaperone-like activity, so as to obtain at least a partial amino acid sequence thereof; (f) designing an oligonucleotide corresponding to the amino acid sequence; and (g) screening a library with the oligonucleotide.

Design and synthesis of oligonucleotides corresponding to a given amino acid sequence and the use thereof for screening libraries are well known in the art, see, for example, the general references listed below in the Examples section. Such oligonucleotides can alternatively be used in a PCR, RT-PCR, RACE and the like procedures to isolate the gene by cDNA amplification.

There is also provided a method of isolating a nucleic acid potentially encoding a denaturant (e.g., boiling and/or detergent) stable and/or protease resistant protein having chaperone-like activity. The method according to this aspect of the invention is effected by screening a cDNA or genomic library with a polynucleotide of at least 17 bases, at least 60 % identical to a contiguous portion of SEQ ID NOs:1, 5, 6, 34, 39 or 40. Such a polynucleotide can be a synthetic oligonucleotide as is further described hereinabove and is preferably labeled with a suitable label.

It is also disclosed a method of identifying a nucleic acid potentially encoding a denaturant (e.g., boiling and/or detergent) stable and/or protease resistant protein having chaperone-like activity. This method is effected by searching an electronic library containing a plurality of nucleic acid and/or amino acid sequences for sequences having a predetermined degree of identity or homology to any of SEQ ID NOs:1, 2, 5-35 or 39-40 or portions thereof of, or corresponding to, at least 15, at least 17, at least 20, at least 25, at least bases 30 or more bases.

Another aspect provides a method of isolating a nucleic acid potentially encoding a denaturant (e.g., boiling and/or detergent) stable and/or protease resistant protein having chaperone-like activity. The method comprising (a) providing at least one pair of oligonucleotides each independently being at least 15, at least 17, at least 20, at least 25, at least bases 30 or more bases in length, the at least one pair of oligonucleotides including at least one oligonucleotide corresponding to SEQ ID NOs:1, 2, 5-35 or 39-40, the at least one pair of oligonucleotides being selected for amplifying a nucleic acid having a degree of identity with, or encoding proteins homologous, to SEQ ID NOs:1, 2, 5-35 or 39-40; (b) contacting the at least one pair of oligonucleotides with a sample of nucleic acid and amplifying the nucleic acid having the degree of identity with, or encoding proteins homologous to, SEQ ID NOs:1, 2, 5-35 or 39-40; and (c) using the nucleic acid having the degree of identity with, or encoding proteins homologous to, SEQ ID NOs:1, 2, 5-35 or 39-40 for isolating a nucleic acid potentially encoding a denaturant (e.g., boiling and/or detergent) stable and/or protease resistant protein.

According to another aspect there is provided a denaturant (e.g., boiling and/or detergent) stable and/or protease resistant polypeptide having a chaperone-like activity, effective, for example, in stabilizing proteins. Preferably, the polypeptide is encoded by a polynucleotide as described herein. Most preferably, the polypeptide has a sequence at least 50 %, preferably at least 60 %, more preferably at least 65 %, still more preferably at least 70 %, still preferably at least 75 %, preferably at least 80 %, yet preferably at least 85 %, preferably at least 90 %, most preferably at least 95 %, homologous (identical + similar amino acids) to SEQ ID NOs:2 or 35, as determined using the BestFit software of the Wisconsin sequence analysis package, utilizing the Smith and Waterman algorithm, where gap creation penalty equals 8 and gap extension penalty equals 2. The polypeptide of this aspect of the invention is preferably natively a homo-oligomer, preferably a homo-oligomer of 14 subunits as is further detailed hereinabove. As is further detailed below, the polypeptide of the invention can be purified from a boiling stable/protease resistant fraction of plants. Alternatively, it can be manufactured using recombinant DNA technology as is further described and exemplified herein. It is shown in the Examples section that follows and it is further discussed hereinabove that a recombinant polypeptide of the invention and its corresponding native protein share similar oligomerization, epitope and chaperone-like activity properties.

The polypeptides can be purified by any of the means known in the art. Various methods of protein purification are described, e.g., in Guide to Protein Purification, ed. Deutscher, Meth. Enzymol. 185, Academic Press, San Diego, 1990; and Scopes, Protein Purification: Principles and Practice, Springer Verlag, New York, 1982.

Thus, according to another aspect there is provided a method of enriching or isolating a denaturant (e.g., boiling and/or detergent) stable and protease resistant protein having chaperone-like activity from a biological source. The method according to this aspect is effected by (a) extracting total proteins from the biological source, so as to obtain a proteins extract; (b) boiling the proteins extract; (c) collecting soluble proteins; and optionally (d) assaying for chaperone-like activity of soluble proteins. Preferably, the method further comprises size fractionating the soluble proteins and assaying a fractionated protein for chaperone-like activity, as is further described herein.

As used herein, the phrase "isolating a protein", means identifying and separating and/or recovering a protein from a component of its natural environment. Contaminant components of its natural environment are materials that would interfere with diagnostic, therapeutic or commercial uses for the protein, and may include enzymes and other proteinaceous or non-proteinaceous solutes. As used herein, the phrase "enriching a protein" means separating a protein from at least 10 %, and preferably 50 % of the contaminating components of its natural environment, as mentioned above.

According to still a further aspect there is provided a method of detergent-free isolation of a protease-resistant protein having chaperone-like activity from biological source. The method is effected by (a) extracting the soluble proteins preferably using a cold extraction procedure (e.g., at least -50 °C, preferably -80 °C), so as to obtain a proteins extract; (b) contacting the protein extract with a protease; (c) isolating a protease-resistant protein; and optionally (d) assaying the protease-resistant protein for chaperone-like activity.

According to another aspect there is provided yet another method of isolating a boiling stable protein having chaperone-like activity from a biological source. The method according to this aspect of the invention is effected by (a) extracting total proteins from the biological source, so as to obtain a proteins extract; (b) boiling the protein extract; (c) recovering soluble protein fraction; and optionally (d) assaying the protease-resistant protein for chaperone-like activity. A protease can also be used in this procedure.

According to still an additional aspect there is provided a method of preventing an aggregating protein from aggregating into an aggregate. The method according to this aspect of the invention is effected by contacting an effective amount of the polypeptide described herein with the aggregating protein.

The "effective amount" for the purposes herein is determined by considerations which are known to the skilled artisan. The amount must be effective to induce in the contacted protein a significant increase in solubility under conditions otherwise producing aggregation, as assessed by physico-chemical or functional measurements, such as resistance to precipitation upon centrifugation, a decrease in refractile properties, decrease in molecular mass upon size fractionation on SDS-PAGE, HPLC, filtration, dialysis or any other size fractionation methodology; and/or retention of biological properties such as catalytic activity, molecular binding activity and antigenic properties.

According to a further aspect there is provided a method of de-aggregating aggregates of an aggregating protein. The method according to this aspect of the invention is effected by contacting an effective amount of the polypeptide described herein with the aggregate.

Hence, there is provided a method of treating a disease associated with protein aggregation of an aggregating protein, the method comprising administering to a subject in need thereof a denaturant stable and/or protease resistant protein, the denaturant stable and/or protease resistant protein having a chaperone-like activity, in an amount sufficient for de-aggregating and/or preventing aggregation of the aggregating protein, the aggregating protein is, for example, beta-amyloid or prion, as is the case in Alzheimer's disease and prion associated diseases, e.g., encephalus spongyform.

According to yet a further aspect there is provided a method of stabilizing a protein against denaturing conditions. The method according to this aspect of the invention is effected by contacting an effective amount of the polypeptide described herein to become in contact with the protein.

In this context, the present invention was reduced to practice with respect to citrate synthase and horseradish peroxidase, which are accepted model systems for evaluating protein anti-aggregation, stabilization and chaperone activity, as is further described and exemplified in the Examples section that follows.

According to still a further aspect there is provided a method of protecting an enzyme preparation from reduction in enzymatic activity. The method according to this aspect of the invention is effected by adding to the enzyme preparation a denaturant stable and/or protease resistant protein, the denaturant stable and/or protease resistant protein having a chaperone-like activity, in an amount sufficient for protecting the enzyme preparation from reduction in enzymatic activity.

According to a further aspect there is provided a method of repairing at least a portion of lost enzymatic activity of an enzyme preparation. The method according to this aspect of the invention is effected by adding to the enzyme preparation a denaturant stable and/or protease resistant protein, the denaturant stable and/or protease resistant protein having a chaperone-like activity, in an amount sufficient for repairing at least the portion of the lost enzymatic activity of the enzyme preparation.

According to yet an additional aspect there is provided an antibody, either polyclonal or monoclonal antibody, recognizing at least one epitope of the polypeptide described herein. The present invention can utilize serum immunoglobulins, polyclonal antibodies or fragments thereof, (i.e., immunoreactive derivative of an antibody), or monoclonal antibodies or fragments thereof. Monoclonal antibodies or purified fragments of the monoclonal antibodies having at least a portion of an antigen binding region, including, such as, Fv, F(abl)2, Fab fragments (Harlow and Lane, 1988 Antibody, Cold Spring Harbor), single chain antibodies (U.S. Patent 4,946,778), chimeric or humanized antibodies and complementarily determining regions (CDR) may be prepared by conventional procedures. Purification of these serum immunoglobulins, antibodies or fragments can be accomplished by a variety of methods known to those of skill, precipitation by ammonium sulfate or sodium sulfate followed by dialysis against saline, ion exchange chromatography, affinity or immunoaffinity chromatography as well as gel filtration, zone electrophoresis, etc. (see Goding in, Monoclonal Antibodies: Principles and Practice, 2nd ed., pp. 104-126, 1986, Orlando, Fla., Academic Press). Under normal physiological conditions antibodies are found in plasma and other body fluids and in the membrane of certain cells and are produced by lymphocytes of the type denoted B cells or their functional equivalent. Antibodies of the IgG class are made up of four polypeptide chains linked together by disulfide bonds. The four chains of intact IgG molecules are two identical heavy chains referred to as H-chains and two identical light chains referred to as L-chains. Additional classes includes IgD, IgE, IgA, IgM and related proteins.

Methods for the generation and selection of monoclonal antibodies, including single chain antibodies, are well known in the art, as summarized for example in reviews such as Tramontano and Schloeder, Methods in Enzymology 178, 551-568, 1989. Purified native SPs, recombinant SPs or recombinant SP-fusion proteins (see below) of the present invention or immunogenic portions thereof including at least one immunogenic epitope may be used to generate the antibodies of the invention.

Preferably, the elicitation of the antibody is through *in vivo* or *in vitro* techniques, the antibody having been prepared by a process comprising the steps of, first, exposing cells (either *in vivo* or *in vitro*) capable of producing antibodies to a SP protein of the invention or an immunogenic portion thereof, thereby generating antibody producing cells. Second, imortalizing the antibody producing cells by, for example fusing them with myeloma cells or infecting them with a transforming virus, thereby generating a plurality of immortalized cells, each producing monoclonal antibodies, and third, screening the plurality of monoclonal antibodies to identify a monoclonal antibody which specifically binds SP. These methods are known in the art and are therefore not further elaborated herein.

According to still another aspect there is provided a fusion protein comprising a denaturant (e.g., boiling and/or detergent) stable protease resistant polypeptide having a chaperone-like activity fused to an additional polypeptide. Preferably the fusion protein acquires an oligomeric form, with the advent that either homo- or hetero oligomeric forms can be assembled. Simultaneous display of a variety of proteins on the same SP oligomer can be achieved by reversible denaturation and re-assemble of mixtures of different fusion proteins as herein described or alternatively, by coexpression of several fusion proteins in the same cells/organism (*in vivo* assembly). Such fusion proteins can exhibit biological properties (such as substrate or ligand binding, enzymatic activity, antigenic activity, etc.) derived from each of the fused sequences. Any conventional fusion partner can be used, including, for example, beta-glucuronidase, beta-galactosidase, etc. Fusion polypeptides are preferably made by the expression of recombinant nucleic acids produced by standard techniques.

The following provides a non-exhaustive list of proteins having known genes which can be fused to a stable protein of the invention: proteins having medicinal properties: aggregating proteins such as beta amyloid, messenger proteins such as the cytokines IL-1 and IL-7, and their receptor proteins, proteins of agents of infectious diseases, such as bacterial exported proteins from *pneumococci, streptococci* and other pathogenic strains, proteins from pathogenic viruses such as hepatitis B and transmissible gastroenteritis, and from protozoa and helminths in parasitic infections; non-infectious diseases, such as poorly antigenic autologous tumor cell proteins or any of their epitopes, interferons and their receptor proteins in the case of autoimmune diseases, proteins useful in research, including protein or polypeptide reagents for immuno-assays such as insulin, gastrin, opiods, growth factors, calcitonin, malarial and other protozoan blood-stage antigens, enzymes such as peroxidase and heat or detergent labile biologically active proteins, including enzymes and proteins useful in commercial applications, e.g., proteases, glycosil-hydrolases and lipases, heterologous proteins aggregating in transformed cells or their culture media such as growth factors, glycosil-hydrolases, peroxidases, transferases, kinases, phosphatases, sulfatases, nucleic-acid-modifying enzymes (ligases, restriction enzymes, reverse-transcriptase, nucleic ccid polymerases).

A fusion protein is obtainable by either genetic engeneering techniques by which two open reading frames are fused into a single nucleic acid creating a continous reading frame, the translation thereof in an expression system yields the fusion protein, or via chemical fusion or linking of pre-existing proteins, using anyone of a plurality of linking reagents known in the art for linking or joining proteins.

Hence, many methods are known in the art to conjugate or fuse (couple) molecules of different types, including proteins or polypeptides. These methods can be used according to the present invention to couple a stable protein with any other protein. Two isolated peptides can be conjugated or fused using any conjugation method known to one skilled in the art. One peptide can be conjugated to another using a 3-(2-pyridyldithio)propionic acid N-hydroxysuccinimide ester (also called N-succinimidyl 3-(2pyridyldithio) propionate) ("SDPD") (Sigma, Cat. No. P-3415), a glutaraldehyde conjugation procedure or a carbodiimide conjugation procedure.

*SPDP conjugation -* Any SPDP conjugation method known to those skilled in the art can be used. For example, in one illustrative embodiment, a modification of the method of Cumber et al. (1985, Methods of Enzymology 112: 207-224) as described below, is used. A first peptide (1.7 mg/ml) is mixed with a 10-fold excess of SPDP (50 mM in ethanol) and the seconf peptide is mixed with a 25-fold excess of SPDP in 20 mM sodium phosphate, 0.10 M NaCl pH 7.2 and each of the reactions incubated, e.g., for 3 hours at room temperature. The reactions are then dialyzed against PBS. The first peptide is reduced, e.g., with 50 mM DTT for 1 hour at room temperature. The reduced peptide is desalted by equilibration on G-25 column (up to 5 % sample/column volume) with 50 mM KH₂PO₄ pH 6.5. The reduced peptide is combined with the SPDP-secong peptide in a molar ratio of 1:10 second peptide:first peptide and incubated at 4 °C overnight to form a peptide-peptide conjugate.

*Glutaraldehyde conjugation* - Conjugation of a peptide with another peptide can be accomplished by methods known to those skilled in the art using glutaraldehyde. For example, in one illustrative embodiment, the method of conjugation by G.T. Hermanson (1996, "Antibody Modification and Conjugation, in Bioconjugate Techniques, Academic Press, San Diego) described below, is used. The peptides (1.1 mg/ml) are mixed at a 10-fold excess with 0.05 % glutaraldehyde in 0.1 M phosphate, 0.15 M NaCl pH 6.8, and allowed to react for 2 hours at room temperature. 0.01 M lysine can be added to block excess sites. After-the reaction, the excess glutaraldehyde is removed using a G-25 column equilibrated with PBS (10 % v/v sample/column volumes).

*Carbodiimide conjugation -* Conjugation of a peptide with another peptide can be accomplished by methods known to those skilled in the art using a dehydrating agent such as a carbodiimide. Most preferably the carbodiimide is used in the presence of 4-dimethyl aminopyridine. As is well known to those skilled in the art, carbodiimide conjugation can be used to form a covalent bond between a carboxyl group of peptide and an hydroxyl group of one peptide (resulting in the formation of an ester bond), or an amino group of the one peptide (resulting in the formation of an amide bond) or a sulfhydryl group of the one peptide (resulting in the formation of a thioester bond). Likewise, carbodiimide coupling can be used to form analogous covalent bonds between a carbon group of one peptide and an hydroxyl, amino or sulfhydryl group of the other peptide. See, generally, J. March, Advanced Organic Chemistry: Reaction's, Mechanism, and Structure, pp. 349-50 & 372-74 (3d ed.), 1985. By means of illustration, and not limitation, the peptide is conjugated to another via a covalent bond using a carbodiimide, such as dicyclohexylcarbodiimide. See generally, the methods of conjugation by B. Neises et al. (1978, Angew Chem., Int. Ed. Engl. 17:522; A. Hassner et al. (1978, Tetrahedron Lett. 4475); E.P. Boden et al. (1986, J. Org. Chem. 50:2394) and L.J. Mathias (1979, Synthesis 561).

It is shown herein that the stable protein of the invention oligomerises. It is further shown herein that a fusion protein which comprises the stable protein of the invention and an additional protein similarly oligomerizes. This feature can serve several purposes including increasing the binding avidity of a binding molecule, and generating heterocomplexes which can serve different functions.

Hence, according to another aspect there is provided a method of increasing a binding avidity of a binding molecule. The method according to this aspect comprises displaying multiple copies of the binding molecule on a surface of an oligomer of a denaturant stable and/or protease resistant protein, the denaturant stable and/or protease resistant protein having a chaperone-like activity. The binding molecule, can be, for example, a receptor, a ligand, an enzyme, a substrate, an inhibitor, an antibody and an antigen. In cases where the binding molecule is a binding protein, the binding protein can be fused to the oligomer units via either genetic engeneering techniques or chemical cross linking. In cases where the binding molecule is not a protein, the binding molecule can be fused or linked to the oligomer units via chemical cross linking techniques.

It is also provided a hetero complex which comprises an oligomer including a plurality of a denaturant stable and/or protease resistant protein, the denaturant stable and/or protease resistant protein having a chaperone-like activity, and at least two different molecules which are fused to the oligomer. The at least two different molecules may comprise at least a first enzyme and a second enzyme. The first enzyme and the second enzyme may catalyze sequential reactions in a synthesis or degradation pathway. The first enzyme and the second enzyme may catalyze different reactions in a synthesis or degradation pathway. In another embodiment, the at least two different molecules comprise at least a binding molecule and a reporter molecule, such as GFP or HRP.

In cases where the molecules are proteins, the proteins can be fused to the oligomer units via either genetic engeneering techniques or chemical cross linking. In cases where the molecules are not proteins, the molecules can be fused or linked to the oligomer units via chemical cross linking techniques.

One of the uses of such fusion proteins emerges from the fact that the stable proteins retain their activity and oligomerability also when in context of a fusion protein. Interestingly, under such conditions, the counterpart fused to the stable protein also retains its activity, as is demonstrated in the Examples section that follows by the fusion CBD-SP1. As such, an oligomerized fusion protein of the invention can serve to better present the counterpart fused to the stable protein for immunization or surface reactions.

Thus, according to yet an additional aspect there is provided a method of immunization comprising subjecting an immune system of a mammal to the fusion protein described herein.

It was uncovered that immunization with an SP1-polypeptide fusion protein reduces the immune response to the polypeptide. Hence, according to yet another aspect of the invention, there is provided a method of administering to an animal having an immune system a polypeptide, while reducing an immune response against the polypeptide. The method according to this aspect is effected by administering the polypeptide to the animal, the polypeptide being fused to a denaturant stable and/or protease resistant protein, the denaturant stable and/or protease resistant protein having a chaperone-like activity, thereby reducing the immune response against said polypeptide, as compared to an immune response that develops by administering to the animal the polypeptide alone.

In an in vitro assay it was shown that SP1 induces faster coverage of scraped regions of fibroblast cells in a petri dish.

Hence, according to another aspect, there is provided a method of increasing cell migration. The method according to this aspect is effected by exposing the cells to an amount of a denaturant stable and/or protease resistant protein, the denaturant stable and/or protease resistant protein having a chaperone-like activity, sufficient for increasing cell migration.

As cell migration is essential for wound healing, there is also provided a method of accelerating wound healing effected by administering onto a wound an amount of a denaturant stable and/or protease resistant protein, the denaturant stable and/or protease resistant protein having a chaperone-like activity, sufficient for accelerating wound healing. There is also further provided a method of inducing wound healing effected administering onto a wound an amount of a denaturant stable and/or protease resistant protein, the denaturant stable and/or protease resistant protein having a chaperone-like activity, sufficient for inducing wound healing.

It is shown in the Examples section below that hair is strengthenes via administration of SP1.

Hence, according to another aspect there is provided a method of strengthening and/or grooming hair, nail or skin. The method is effected by administering onto the hair, nail or skin an amount of a denaturant stable and/or protease resistant protein, the denaturant stable and/or protease resistant protein having a chaperone-like activity, sufficient for strengthening and/or grooming the hair, nail or skin.

The polypeptides can be formulated into pharmaceutical (including cosmetical and cosmoceutical) compositions that comprise, as an active ingredient, a denaturant stable and/or protease resistant protein, the denaturant stable and/or protease resistant protein having a chaperone-like activity, and a pharmaceutically acceptable carrier, approved for use in humans or for veterinary use by an appropriate regulatory agency such as the Food and Drug Administration in the United States of America. For use in wound healing, the pharmaceutical composition is packaged in a package and identified in print for use in a wound healing application. For use in strengthening/grooming hair, nail or skin, the pharmaceutical composition is packaged in a package and identified in print for use in a strengthening and/or grooming hair, nail or skin application. Additional ingredients can be used in such compositions. For example, the stable protein of the invention can be added to hair, skin or nail grooming compositions such as soaps, shampoos, conditioners, creams, gels, sprays, lacs, etc., the other ingredients thereof are well known in the art and are typically listed on the containers of such products.

Additional objects, advantages, and novel features will become apparent to one ordinarily skilled in the art upon examination of the following examples, which are not intended to be limiting. Additionally, each of the various embodiments and aspects as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### MATERIALS AND EXPERIMENTAL METHODS

***Purification of plant-derived boiling-stable proteins:*** Boiling stable protein fractions of aspen, tomato M82, VF36 and pine were prepared as follows: Crude plant extracts were centrifuged at 10,000 g for 10 minutes and supernatants were transferred to fresh tubes. The supernatants were subjected to a 10-minutes boiling session, then kept on ice for 5 minutes and centrifuged for 10 minutes at 10,000 g. Resulting supernatants were precipitated by adding 4 volumes of cold acetone, and centrifuged for 10 minutes at 10,000 g. Boiling stable proteins were then recovered by dissolving the pellets in 10 mM Tris-HCl buffer (pH 7.5). Total protein concentration was determined as for SP1 preparations (see below).

Only when the total boiling-stable proteins were separated on a 17 % SDS-tricine PAGE, a 66 kDa band that appears using the electrophoresis condition described by Pelah (1995) separated as two bands of 66 and 116 kDa. The 66 kDa band was found to represent a germin-like protein.

***Purification of plant SP1 protein:*** Acetone-precipitated boiling-stable proteins of aspen plant prepared as described above were dissolved in 1X tricine-SDS sample buffer (100 mM Tris-HCl, pH 6.8, 20 % glycerol, 1 % SDS, 0.025 % Coomassie blue G-250), then separated on a preparative 17 % polyacrylamide tricine-SDS gel. Major bands corresponding to SP1 (116 kDa oligomer and 12.4 kDa monomer) protein were excised from the gel. SP1 oligomer and monomer were electro-eluted separately, in a dialysis bag. The eluent was further dialyzed against 500 volumes of 10 mM Tris-HCl (pH 7.5) overnight at 4 °C, followed by acetone precipitation and centrifugation. Purified SP1 was obtained by dissolving the pellet in 10 mM Tris-HCl (pH 7.5). Protein concentration was determined using the BIO-RAD protein assay kit (Hercules, CA, USA) employing bovine serum albumin as the standard.

***Proteolytic resistance and non-detergent purification of SP1:*** Aspen plant shoots or leaves were homogenized in liquid nitrogen in 50 mM Tris-HCl (pH 7.0) and 2 % PVPP. The extract was centrifuged at 10,000 g for 15 minutes at 4 °C, and the total soluble protein fraction was recovered from the supernatant fraction. Soluble proteins were further concentrated with 4 volumes of cold acetone, and then redissolved in 1X protease digestion buffer (20 mM Tris- HCl, pH 7.5, 1 mM EDTA, 50 mM NaCl). Proteolysis was initiated by the addition of Proteinase K to a final concentration of 50 µg per milliliter, and continued for 60 minutes at 37 °C. The proteolysis was terminated by addition of 1 mM PMSF to the mixture, incubation at room temperature for 30 minutes, and 10 minutes boiling. Aggregates were removed by 10 minutes high-speed centrifugation, and the supernatant, which was highly enriched in SP1, was concentrated under pressure using a 50-kDa cut-off concentrator.

***Generation of polyclonal antibodies**:* Gel-purified native SP1 or recombinant CBD-SP1 (50 µg per injection) were injected to rabbits with complete Freuid's adjuvant. Two additional boosts (at 14 days intervals) were injected and 14 days later anti-serum was collected.

***cDNA cloning**:* Polyadenylated (poly A+) RNA extraction was performed according to Bartels and Thompson (1983) from water-stressed aspen shoots, and the mRNA was used as a template for cDNA synthesis. A lambda ZAPII (Stratagene, La Jolla, CA, USA) cDNA library was constructed according to the supplier's instructions, and immuno-screened with SP1 polyclonal antibodies raised against the natural protein as described above (diluted 1:500, v/v). *In vivo* excision was performed according to the supplier's instructions and the sequence was determined (Sequencing Lab, The Weizmann Institute of Science, Rehovot, Israel).

***Generation of a CBD-SP1 fusion protein in E. coli:*** SP1 cDNA was cloned into pET-CBD-180 (Shpigel et al., 1999) expression vector by subcloning therein a PCR product generated using two amplification primers carrying an *Nco*I site (forward primer) 5'-AAAACCATGGCAACCAGAACTCCAAAGC-3' (SEQ ID NO:3) and a *Bam*HI site (reverse primer) 5'-AAAAGGATCCTTACTTTATTACCATGAAATAGCC-3' (SEQ ID NO:4) for amplification of the corresponding ORF of SP1 cDNA. The resulting plasmid (pET-CBD-180-SP1) was used to transform *E. coli* strain BL21 (DE3). Recombinant CBD-SP1 fusion protein synthesis was induced in BL21 (DE3) by the addition of IPTG (isopropyl-D-thiogalactoside) to a final concentration of 1 mM to mid-log phase of the bacterial culture, followed by five additional hours induction at 37 °C. Recombinant CBD-SP1 protein was purified on cellulose according to Shpigel et al. (1999). The recombinant CBD-SP1 fusion protein was detected using SDS-PAGE.

### Generation and secretion of recombinant SP1 by Pichia pastoris:

A DNA fragment of SP1 protein coding region was cloned in-frame at the *Eco*RI and *Not*I restriction sites of the secretory *Pichia pastoris* expression vector pPIC9K (Invitrogen®, Groningen, The Netherlands) to generate pPIC9K-SP1. The construct sequence was confirmed by sequencing (Sequence lab, Weizmann Institute, Rehovot, Israel). In order to transform *Pichia pastoris* cells, pPIC9K-SP1 was linearized by *Sal*I or *Bgl*II restriction enzymes. The linearized constructs were each independently used to transform *Pichia* competent cells by electroporation, according to supplier's instruction (Invitrogen®, Groningen, The Netherlands). To this end, 5-10 µg of *Sal*I or *Bgl*II linear pPIC9K-SP1 DNA were used to transform *Pichia* SMD1168, a protease deficient mutant, His⁺, Mut⁺ (Methanol utilization plus) phenotype stain. Transformed competent cells were plated onto RDB plates and incubated at 30 °C. Five days later, 240 colonies from *Sal*I (Mut⁺) and 180 colonies from *Bgl*II (Mut^{s}: Methanol utilization slow) transformants were first transferred to YPD plates containing 0.25 mg/ml G418 antibiotics; 73 % and 16 % of Mut⁺ and Mut^{s} transformants survived. For Mut⁺ transformants, surviving colonies were further transferred to YPD plates containing a higher concentration of G418. Mut^{s} transformants were transferred to MM plates. To select the high expression level transformants, 2 Mut⁺ transformants from 4 mg per ml and 2 from 1 mg per ml G418, respectively and 4 Mut^{s} colonies were used in a small volume expression system according to the manual for expression of recombinant proteins in Pichia pastoris (Invitrogen®). The screening of high-copy-number transformants and expression of recombinant SP1 were performed according to the instructions in the manual of *Pichia Pastoris* (Invitrogen®). The secreted recombinant SP1 was detected from the culture medium by SDS-PAGE. The gels were either stained with Coomassie blue for total protein visualization, or blotted onto nitrocellulose (Western blots) for immunodetection of SP1 with polyclonal anti-SP1.

***Gel filtration, HPLC and native SP1 detection:*** An HPLC system (Merck, Hitachi) equipped with a TSKSWX3000 (30 cm x 7.8 mm) column (SUPELCO, Sigma, Israel) was employed to study the size of SP1 in its native state. A 100 µl aliquot of total soluble proteins extract from water-stressed aspen plants, or the total boiling-stable fraction of the same extract was separated using PBS buffer at pH 6.6. The flow rate was adjusted to 0.8 ml per minute and a UV monitor was used at 280 nm to detect elution of proteins from the column. Fractions were collected every minute. Each fraction was further concentrated by adding four volumes of cold acetone, followed by 10 minutes centrifugation at 10,000 g. The resulting pellets were dissolved in 1 X SDS-sample buffer. An aliquot was separated on 17 % tricine-SDS-PAGE, and the resultant protein profiles were either visualized by Coomassie staining or Western blot analysis using anti-recombinant SP1 antibodies (see above). Purified native SP1 and recombinant CBD-SP1 (50 µl aliquots) at a concentration of 1 milligram per milliliter and 0.5 milligram per milliliter were also analyzed. To determine the size of the protein, cytochrome C (12.4 kDa), carbonic anhydrase (29 kDa), bovine serum albumin (66 kDa), alcohol dehydrogenase (150 kDa), beta-amylose (200 kDa) and apoferritin (443 kDa) (Sigma-Aldrich Israel Ltd.) were used as molecular standards. Blue dextran (2000 kDa) was used to evaluate the void volume of the column. A linear relationship was obtained by plotting the logarithms of the molecular weights of standard proteins against their respective elution parameters (Kav). The Kav value was calculated using the equation: Kav = (Ve-Vo)/(Vt-Vo), where Ve = elution volume of the protein, Vo = column void volume, Vt = total packed bed volume.

***SP1 stability following exposure to SDS and heating**:* For evaluating the stability of SP1 complexes to detergents, equal amounts of purified SP1 protein were prepared in a sample buffer containing SDS at a final concentration ranging from 0 % (native sample buffer) to 2 % (conventional Laemmli sample buffer), and corresponding to a final molar ratio of 1:0, 1:200, 1:400, 1:500, 1:600 or 1:4334 (SP1 monomer:SDS). The samples were boiled (or not boiled) for 5 minutes prior to separation on a 17 % tricine-SDS-gel. To examine the stability of SP1 oligomer to heating, SP1 was prepared in SDS sample buffer at a final molar ratio of 1:900 (SP1 monomer:SDS) and was heated for 5, 10 or 20 minutes at a range of temperatures from room temperature to 100 °C before separation on SDS- tricine PAGE.

***In vitro assay of thermal stabilization by SP1*** : The heat-protective activity of SP1 was examined *in vitro* by measuring the effect of SP1 on the thermal stability of Citrate Synthase (CS) and Horseradish Peroxidase (HRP) enzymatic activity .

***Protein preparation:*** CS (Roche Diagnostics GmbH, Mannheim, Germany) was prepared according to the method of Buchner et al. (1998). HRP, BSA, lysozyme (SIGMA, Israel) and CBD (CBD-Technologies Ltd. Rehovot, Israel) were dissolved in water to about 10 mg/ml, then dialyzed overnight against 200 volumes of 40 mM HEPES-KOH buffer (pH 7.5) at 4 °C. After dialysis, proteins were centrifuged at 13,000 rpm for 15 minutes at 2 °C to remove any insoluble particles. 20 µM HRP, 60 µM of BSA and lysozyme stock solution was prepared in filtered HEPES buffer and aliquoted. Aliquots were stored at -20 °C. Thawed aliquots of proteins were discarded after use. The protein concentration was determined as for SP1. Lyophilized alpha-crystallin (Stressgen Inc., Canada) was resuspended in water according to supplier's instruction.

***CS and HRP activity assay:*** Enzyme activity assays were performed at 25 °C, in an ELISA plate. The colorimetric reaction was recorded by a microplate reader (BIO-RAD,) at 412 nm for CS, and 650 nm for HRP.

CS activity assay was according to the method of Buchner et al. (1998) with a slight modification: the volume of the reaction components was proportionally reduced for the ELISA plate volume. Briefly, 4 µl of 0.15 µM CS was mixed with 200 µl of reaction mixture composed of TE buffer (50 mM Tris, 2 mM EDTA, pH 8.0), 100 µM oxaloacetic acid (in 50 mM Tris, pH not adjusted), 100 µM DTNB (in TE buffer), and 150 µM acetyl-CoA (in TE buffer). The change in absorbency was recorded in 20-second intervals for 1 minutes. The linear portion of the plot was used to calculate the specific activity of the CS. CS activity was expressed as µmol per minute per mg (defined herein as activity unit) using a molar extinction coefficient of DTNB of 1.36 x 10⁻⁴ M per cm.

***Sensitive one-step TM Slow TMB-ELISA:*** TMB (3,3',5,5'-tetramethylbenzidine; PIERCE, Rockford, USA) was used as substrate for HRP activity assay in the experiments. An optimal colorimetric reaction of HRP was determined experimentally. The linear portion of the graph representing absorbency vs. time was used to calculate the rate of change in absorbency at 650 nm. Optimal reaction conditions were determined to be 4 µl of 2.5 nM HRP in 100 µl of TMB substrate at 25°C. The reaction was recorded at 30-second intervals for 5 minutes. HRP activity was expressed as µmol per minute per mg (defined herein as enzyme activity unit) by using a molar absorption coefficient for blue charge-complex of 3.9 x 10⁻⁴ M per cm (Josephy et al., 1982).

***Heat inactivation of CS and HRP:*** A 100 µl aliquot of 0.15 µM CS or 2.5 nM of HRP prepared in pre-chilled 40 mM HEPES buffer, pH 7.5, was heated in the absence or presence of proteins (SP1, CBD-SP1, BSA, lysozyme, and other plant boiling stable proteins (see above)) using a programming T-gradient thermocycler (Biometra, Gottingen, Germany) for desired temperature and length of time. Aliquots were removed for enzyme activity assay during the course of the heat challenge.

The degree of protection conferred by the specific protein at each time point was expressed as % remaining activity of the full enzyme activity. Each point represents at least 4 replicates. Data were analyzed by JMP (version 3.11) program.

***Stability of recombinant SP1 from Pichia pastoris:*** Culture medium containing secreted recombinant SP 1 was boiled for 10 minutes, followed by 10 minutes centrifugation at 10,000 g. Supernatant samples were prepared in either full strength SDS (2 %) sample buffer or native sample buffer (0 % SDS). Samples were boiled in sample buffers for 5 minutes before separation on 17 % tricine-SDS-PAGE.

***Transmission electron microscopy (TEM) study:*** Purified native SP 1 at a concentration of 0.45 mg per ml was applied to carbon grids and stained with uranyl acetate. The images were visualized in a Philips CM12 EM and recorded on a Tietz CCD camera (Dr. Sharon Wolf, Electron Microscope Center, Weizmann Institute of Science, Rehovot, Israel).

***Additional Experimental procedures:*** Additional methods and procedures are described in detail under the brief description of the drawings in context of specific Figures.

### EXPERIMENTAL RESULTS

***Stability of native SP1 oligomer to heat and detergent denaturation:*** SP1 from aspen plants was first identified as a large size protein on SDS-PAGE, appearing as a complex in the total soluble proteins extract. When partially denatured, a large (116 kDa) and small molecular size (12.4 kDa) form of the protein are detected (Figure 1). These two forms represent the monomeric (12.4 kDa) and native homo-oligomeric (116 kDa) states of the SP1 protein, as demonstrated by the interconversion of gel-purified samples of the two forms under extreme denaturing conditions (Figure 1).

The remarkable resistance of the native SP1 oligomer to denaturation by detergent was examined throughout a range of SDS concentrations. Despite the presence of SDS in the gel and the running buffer (0.1 %), it was found that only a small amount of monomer could be observed when SP1 was prepared in native (0 %) sample buffer (Figure 2a). The SP1 complex remained stable when boiled in SDS concentrations up to 600:1 (SDS:SP1 monomer) molar ratio, and at even at much higher SDS concentrations without boiling (Figure 2a).

Thus, the SP1 oligomer also exhibits unusual thermal stability. This was further demonstrated by the consistent stability of the oligomeric form of SP1 at temperatures up to 80 °C and 900:1 SDS: SP1 monomer concentration (Figures 2b), regardless of the length of incubation (Figure 2c).

***Protease resistance and detergent-free purification of SP1**:* The detergent-free purification and protease resistance of SP1 from aspen plant was demonstrated by cryogenic extraction (at - 50 °C) followed by 60 minutes protease K treatment of the soluble protein fraction from aspen shoots or leaves at 37 °C. Upon termination of proteolytic digestion, the predominant protein in the remaining soluble fraction was SP1. Size fractionation by molecular filtration demonstrated that the protease-resistant SP1 was greater than 50 kDa molecular mass, indicating that the resistant protein maintained oligomeric structure.

***SP1 increases the thermal stability of Citrate Synthase (CS) and Horseradish Peroxidase (HRP) enzymatic activity:*** The chaperone-like activity of SP1 was assessed *in vitro* by measuring the resistance of CS and HRP enzymatic activity to heat-denaturation in the presence of SP1. CS is a commercially available, heat-labile dimeric enzyme, undergoing inactivation after 15 minutes at 43 °C in the absence of any protectant (Figure 3a). In the presence of high concentrations of SP1 (CS:SP1 ratio of 1:50), CS activity remained nearly 100 % for 15 minutes and retained at least 93 % of its activity for the duration of the assay (40 minutes). Lower concentrations of SP1 conferred proportionally less protection against heat inactivation (at a CS:SP1 ratio of 1:5, 22 % protection was achieved at 40 minutes). In contrast to the dramatic protection afforded by SP1, neither BSA nor lysozyme affected heat inactivation of CS activity (Figure 3a). In a separate assay, the protein stabilizers glycerol (10 and 20 %) and the Hsp alpha-crystallin were equally ineffective in protecting CS enzyme activity from thermal inactivation (Figure 3c).

HRP is a commercially available monomeric protein with a molecular mass of 44 kDa. When incubated at 55 °C under standard assay conditions (see Materials and Methods), 60 % of the enzyme activity was lost after 10 minutes and more than 90 % was lost after 60 minutes. Only 3 % of original HRP activity could be measured after 2 hours at 55 °C (Figure 4). No recovery of activity was observed following exhaustive (16 hours) incubation of the heat-inactivated enzyme at 25 °C. Thus, HRP activity is heat-labile at 55 °C. When native purified SP1 was added, protection of HRP activity from heat-inactivation was significant at HRP:SP1 molar ratios of 1:50 and above. At a HRP:SP1 molar ratio of 1:300, greater than 60 % protection was achieved at 60 minutes, with 53 % activity remaining after 2 hours incubation at 55 °C. SP1 mediated protection from heat-inactivation of HRP was significant, but proportionally weaker at ratios of 200:1, 100:1 and 50:1 (Figure 4). At a 1:25 HRP to SP1 molar ratio, little protection was observed. BSA addition (HRP:SP1 ratio of 1:300) also afforded a degree of protection, but SP1 was approximately 3-fold more effective (Figure 4).

***Cloning and sequence analysis of SP1 cDNA**:* A lambda expression library was prepared from polyadenylated RNA of water-stressed aspen shoots, as described in Materials and Experimental Methods above. After screening 7 x 10⁵ recombinant phage plaques with polyclonal anti-SP1 antibodies, a 567 nucleotide cDNA sequence encoding a SP1 polypeptide (SP1 cDNA) was isolated (Figure 5 and SEQ ID NO:1 and SEQ ID NO:2 for the nucleotide and amino acid sequences of SP1, respectively). Nucleotide sequence analysis of the cDNA (Wisconsin Package Version 9.1, Genetics Computer Group-GCG, Madison WI.) indicated that the SP1 cDNA encodes a 12.368 kDa polypeptide with a calculated pI of 4.87. Analysis of the open reading frame revealed that this polypeptide lacks Cystein residues, is low in Tryptophan residues (0.9 %), and is rich in Leucine (13.8 %), Threonine (9.2 %), Alanine (8.3 %), Glutamic (7.4%) and Serine (7.4%) residues. No homology was detected with any known protein sequences in the SWISS-PROT protein bank. Coding sequences exhibiting sequence homology with SP1 from various evolutionary distant plant species were identified using the EST database (Plurality = 10.0; Threshold = 4; Average Weight = 1.00; Average Match = 2.91; Average Mismatch = -2.00). 25 sequences with significant homology (E value below 0.5) were identified (3 in Arabidopsis, 2 in maize, 1 in potato, 2 in rice, 1 in sorghum, 7 in soybean, 2 in tomato and 7 in wheat, see Figure 12 and SEQ ID NOs:7-32, Consensus Sequence - SEQ ID NO:33). The putative peptide sequences were aligned and compared with the peptide sequence of SP1 (SEQ ID NO:2), revealing a few conserved consensus sequences: "HAFESTFES" (61-75, SEQ ID NO:36), "VKH" (9-11, SEQ ID NO:37) and "KSF" (47-49, SEQ ID NO:38) for example, indicating that SP1 is a member of a family of protein genes with wide representation in both dicot and monocot plant genomes. However so far, no function has been discovered or ascribed for any of the proteins in this family, except as reported herein.

In addition to the above sequences, high DNA homology with SP1 cDNA (SEQ ID NO:1) was noted for a number of ESTs from *Populus:* 97 % homology with ESTs AI161912 (SEQ ID NO:5) and AI163063 (SEQ ID NO:6), 90 % homology with AI161643 (SEQ ID NO:39) and 92 % homology with AI163329 (SEQ ID NO:40) of a hybrid aspen (*Populus tremula x Populus tremuloides*); 96.6 % homology with *Populus trichocarpa* x *Populus deltoides* pop3 mRNA sequence (SEQ ID NOs:34 and 35 for nucleic acid and amino acid sequences, respectively, see also Figure 13, for homology alignment of the protein encoded by the pop 3 mRNA - SEQ ID NO:35, and the SP1 protein - SEQ ID NO:2), 61.6 % homology with *Populus trichocarpa* x *Populus deltoides* wound responsive mRNA (EMBL Acession Numbers M18538 and X55440, respectively). The SP1 protein was identified in all of the *Populus* species studied. The SP1 DNA nucleotide sequence was submitted to EMBL (under Accession Number AJ276517). Analysis of the polypeptide encoded by SP1 using Kyte and Doolittle (1984) and Goldman et al. (1986) hydropathy plots indicated that SP1 is a highly hydrophilic protein, except for it's hydrophobic C-terminus.

***SP1 expression in E. coli, purification of recombinant CBD-SP1 fusion protein, and the antigenic character of recombinant CBD-SP1 protein:*** Introduction of the cloned SP1 cDNA sequence into the pET-CBD-180 CBD expression vector (Figure 11, as described in Materials and Experimental Methods) resulted in a nucleotide sequence which encoded a CBD-SP1 fusion protein. Recombinant CBD-SP1 was expressed at high levels by the bacteria (approximately 300 milligrams per liter culture medium) and accumulated as inclusion bodies. When total *E. coli* extract was separated on SDS-PAGE, a 32.4 kDa band was detected by Coomassie blue staining, apparently absent from the un-transformed bacterial protein fraction (Figure 6a). The antigenic identity of the fused protein with SP1 was demonstrated by a strong reaction upon immunodetection of the 32.4 kDa fused monomeric protein band on Western blots of the same gels, using the polyclonal anti-SP1 antibodies (Figure 6b). An immunoreactive 65 kDa band was also detected on the SDS-PAGE of total transformed bacterial protein, possibly representing a dimer of the 32.4 kDa fusion protein (Figure 6b). Recombinant CBD-SP1 fusion protein was purified on cellulose beads from 4.5 M urea-solubilized inclusion bodies, taking advantage of the affinity of CBD to cellulose beads. The highly purified CBD-SP1 fusion protein obtained was used to prepare polyclonal anti-CBD-SP1 antibodies in rabbits. These polyclonal anti-CBD-SP1 antibodies also recognized 32.4 kDa and 65 kDa CBD-SP1 protein bands on Western blots of transformed cell extracts, further confirming the antigenic identity of the recombinant CBD-SP1 and native SP1 polypeptides. The molecular weights of purified native SP1 protein and recombinant CBD-SP1 protein under non-denaturing conditions (PBS buffer) were estimated by gel-filtration HPLC and immunodetection of the eluted protein fractions on Western blots with anti-SP1 and anti-CBD-SP1 antibodies. Both the native SP1 and the recombinant CBD-SP1 proteins eluted as single peaks, at about 9.8 and 9.2 minutes, respectively (Figure 7). These peaks corresponded to molecular weights of 172.5 + 1.25 kDa and 267.5 ± 2.5 kDa, representing a complex of 14 units (13.9) of SP1 monomer (12.369 kDa) and 8.4 units of CBD-SP1 monomer (32.4 kDa), respectively. Naturally, the number of subunits can only be estimated since the results are influenced by the shape of the complex.

***Cloning of SP1 DNA in Pichia pastoris and secretion of recombinant SP1 protein:*** Recombinant, non-fused SP1 secretory protein was generated by transforming *Pichia* SMD1168 (a protease deficient mutant, His+, Mut +) cells with SP1 DNA from *Sal*I*-* or *Bgl*II linearized pPIC9K plasmids as described in Materials and Methods. High levels of SP1-expression were induced and maintained in the transformed cells by the addition of methanol to the culture for 96 hours. One Mut⁺ and one Mut^{s} transformant were found to express and secrete relatively high levels of recombinant SP1, absent from the control cell culture media, as verified by SDS-PAGE (Figure 8) and immunodetection on Western blot with anti-SP1 antibody.

*S**DS- and Heat stable properties of recombinant SP1 protein:*** Recombinant SP1 protein from the culture medium of transformed cells was exposed to extreme of heat and SDS concentrations in order to determine the functional similarity of the recombinant and native polypeptide (Figure 8). Separation of heat- and SDS-treated culture medium on SDS-PAGE demonstrates that, as with native SP1, the recombinant SP1 oligomer is boiling resistant, dissociating to the monomeric form only in the presence of high concentrations (2 %) of SDS (Figure 8).

***Recombinant CBD-SP1 fusion protein increases the thermal stability of CS**:* The ability of recombinant CBD-SP1 fusion protein to protect against thermal inactivation of citrate synthase enzymatic activity was demonstrated employing the CS colorimetric assay (as described in Materials and Methods). Like the native SP1 protein, purified recombinant CBD-SP1 conferred significant, concentration-dependent protection against thermal inactivation of CS enzymatic activity at 43 °C (Figure 3b). After 40 minutes, 73 % activity remained at CS:CBD-SP1 monomeric molar ratio of 1:20. At a ratio of 1:5, 33 % of the enzymatic activity was retained, compared to the controls. In contrast to this, incubation with high concentrations of non-fused CBD protein (Figure 3b), BSA or lysozyme protein (Figure 3a) had no protective effect on the inactivation of CS. Incubation with other protein stabilizers, such as glycerol (10 or 20 %) or the Hsp alpha-crystallin (1:12.5 CS:alpha-crystallin ratio) was also without effect on CS inactivation (Figure 3c). Thus, the portion of the SP1 protein encoded by the cloned sequence retains the thermally protective properties of the native protein.

***Boiling stable proteins from plants protect against thermal inactivation of CS enzyme activity:*** The existence of SP1-like proteins in other plant species was investigated by assaying the effect of boiling-stable protein fractions from tomato and pine (which are evolutionary distant plants) on heat-inactivation of CS enzymatic activity. Total boiling-stable proteins from tomato M82, tomato VF36 and pine plants were prepared (as described under Materials and Methods), and compared with crude Aspen boiling-stable protein fractions for their thermal stabilizing effect on CS enzymatic activity at 43 °C. Significant protection against thermal inactivation (greater than 60 % activity remaining after 40 minutes) was demonstrated by the tomato and pine boiling-stable fractions (Figure 9).

***Immune cross reactivity, stress responsiveness and oligomeric structure of SPs from Pine and Tomato:*** Antigenic cross reactivity of stable proteins from phylogenetically remote species was investigated by Western blotting and immune detection with anti-SP1 antibodies. Total boiling stable proteins from salt- and drought stressed tomato leaves, and temperature- and drought stressed pine material was prepared (as described under Material and Experimental Methods above), separated on SDS PAGE, blotted onto nitrocellulose and immune reacted with either anti-native oligomeric SP1 antibodies or anti recombinant SP1 antibodies (anti-CBD-SP1). Cross reactive proteins were detected in blots of both tomato (Figures 14c and 14d) and pine extracts (Figures 14a and 14b), with a predominant, stress-responsive band at 45-50 kDa

***Characterization of native SP1 molecular structure by Electron Microscopy:*** The molecular structure of native SP1 was examined using Transmission Electron Microscope (Materials and Methods). These TEM studies of purified SP1 protein indicated a ring-like protein with a central cavity. The entire structure diameter is approximately 11 nanometers (Figure 10).

***Protection of α-Amylase by SP1:*** In addition to HRP and CS protection by SP1, it is shown herein that SP1 can be used to protect α-amylase from inactivation induced by both high CaCl₂ concentrations (known as salt denaturation) and upon incubation for extended time periods at room temperature. As shown in Figure 15, CaCl₂ at high concentration inactivates α-amylase; after 2 hours incubation at 1 M and 2 M CaCl₂ α-amylase activity was dropped to less than 60 % and to less than 10 %, respectively. SP1 treated enzyme was fully protected in the presence of 1 M CaCl₂ and 50 % protected in the presence of 2 M CaCl₂. Long incubation of diluted α-amylase solution at room temperature also caused a dramatic loss of enzyme activity. As shown in Figure 16, only about 25 % activity remained after one-week incubation at room temperature. However in the presence of SP1, more than 40 % activity remained following one-week incubation at room temperature. Thus, SP1 protects α-amylase from inactivation induced by both high CaCl₂ and a long incubation of diluted enzyme solution at room temperature.

***Repair of enzyme activity by SP1**:* The ability of SP1 to repair enzyme activity was evaluated with respect to the enzymes α-amylase, SOD and HRP.

***Repair of α-amylase activity by SP1:*** As shown in Figure 17, addition of SP1 to α-amylase, resulted in a 60 % increase in α-amylase activity compared to the enzyme without SP1. This result clearly indicates that SP1 repairs α -amylase that lost partial activity during storage or activity assay.

***Repair of horseradish peroxidase (HRP) activity by SP1**:* Diluted HRP is readily inactivated upon exposure to room temperature. As shown in Figure 18, over 35 % of HRP activity was lost upon 30 minutes exposure to room temperature. As is further shown in Figure 18, SP1 not only protects HRP from room temperature induced inactivation, it also repairs damaged HRP, as about 10 % of HRP activity was rescued upon SP1 addition, and was maintained for at least 6 hours thereafter. This is in sharp distinction to the SP1 untreated HRP that continued to lose activity throughout the experiment.

***Repair of superoxide dismutase (SOD) activity by SP1:*** The repair activity of SP1 was also evaluated with respect to SOD. As is shown in Figure 19, addition of SP1 to cosmetic grade SOD (Pentapharm), resulted in a 60 % higher activity compared to SP1 untreated SOD. The repair activity is concentration dependent and demonstrates that SP1 can repair SOD that lost partial activity during storage or assay.

***Reduced immune response as a result of fusion of a polypeptide with SP1**:* 16 mice (C57BL/6) were injected peritoneally (100 µl) with either CBD {5 µM (mice 1-4), 0.05 µM (mice 9-12)} or CBD- SP1 fusion protein {5 µM (mice 5-8), 0.05 µM (mice 12-16)}. As shown in the Figure 20a, 35 days post immunization, blood titer of anti-CBD antibody in mice injected with CBD-SP1 fusion was far lower than blood titer of anti-CBD antibody in mice injected with CBD alone. The difference between antibody titer of mice injected with CBD and mice injected with CBD-SP1 fusion is even larger when the mice were immunized with lower amounts of antigen and after shorter time from injection (Figure 20b(i)-(iv)).

***SP1 confers salt tolerance in plants:*** Insertion of abiotic stress tolerance genes to plants is used to develop stress-tolerant crops. The effect of SP1 protein expression levels on salt tolerance was tested in SP1-transgenic aspen (P. tremula) lines. NT (non-transformed plant) plants as well as M4 and L3 transgenic plants express normal level of SP1-protein, whereas H3 transgenic plants express a considerable higher level of SP1 protein. Stem length, leaf retention and final dry weight of plant organs were measured in P. tremula plants (NT) and in the three SP1-transformed P. tremula lines, following salt stress and recovery from salt stress, in pot experiment, relative to normal irrigation regime (Figures 21 a-c). A severe growth suppression was observed as a result of salt stress. However, H3 plants, which express a considerably higher level of SP1 protein, show much better tolerance to salt stress than plants which express normal or low SP1 levels. The beneficial effect of high SP1 levels during the recovery period was even more clear: H3 plants recovered from salt stress much better than the other lines. It is important to note that no significant difference between the different lines was observed under normal irrigation regiments.

***SP1 induces wound healing:*** As shown in Figure 22, SP1 stimulated the migration of denuded area scratched in a confluent monolayer, indicating a positive effect of SP1 in wound healing processes.

***Effect of SP1 on hair strength:*** Hair is composed of proteins such as mostly keratin and hence SP1 may stabilize and strengthen the hair. Hair strength was tested by measurement of its ability to carry weight, and was defined as the weight above which it was torn. Because hair strength varies, even among the same donor, each hair was cut into two fragments, one fragment was treated with Tris buffer and the other with the same buffer containing SP 1. The strength of each individual fragment was compared with the strength of the other. As shown in Figure 25, the average strength of the SP1 treated hair was 16 %, significantly, higher than that of control untreated hair. Thus SP1 treatment strengthens human hair.

***SP1 serving as a molecular scaffold:*** The fusion between SP1 and cellulose binding domain (CBD) was used to demonstrate that fusion of SP1 with a polypeptide maintains the characteristics of both components. It was demonstrated that recombinant CBD-SP1 fusion maintains the ability to assemble spontaneously into a 12-mer oligomer as SP1 does, it maintain the cellulose binding ability as CBD does, and can stabilizes HRP as SP1 does. Figure 7 shows a size exclusion HPLC profile of both SP1 and CBD-SP1. Both spontaneously assemble into a 12-mer oligomer (Figure 10). Figures 23a compares the binding ability of CBD-SP1 to cellulose with that of CBD. Equimolar amount of CBD and CBD-SP1 proteins (first two lanes from left; 15 pmol, calculated based on CBD molecular weight) were applied to 30 mg of cellulose (Sigmacell type 20). The same binding and elution procedures were carried out for these two proteins. Similar to CBD, CBD-SP1 bound to the cellulose, and was eluted under the same conditions. The HRP protection activity of both CBD-SP1 and SP1 is shown in Figure 23b. It is evident that CBD-SP1 stabilizes HRP as SP1 does (note that the molecular weight of CBD-SP1 is about two-fold higher than that of SP1). Thus, these results demonstrate that the fusion of SP 1 with CBD maintains the characteristics of both SP1 and CBD.

***SP1 Production:*** SP1 extraction and purification from both fresh aspen leaves and *sp1*-transformed bacteria takes advantage of the protein resistance to boiling and proteases. As shown in Figures 24a(i)-(ii) and 24b, most proteins present in crude extract of both fresh aspen leaves and *sp1*-transformed bacteria are removed by either boiling or proteolysis by Subtilisin, but SP1. The predominant protein found after such treatment is SP 1.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### References Cited

### (Additional references are cited in the text)

1. Bartels, D. and Thompson, R.D. (1983) The characterization of cDNA clones coding for wheat storage proteins. Nucl. Acids Res., 11, 2961-2978.
2. Buchner, J., Grallert, H., and Jakob, U. (1998) Analysis of chaperone function using citrate synthase as nonnative substrate protein. pp. 323-338. In: Methods in Enzymology, Vol, 290 (Molecular Chaperones) George H. Lorimer and Thomas O. Baldwin (Edt) Academic Press, New York, USA
3. Chen, Q., Osteryoung, K., and Vierling, E. (1994) A 21-kDa chloroplast heat shock protein assembles into high molecular weight complexes in vitro and in organelle. J. Biol. Chem. 269, 13216-13233.
4. Collada, C., Gomez, L., Casado, R., and Aragoncillo, C. (1997) Purification and in vitro chaperone activity of a class I small heat-shock protein abundant in recalcitrant chestnut seeds. Plant Physiol. 115, 71-77.
5. Ehrnsperger, M., Gr?ber, S., Gaestel, M., and Buchner, J. (1997) Binding of non-native protein to Hsp25 during heat shock creates a reservoir of folding intermediates for reactivation EMBO J. 16, 221-229.
6. Goldman, A., Engelman, D. M., Steiz, T. A. (1986) Identifying nonpolar transbilayer helices in amino acid sequences of membrane proteins. Annu. Rev. Biophys. Chem. 15, 321-353.
7. H?rndahl, U., Hall, R. B., Osteryoung, K. W., Vierling, E., Bornman, J. F., and Sundby C. (1999) The chloroplast small heat shock protein undergoes oxidation-depent conformational changes and may protect plants from oxidative stress. Cell Stress & Chaperones 4, 129-138.
8. Hartl, F. U. (1996) Molecular chaperones in cellular protein folding. Nature 381: 571-579
9. Haslbeck, H., Walke, S., Stromer, T., Ehrnsperger, M., White, H. E., Chen, S., Saibil, H. R., and Buchner, J. (1999) Hsp26: a temperature-regulated chaperone. EMBO J. 18, 6744-6751.
10. Ijssel, P. R.L.A. van den, Overkamp, K., Knauf, U., Gaestel, M., and Jong, W. W. de. (1994) oA-crystallin confers cellular thermoresistance. FEBS Letters 355, 54-56.
11. Josephy, P. D., Eling, T. and Mason, R. P. (1982) The horseradish peroxidase-catalyzed oxidation of 3,5,3',5'-tetramethylbenzidine. J.B.C. 257, 3669-3675.
12.Knauf, U., Bielka, H., and Gaestel, M. (1992) Over-expression of the small heat-shock protein, hsp25, inhibits growth of Ehrlich ascites tumor cells. FEBS Letters 309, 297-302.
13.Kyte, J., Doolittle, R. E. (1984) Hydrophobic analysis of polypeptides. J. Mol. Bio1 157, 105-132.
14. Laemmli, U. K. (1970) Cleavage of structureal proteins during the assembly of the head of bacteriophage T4. Nature 227, 680-685.
15. Lee, G. J., Pokala, N., and Vierling E. (1995) Structure and in vitro molecular chaperone activity of cytosolic small heat shock protein from pea. J. Biol. Chem. 270, 10432-10438.
16. Mtwisha, L., Brandt, W., McCready, S. and Lindsey, G. G. (1998) HSP 12 is a LEA-like protein in Saccharomyces cerevisiae. Plant Mol. Biol. 37, 513-521.
17.Muchowski, P. J., and Clark, J. I. (1998) ATP-enhanced molecular chaperone functions of the small heat shock protein humam aB crystallin. Biochemistry 95, 1004-1009.
18.Pelah, D., Shoseyov, O., Altman, A. (1995) Characterization of BspA, a major boiling-stable, water-stress-responsive protein in aspen (populus tremula) Tree Physiol. 15, 673-678.
19. Pelah, D., Wang, W. X., Altman, A., Shoseyov, O., Bartels, D. (1997) Differential accumulation of water-stress related proteins, sucrose synthase and soluble sugars in Populus genotypes which differ in their water-stress response. Physiol. Plant. 99, 153-159.
20.Praekel, U.M. and Meacock, P.A. (1990) HSP12, a new small heat shock gene of Saccharomyces cerevisiae: Analysis of structure, regulation and function. Mol.Gen. Genet. 223, 97-106.
21. Rogalla, T., Ehrnsperger, M., Preville, X., Kotlyarov, A., Lutsch, G., Ducasse, C., Paul, C., Wieske, M., Arrigo., A-P., and Buchner, J. (1999) Regulation of Hsp27 oligomerization, chaperone function, and protective activity against oxdative stress/tumor necrosis factor a by phosphrylation. J. Biol.Chem. 274: 27, 18947-18956.
22. Shpigel, E., Goldlust, A., Efroni, G., Avraham, A., Eshel, A., Dekel, M. and Shoseyov, O. (1999) Immobilization of recombinant Heparinase I fused to cellulose-binding domain. Biotech. Bioeng., 65, 17-23.
23. Soto, A., Allona, I., Collada, C., Guevara, M-A., Casado, R., Rodriguez-Cerezo, E., Aragoncillo, C., and Gomez, L. (1999) Heterologous expression of a plant small heat-shock protein enhances Eschrichia coli viability under heat and cold stress. Plant Physiol. 120, 521-528.
24. Suzuki, T.C., Krawitz, D.C. and Vierling, E. (1998) The chloroplast small heat-shock protein oligomer is not phosphorylated and does not dissociate during heat stress in vivo. Plant Physiol. 116, 1151-1161.
25. Veinger, L., Diamant, S., Buchner, J., and Goloubinoff, P. (1998) The small heat-shock protein IbpB from Escherichia coli stabilizes stress-denatured proteins for subsequent refolding by a multichaperone network. J. Biol. Chem. 273, 11032-11037.
26. Waters, E. R., Lee, G. J., and Vierling, E. (1996) Evolution, structure and function of the small heat shock proteins in plants. J. Exper. Bot. 47:(296)325-338
27. Yeh, C-H., Chang, P-F. L., Yeh, K-W., Lin, W-M., Chen, Y-M., and Lin C-Y. (1997) Expression of a gene encoding a 16.9-kDa heat-shock protein, Oshsp 16.9, in Escherichia coli enhances thermotolerance. PNAS 94, 10967-10972.

## Claims

1. A nucleic acid construct comprising a nucleic acid comprising:
(a) a first polynucleotide encoding a boiling stable, detergent stable, or protease resistant protein, said protein having a chaperone-like activity and wherein said stable protein has a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2; and
(b) a second polynucleotide including a promoter sequence being operably linked to said first polynucleotide for directing an expression of said protein.

2. The nucleic acid construct of claim 1, wherein:
(i) said constitutive plant promoter is selected from the group consisting of CaMV35S plant promoter, CaMV19S plant promoter, FMV34S plant promoter, sugarcane bacilliform badnavirus plant promoter, CsVMV plant promoter, Arabidopsis ACT2/ACT8 actin plant promoter, Arabidopsis ubiquitin UBQ1 plant promoter, barley leaf thionin BTH6 plant promoter, and rice actin plant promoter;
(ii) said tissue specific plant promoter is selected from the group consisting of bean phaseolin storage protein plant promoter, DLEC plant promoter, PHS plant promoter, zein storage protein plant promoter, conglutin gamma plant promoter from soybean, AT2S1 gene plant promoter, ACT11 actin plant promoter from Arabidopsis, napA plant promoter from Brassica napus and potato patatin gene plant promoter; and
(iii) said inducible plant promoter is selected from the group consisting of a slight-inducible plant promoter derived from the pea rbcS gene, a plant promoter from the alfalfa rbcS gene, DRE, MYC and MYB plant promoters which are active in drought; INT, INPS, prxEa, Ha hsp17.7G4 and RD21 plant promoters active in high salinity and osmotic stress, and hsr203J and str246C plant promoters active in pathogenic stress.

3. The nucleic acid construct of claim 1, wherein said promoter sequence is a prokaryote promoter.

4. The nucleic acid construct of claim 1, wherein said stable protein is natively an oligomer.

5. The nucleic acid construct of any of claims 1-4, wherein said stable protein has the sequence as set forth in SEQ ID NOs:2.

6. The nucleic acid construct of any of claims 1-4, wherein said stable protein has the sequence as set forth in SEQ ID NO:35.

7. The nucleic acid construct of any of claims 1-4, wherein said first polynucleotide has the sequence as set forth in SEQ ID NOs:1.

8. The nucleic acid construct of any of claims 1-4, wherein said first polynucleotide has the sequence as set forth in SEQ ID NO:34.

9. A cell transformed with the nucleic acid construct of any of claims 1-8.

10. A non-human organism transformed with the nucleic acid construct of any of claims 1-9.

11. The nucleic acid construct of any of claims 1-9, further comprising (c) a third polynucleotide encoding an additional protein, said third polynucleotide being adjacent and in frame to said first polynucleotide, said first and third polynucleotides encoding, in combination, a fusion protein of said stable protein and said additional protein.

12. An antibody which specifically binds to an isolated boiling stable and detergent stable polypeptide, and/or a protease resistant polypeptide, said polypeptide having a chaperone-like activity and having a sequence at least 65% homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

13. A method of stabilizing a protein against denaturing conditions comprising causing an effective amount of a boiling stable and detergent stable polypeptide, and/or a protease resistant polypeptide to become in contact with said protein, said polypeptide having a chaperone-like activity and having a sequence at least 65% homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

14. A fusion protein comprising a boiling stable and detergent stable polypeptide, and/or a protease resistant polypeptide, said polypeptide having a chaperone-like activity and having a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2 fused to an additional polypeptide.

15. The fusion protein of claim 14, wherein said boiling stable and detergent stable and/or protease resistant polypeptide having said chaperone-like activity is fused to said additional polypeptide via a peptide bond.

16. The fusion protein of claim 14 or 15, wherein said boiling stable and detergent stable and/or protease resistant polypeptide having said chaperone-like activity is fused to said additional polypeptide via a cross-linker.

17. The fusion protein of any of claims 14-16, having an oligomeric form,

18. The fusion protein of any of claims 14-17, wherein said stable polypeptide has the sequence as set forth in SEQ ID NOs:2.

19. The fusion protein of any of claims 14-18, wherein said stable polypeptide has the sequence as set forth in SEQ ID NO:35.

20. A pharmaceutical composition comprising the fusion protein of any of claims 14-19 and a pharmaceutically acceptable carrier.

21. A method of protecting an enzyme preparation from reduction in enzymatic activity, the method comprising adding to the enzyme preparation a boiling stable and detergent stable polypeptide, and/or a protease resistant polypeptide having a chaperone-like activity and having a sequence at least 65% homologous to SEQ ID NOs:2 or 35, as determined using the BestFit software of the Wisconsin sequence analysis package, utilizing the Smith and Waterman algorithm, where gap creation penalty equals 8 and gap extension penalty equals 2 in an amount sufficient for protecting the enzyme preparation from reduction in enzymatic activity.

22. A transgenic plant transformed with the nucleic acid construct of any of claims 1-8

23. A method of rendering a plant more tolerant to a biotic or abiotic stress, the method comprising engineering the plant to express the nucleic acid construct of any of claims 1-8

24. Use of a boiling stable and detergent stable polypeptide, and/or a protease resistant polypeptide for the manufacture of a medicament for accelerating wound healing, said polypeptide having a chaperone-like activity and having a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

25. A pharmaceutical composition, comprising, as an active ingredient, a boiling stable and detergent stable polypeptide, and/or a protease resistant polypeptide, said polypeptide having a chaperone-like activity and having a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2 and a pharmaceutically acceptable carrier.

26. The composition of claim 25, wherein said stable polypeptide has the sequence as set forth in SEQ ID NOs:2.

27. The composition of claim 25, wherein said stable polypeptide has the sequence as set forth in SEQ ID NO:35.

28. A method of increasing a binding avidity of a binding molecule, the method comprising displaying multiple copies of the binding molecule on a surface of an oligomer of a boiling stable and detergent stable polypeptide, and/or a protease resistant polypeptide, said polypeptide having a chaperone-like activity and having a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2.

29. The method of claim 28, wherein said binding molecule is selected from the group consisting of a receptor, a ligand, an enzyme, a substrate, an inhibitor, an antibody and an antigen.

30. The method of claim 28 or 29, wherein said stable polypeptide has the sequence as set forth in SEQ ID NOs:2.

31. The method of claims 28-29, wherein said stable polypeptide has the sequence as set forth in SEQ ID NO:35.

32. A hetero complex comprising an oligomer including a plurality of a boiling stable and detergent stable polypeptide, and/or a protease resistant polypeptide, said polypeptide having a chaperone-like activity and having a sequence at least 65 % homologous to SEQ ID NOs:2 or 35, as determined using the BestFit software of the Wisconsin sequence analysis package, utilizing the Smith and Waterman algorithm, where gap creation penalty equals 8 and gap extension penalty equals 2, and at least two different molecules being fused to said oligomer.

33. The composition of claim 32, wherein said stable polypeptide has the sequence as set forth in SEQ ID NOs:2.

34. The composition of claim 32, wherein said stable polypeptide has the sequence as set forth in SEQ ID NO:35.

## Patentansprüche

1. Nucleinsäurekonstrukt, umfassend eine Nucleinsäure, die umfasst:
(a) ein erstes Polynucleotid, das für ein kochstabiles, detergensstabiles oder proteasebeständiges Protein codiert, wobei das Protein eine Chaperon-ähnliche Aktivität aufweist und wobei das stabile Protein eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht; und
(b) ein zweites Polynucleotid, das eine Promotorsequenz enthält, die funktionell an das erste Polynucleotid gebunden ist, um eine Expression des Proteins zu steuern.

2. Nucleinsäurekonstrukt nach Anspruch 1, wobei:
(i) der konstitutive Pflanzenpromotor ausgewählt ist aus der Gruppe bestehend aus dem Pflanzenpromotor CaMV35S, dem Pflanzenpromotor CaMV19S, dem Pflanzenpromotor FMV34S, dem Pflanzenpromotor des stäbchenförmigen Zuckerrohr-Badnavirus, dem Pflanzenpromotor CsVMV, dem ACT2/ACT8-Aktin-Pflanzenpromotor von Arabidopsis, dem Ubiquitin-Pflanzenpromotor UBQ1 von Arabidopsis, dem Pflanzenpromotor BTH6 von Gerstenblatt-Thionin und dem Reis-Aktin-Pflanzenpromotor;
(ii) der gewebespezifische Pflanzenpromotor ausgewählt ist aus der Gruppe bestehend aus dem Pflanzenpromotor des Speicherproteins Phaseolin von Bohne, dem DLEC-Pflanzenpromotor, dem PHS-Pflanzenpromotor, dem Pflanzenpromotor des Speicherproteins Zein, dem Pflanzenpromotor des Conglutin gamma von Sojabohne, dem AT2S1-Gen-Pflanzenpromotor, dem ACT11-Aktin-Pflanzenpromotor von Arabidopsis, dem napA-Pflanzenpromotor von Brassica napus und dem Pflanzenpromotor des Kartoffelpatatin-Gens; und
(iii) der induzierbare Pflanzenpromotor ausgewählt ist aus der Gruppe bestehend aus einem lichtinduzierbarem Pflanzenpromotor, der vom Erbsen-Gen rbcS stammt, einem Pflanzenpromotor vom Alfalfa-Gen rbcS, den Pflanzenpromotoren DRE, MYC und MYB, die bei Trockenheit aktiv sind; den Pflanzenpromotoren INT, INPS, prxEa, Ha hsp17.7G4 und RD21, die bei hohem Salzgehalt und osmotischem Stress aktiv sind, und den Pfanzenpromotoren hsr2Q3J und str246C, die bei pathogenem Stress aktiv sind.

3. Nucleinsäurekonstrukt nach Anspruch 1, wobei es sich bei der Promotorsequenz um einen Prokaryot-Promotor handelt.

4. Nucleinsäurekonstrukt nach Anspruch 1, wobei das stabile Protein nativ ein Oligomer ist.

5. Nucleinsäurekonstrukt nach einem der Ansprüche 1 bis 4, wobei das stabile Protein die Sequenz aufweist, wie in SEQ ID NO:2 dargelegt.

6. Nucleinsäurekonstrukt nach einem der Ansprüche 1 bis 4, wobei das stabile Protein die Sequenz aufweist, wie in SEQ ID NO:35 dargelegt.

7. Nucleinsäurekonstrukt nach einem der Ansprüche 1 bis 4, wobei das erste Polynucleotid die Sequenz aufweist, wie in SEQ ID NO:1 dargelegt.

8. Nucleinsäurekonstrukt nach einem der Ansprüche 1 bis 4, wobei das erste Polynucleotid die Sequenz aufweist, wie in SEQ 10 NO:34 dargelegt.

9. Zelle, die mit dem Nucleinsäurekonstrukt nach einem der Ansprüche 1 bis 8 transformiert ist.

10. Nichthumaner Organismus, der mit dem Nucleinsäurekonstrukt nach einem der Ansprüche 1 bis 9 transformiert ist.

11. Nucleinsäurekonstrukt nach einem der Ansprüche 1 bis 9, ferner umfassend (c) ein drittes Polynucleotid, das für ein zusätzliches Protein codiert, wobei das dritte Polynucleotid benachbart und im Rahmen zum ersten Polynucleotid ist, und die ersten und dritten Polynucleotide zusammen für ein Fusionsprotein des stabilen Proteins und des zusätzlichen Proteins codieren.

12. Antikörper, der spezifisch an ein isoliertes kochstabiles und detergensstabiles Polypeptid und/oder ein proteasebeständiges Polypeptid bindet, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht.

13. Verfahren zum Stabilisieren eines Proteins gegen Denaturierungsbedingungen, umfassend ein Bewirken, dass eine wirksame Menge eines kochstabilen und detergensstabilen Polypeptids und/oder eines proteasebeständigen Polypeptids mit dem Protein in Kontakt tritt, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht.

14. Fusionsprotein, umfassend ein kochstabiles und detergensstabiles Polypeptid und/oder ein proteasebeständiges Polypeptid, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht.

15. Fusionsprotein nach Anspruch 14, wobei das kochstabile und detergensstabile und/oder proteasebeständige Polypeptid mit der Chaperon-ähnlichen Aktivität über eine Peptidbindung zu dem zusätzlichen Polypeptid fusioniert wird.

16. Fusionsprotein nach Anspruch 14 oder 15, wobei das kochstabile und detergensstabile und/oder proteasebeständige Polypeptid mit der Chaperon-ähnlichen Aktivität über einen Vernetzer zu dem zusätzlichen Polypeptid fusioniert wird.

17. Fusionsprotein nach einem der Ansprüche 14 bis 16, aufweisend eine oligomere Form.

18. Fusionsprotein nach einem der Ansprüche 14 bis 17, wobei das stabile Polypeptid die Sequenz aufweist, wie in SEQ ID NO:2 dargelegt.

19. Fusionsprotein nach einem der Ansprüche 14 bis 18, wobei das stabile Polypeptid die Sequenz aufweist, wie in SEQ ID NO:35 dargelegt.

20. Pharmazeutische Zusammensetzung, umfassend das Fusionsprotein nach einem der Ansprüche 14 bis 9 und einen pharmazeutisch unbedenklichen Träger.

21. Verfahren zum Schützen eines Enzympräparats vor einer Reduktion der enzymatischen Aktivität, wobei das Verfahren ein Hinzufügen zu dem Enzympräparat eines kochstabilen und detergensstabilen Polypeptids und/oder ein proteasebeständigen Polypeptids mit einer Chaperon-ähnlichen Aktivität und mit einer Sequenz, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht, in einer Menge umfasst, die zum Schützen des Enzympräparats vor einer Reduktion der enzymatischen Tätigkeit ausreicht.

22. Transgene Pflanze, die mit dem Nucleinsäurekonstrukt nach einem der Ansprüche 1 bis 8 transformiert ist.

23. Verfahren, um eine Pflanze toleranter gegenüber biotischem oder abiotischem Stress zu machen, wobei das Verfahren ein gentechnisches Behandeln der Pflanze umfasst, um das Nucleinsäurekonstrukt nach einem der Ansprüche 1 bis 8 zu exprimieren.

24. Verwendung eines kochstabilen und detergensstabilen Polypeptids und/oder eines proteasebeständiges Polypeptids für die Herstellung eines Medikaments zum Beschleunigen von Wundheilung, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht.

25. Pharmazeutische Zusammensetzung, umfassend als einen aktiven Bestandteil ein kochstabiles und detergensstabiles Polypeptid und/oder ein proteasebeständiges Polypeptid, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht, und einen pharmazeutisch unbedenklichen Träger.

26. Zusammensetzung nach Anspruch 25, wobei das stabile Polypeptid die Sequenz aufweist, wie in SEQ ID NO:2 dargelegt.

27. Zusammensetzung nach Anspruch 25, wobei das stabile Polypeptid die Sequenz aufweist, wie in SEQ ID NO:35 dargelegt.

28. Verfahren zum Erhöhten einer Bindungsaktivität eines Bindemoleküls, wobei das Verfahren ein Anzeigen von mehreren Kopien des Bindemoleküls auf einer Oberfläche eines Oligomers eines kochstabilen und detergensstabilen Polypeptids und/oder eines proteasebeständigen Polypeptids umfasst, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht.

29. Verfahren nach Anspruch 28, wobei das Bindemolekül aus der Gruppe bestehend aus einem Rezeptor, einem Liganden, einem Enyzm, einem Substrat, einem Inhibitor, einem Antikörper und einem Antigen ausgewählt ist.

30. Verfahren nach Anspruch 28 oder 29, wobei das stabile Polypeptid die Sequenz aufweist, wie in SEQ ID NO:2 dargelegt.

31. Verfahren nach Anspruch 28 oder 29, wobei das stabile Polypeptid die Sequenz aufweist, wie in SEQ ID NO:35 dargelegt.

32. Heterokomplex, umfassend ein oligomer, enthaltend eine Mehrzahl eines kochstabilen und detergensstabilen Polypeptids und/oder eines proteasebeständigen Polypeptids, wobei das Polypeptid eine Chaperon-ähnliche Aktivität aufweist und eine Sequenz aufweist, die mindestens zu 65 % homolog zu SEQ ID NOs:2 oder 35 ist, wie unter Verwendung der BestFit-Software des Wisconsin-Sequenzanalysepakets bei Einsatz des Smith-Waterman-Algorithmus bestimmt, wobei der Abzug für Lückenbildung 8 entspricht und der Abzug für Lückenerweiterung 2 entspricht, und mindestens zwei verschiedene Moleküle, die zu dem Oligomer fusioniert sind.

33. Zusammensetzung nach Anspruch 32, wobei das stabile Polypeptid die Sequenz aufweist, wie in SEQ ID NO:2 dargelegt.

34. Zusammensetzung nach Anspruch 32, wobei das stabile Polypeptid die Sequenz aufweist, wie in SEQ ID NO:35 dargelegt.

## Revendications

1. Produit de recombinaison d'acide nucléique comprenant un acide nucléique comprenant :
(a) un premier polynucléotide codant pour une protéine stable à l'ébullition, stable aux détergents, ou résistante aux protéases, ladite protéine ayant une activité de type chaperonne et où ladite protéine stable possède une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2 ; et
(b) un second polynucléotide comprenant une séquence promotrice qui est en liaison fonctionnelle avec ledit premier polynucléotide afin de diriger une expression de ladite protéine.

2. Produit de recombinaison d'acide nucléique selon la revendication 1, dans lequel :
(i) ledit promoteur végétal constitutif est sélectionné dans le groupe consistant en le promoteur végétal CaMV35S, le promoteur végétal CaMV19S, le promoteur végétal FMV34S, le promoteur végétal du badnavirus bacilliforme de la canne à sucre, le promoteur végétal du CsVMV, le promoteur végétal de l'actine ACT2/ACT8 d'Arabidopsis, le promoteur végétal UBQ1 de l'ubiquitine d'Arabidopsis, le promoteur végétal BTH6 de la thionine des feuilles de l'orge, et le promoteur végétal de l'actine du riz ;
(ii) ledit promoteur végétal spécifique à un tissu est sélectionné dans le groupe consistant en le promoteur végétal de la protéine de stockage phaséoline, le promoteur végétal DLEC, le promoteur végétal PHS, le promoteur végétal de la protéine de stockage zéine, le promoteur végétal de la gamma-conglutine du soja, le promoteur végétal du gène AT2S1, le promoteur végétal de l'actine ACT11 d'Arabidopsis, le promoteur végétal napA de Brassica napus et le promoteur végétal du gène de la patatine de la pomme de terre ; et
(iii) ledit promoteur végétal inductible est sélectionné dans le groupe consistant en un promoteur végétal inductible par la lumière dérivé du gène rbcS du pois, un promoteur végétal du gène rbcS de la luzerne, les promoteurs végétaux DRE, MYC et MYB qui sont actifs en cas de sécheresse ; les promoteurs végétaux INT, INPS, prxEa, Ha hsp17.7G4 et RD21 qui sont actifs en cas de forte salinité et de stress osmotique, et les promoteurs végétaux hsr203J et str246C qui sont actifs en cas de stress pathogène.

3. Produit de recombinaison d'acide nucléique selon la revendication 1, dans lequel ladite séquence promotrice est un promoteur procaryote.

4. Produit de recombinaison d'acide nucléique selon la revendication 1, dans lequel ladite protéine stable est, sous forme native, un oligomère.

5. Produit de recombinaison d'acide nucléique selon l'une quelconque des revendications 1 à 4, dans lequel ladite protéine stable possède la séquence telle que décrite dans SEQ ID NO: 2.

6. Produit de recombinaison d'acide nucléique selon l'une quelconque des revendications 1 à 4, dans lequel ladite protéine stable possède la séquence telle que décrite dans SEQ ID NO: 35.

7. Produit de recombinaison d'acide nucléique selon l'une quelconque des revendications 1 à 4, dans lequel ledit premier polynucléotide possède la séquence telle que décrite dans SEQ ID NO: 1.

8. Produit de recombinaison d'acide nucléique selon l'une quelconque des revendications 1 à 4, dans lequel ledit premier polynucléotide possède la séquence telle que décrite dans SEQ ID NO: 34.

9. Cellule transformée avec le produit de recombinaison d'acide nucléique selon l'une quelconque des revendications 1 à 8.

10. Organisme non humain transformé avec le produit de recombinaison d'acide nucléique selon l'une quelconque des revendications 1 à 9.

11. Produit de recombinaison d'acide nucléique selon l'une quelconque des revendications 1 à 9, comprenant en outre (c) un troisième polynucléotide codant pour une protéine supplémentaire, ledit troisième polynucléotide étant en position adjacente et dans le cadre par rapport audit premier polynucléotide, lesdits premier et troisième polynucléotides codant, en combinaison, pour une protéine de fusion de ladite protéine stable et de ladite protéine supplémentaire.

12. Anticorps qui se lie spécifiquement à un polypeptide isolé stable à l'ébullition et stable aux détergents, et/ou à un polypeptide résistant aux protéases, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2.

13. Procédé pour stabiliser une protéine contre des conditions dénaturantes, qui consiste à mettre en contact une quantité efficace d'un polypeptide stable à l'ébullition et stable aux détergents, et/ou d'un polypeptide résistant aux protéases, avec ladite protéine, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2.

14. Protéine de fusion comprenant un polypeptide stable à l'ébullition et stable aux détergents, et/ou un polypeptide résistant aux protéases, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2, fusionné à un polypeptide supplémentaire.

15. Protéine de fusion selon la revendication 14, dans laquelle ledit polypeptide stable à l'ébullition et stable aux détergents et/ou polypeptide résistant aux protéases ayant ladite activité de type chaperonne, est fusionné audit polypeptide supplémentaire via une liaison peptidique.

16. Protéine de fusion selon la revendication 14 ou 15, dans laquelle ledit polypeptide stable à l'ébullition et stable aux détergents et/ou polypeptide résistant aux protéases ayant ladite activité de type chaperonne, est fusionné audit polypeptide supplémentaire via un agent de réticulation.

17. Protéine de fusion selon l'une quelconque des revendications 14 à 16, ayant une forme oligomérique.

18. Protéine de fusion selon l'une quelconque des revendications 14 à 17, dans laquelle ledit polypeptide stable possède la séquence telle que décrite dans SEQ ID NO: 2.

19. Protéine de fusion selon l'une quelconque des revendications 14 à 18, dans laquelle ledit polypeptide stable possède la séquence telle que décrite dans SEQ ID NO: 35.

20. Composition pharmaceutique comprenant la protéine de fusion selon l'une quelconque des revendications 14 à 19, et un véhicule pharmaceutiquement acceptable.

21. Procédé pour protéger une préparation enzymatique contre une réduction d'activité enzymatique, le procédé consistant à ajouter à la préparation enzymatique un polypeptide stable à l'ébullition et stable aux détergents, et/ou un polypeptide résistant aux protéases ayant une activité de type chaperonne et ayant une séquence au moins 65 homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2, en une quantité suffisante pour protéger la préparation enzymatique contre une réduction d'activité enzymatique.

22. Plante transgénique transformée avec le produit de recombinaison d'acide nucléique selon l'une quelconque des revendications 1 à 8.

23. Procédé pour rendre une plante plus tolérante à un stress biotique ou abiotique, le procédé consistant à manipuler la plante pour qu'elle exprime le produit de recombinaison d'acide nucléique selon l'une quelconque des revendications 1 à 8.

24. Utilisation d'un polypeptide stable à l'ébullition et stable aux détergents, et/ou d'un polypeptide résistant aux protéases, pour la fabrication d'un médicament destiné à accélérer la cicatrisation de lésions, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2.

25. Composition pharmaceutique comprenant, en tant qu'ingrédient actif, un polypeptide stable à l'ébullition et stable aux détergents, et/ou un polypeptide résistant aux protéases, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2, et un véhicule pharmaceutiquement acceptable.

26. Composition selon la revendication 25, dans laquelle ledit polypeptide stable possède la séquence telle que décrite dans SEQ ID NO: 2.

27. Composition selon la revendication 25, dans laquelle ledit polypeptide stable possède la séquence telle que décrite dans SEQ ID NO: 35.

28. Procédé pour augmenter l'avidité de liaison d'une molécule de liaison, le procédé consistant à présenter de multiples copies de la molécule de liaison sur la surface d'un oligomère d'un polypeptide stable à l'ébullition et stable aux détergents, et/ou d'un polypeptide résistant aux protéases, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2.

29. Procédé selon la revendication 28, dans lequel ladite molécule de liaison est sélectionnée dans le groupe consistant en un récepteur, un ligand, une enzyme, un substrat, un inhibiteur, un anticorps et un antigène.

30. Procédé selon la revendication 28 ou 29, dans lequel ledit polypeptide stable possède la séquence telle que décrite dans SEQ ID NO: 2.

31. Procédé selon les revendications 28 à 29, dans lequel ledit polypeptide stable possède la séquence telle que décrite dans SEQ ID NO: 35.

32. Hétérocomplexe comprenant un oligomère comprenant une pluralité de polypeptides stables à l'ébullition et stables aux détergents, et/ou polypeptides résistants aux protéases, ledit polypeptide ayant une activité de type chaperonne et ayant une séquence au moins 65 % homologue à SEQ ID NO: 2 ou 35, tel que déterminé en utilisant le logiciel BestFit du package d'analyse de séquences Wisconsin, en utilisant l'algorithme de Smith et Waterman, où la pénalité de création de brèche est de 8 et la pénalité d'extension de brèche est de 2, et au moins deux molécules différentes qui sont fusionnées audit oligomère.

33. Composition selon la revendication 32, dans lequel ledit polypeptide stable possède la séquence telle que décrite dans SEQ ID NO: 2.

34. Composition selon la revendication 32, dans lequel ledit polypeptide stable possède la séquence telle que décrite dans SEQ ID NO: 35.


### SEQUENCE LISTING

<110> Wang, Wangxia
   Pelah, Dan
   Alegrand, Tal
   Shoseyov, Oded
   Altman, Arie
<120> BOILING AND/OR DETERGENT STABLE AND/OR PROTEASE RESISTANT, CHAPERONE-LIKE OLIGOMERIC PROTEINS, POLYNUCLEOTIDES ENCODING SAME AND THEIR USES
<130> 01/22939
<160> 40
<170> Patent In version 3.0
<210> 1
   <211> 567
   <212> DNA
   <213> Populus tremula x Populus tremuloides
<900> 1
<210> 2
   <211> 108
   <212> PRT
   <213> Populus tremula x Populus tremuloides
<400> 2
   Met Ala Thr Arg Thr Pro Lys Leu Val Lys His Thr Leu Leu Thr Arg
   1               5                   10                  15
   Phe Lys Asp Glu Ile Thr Arg Glu Gln Ile Asp Asn Tyr Ile Asn Asp
               20                  25                  30
   Tyr Thr Asn Leu Leu Asp Leu Ile Pro Ser Met Lys Ser Phe Asn Trp
           35                  40                  45
   Gly Thr Asp Leu Gly Met Glu Ser Ala Glu Leu Asn Arg Gly Tyr Thr
       50                  55                  60
   His Ala Phe Glu Ser Thr Phe Glu Ser Lys Ser Gly Leu Gln Glu Tyr
   65                  70                  75                  80
   Leu Asp Ser Ala Ala Leu Ala Ala Phe Ala Glu Gly Phe Leu Pro Thr
                   85                  90                  95
   Leu Ser Gln Arg Leu Val Ile Asp Tyr Phe Leu Tyr
               100                 105
<210> 3
   <211> 28
   <212> DNA
   <213> Artificial
<220>
   <223> synthetic oligonucleotide
<400> 3
   aaaaccatgg caaccagaac tccaaagc 28
<210> 4
   <211> 34
   <212> DNA
   <213> Artificial
<220>
   <223> synthetic oligonucleotide
<400> 4
   aaaaggatcc ttactttatt accatgaaat agcc 34
<210> 5
   <211> 593
   <212> DNA
   <213> Populus tremula x Populus tremuloides
<220>
   <221> misc_feature
   <222> (224)..()
   <223> any nucleotide
<220>
   <221> misc_feature
   <222> (451)..()
   <223> any nucleotide
<220>
   <221> misc_feature
   <222> (566)..()
   <223> any nucleotide
<400> 5
   gtcgaaccca cgcgtccgtc tatagcatcc agcagagaga aagagaagac atggcaacca 60
   gaactccaaa gcttgtgaag cacacattgt tgactcggtt caaggatgag atcacacgag 120
   aacagatcga caactacatt aatgactata ccaatctgct cgatctcatt ccaagcatga 180
   agagtttcaa ttggggcacg gatctgggca tggagtctgc ggantaaacc gaggatacac 240
   tcatgccttt gaatctacat ttgagagcaa gtctggtttg caagagtacc tcgattctgc 300
   tgctcttgct gcatttgcag aagggttttt gcctactttg tcacagcgtc ttgtgataga 360
   ctactttctc tactaaacgc tcaggggtaa cgacttcggc cgggctattt cattggataa 420
   agtaatgtat gttcataaat gctggttttg naccactgaa tgttcgtgtc ttgatttctt 480
   gttgtgtaag tgaagggagt gtgctattcc ttaaaattaa gccttgggtt gagttgtgtt 540
   ttccatcttt tcccggttat tcgggncagg tgtgtttcct cccatttagg cca 593
<210> 6
   <211> 357
   <212> DNA
   <213> Populus tremula x Populus tremuloides
<220>
   <221> misc_feature
   <222> (201).. ()
   <223> any nucleotide
<220>
   <221> misc_feature
   <222> (302)..()
   <223> any nucleotide
<400> 6
   tacatccaca gagagaaaga gaagacatgg caaccagaac tccaaagctt gtgaagcaca     60
   cattgttgac tcggttcaag gatgagatca cacgagaaca gatcgacaac tacattaatg    120
   actataccaa tctgctcgat ctcattccaa gcatgaagag tttcaattgg ggcacggatc    180
   tgggcatgga gtctgcggag ntaaaccgag gatacactca tgcctttgaa tctacatttg    240
   agagcaagtc tggttgcaag agtacctcga ttctgctgct cttgctgcat ttgcagaagg    300
   gnttttgcta ctttgcacag cgcttgtgat agactacttc tctactaaac gctcagg      357
<210> 7
   <211> 98
   <212> PRT
   <213> Triticum aestivum
<220>
   <221> misc_feature
   <222> (50)..(51)
   <223> any amino acid
<400> 7
   Val Val Lys His Leu Val Ile Val Gln Phe Lys Glu Asp Val Thr Pro
   1               5                   10                  15
   Glu Arg Leu Asp Gly Leu Ile Arg Gly Tyr Ala Gly Leu Val Asp Lys
               20                  25                  30
   Val Pro Ser Met Lys Ala Phe His Trp Gly Thr Asp Val Ser Ile Glu
           35                  40                  45
   Asn Xaa Xaa Met His Gln Gly Phe Thr His Val Phe Glu Ser Thr Phe
       50                  55                  60
   Glu Ser Thr Glu Gly Val Lys Glu Tyr Val Tyr His Pro Ala His Val
   65                  70                  75                  80
   Glu Phe Ala Thr Asp Phe Leu Gly Ser Thr Glu Lys Val Leu Ile Ile
                   85                  90                  95
   Asp Phe
<210> 8
   <211> 98
   <212> PRT
   <213> Triticum aestivum
<220>
   <221> misc_feature
   <222> (50)..(51)
   <223> any amino acid
<400> 8
   Val Val Lys His Leu Val Ile Val Gln Phe Lys Glu Asp Val Thr Pro
   1               5                   10                  15
   Glu Arg Leu Asp Gly Leu Ile Arg Gly Tyr Ala Gly Leu Val Asp Lys
               20                  25                  30
   Val Pro Ser Met Lys Ala Phe His Trp Gly Thr Asp Val Ser Ile Glu
           35                  40                  45
   Asn Xaa Xaa Met His Gln Gly Phe Thr His Val Phe Glu Ser Thr Phe
       50                  55                  60
   Glu Ser Thr Glu Gly Val Lys Glu Tyr Val Tyr His Pro Ala His Val
   65                  70                  75                  80
   Glu Phe Ala Thr Asp Phe Leu Gly Ser Thr Glu Lys Val Leu Ile Ile
                   85                  90                  95
   Asp Phe
<210> 9
   <211> 98
   <212> PRT
   <213> Triticum aestivum
<220>
   <221> misc_feature
   <222> (50)..(51)
   <223> any amino acid
<400> 9
   Val Val Lys His Leu Val Ile Val Gln Phe Lys Glu Asp Val Thr Pro
   1               5                   10                  15
   Glu Arg Leu Glu Gly Leu Ile Arg Gly Tyr Ala Gly Leu Val Asp Lys
               20                  25                  30
   Val Pro Ser Met Lys Ala Phe His Trp Gly Thr Asp Val Ser Ile Glu
           35                  40                  45
   Asn Xaa Xaa Met His Gln Gly Phe Thr His Val Phe Glu Ser Thr Phe
       50                  55                  60
   Glu Ser Thr Glu Gly Val Lys Glu Tyr Val Tyr His Pro Ala His Val
   65                  70                  75                  80
   Glu Phe Ala Thr Asp Phe Leu Gly Ser Thr Glu Lys Val Leu Ile Ile
                   85                  90                  95
   Asp Phe
<210> 10
   <211> 84
   <212> PRT
   <213> Zea mays
<220>
   <221> misc_feature
   <222> (50).. (51)
   <223> any amino acid
<400> 10
   Val Val Lys His Ile Leu Leu Ala Ser Phe Lys Glu Glu Val Thr Gln
   1               5                   10                  15
   Glu Arg Leu Asp Glu Leu Ile Arg Gly Tyr Ala Ala Leu Val Gly Val
               20                  25                  30
   Val Pro Ser Met Lys Ala Phe His Trp Gly Thr Asp Val Ser Ile Glu
           35                  40                  45
   Asn Xaa Xaa Met His Gln Gly Phe Thr His Val Phe Glu Ser Thr Phe
       50                  55                  60
   Glu Ser Thr Glu Gly Ile Lys Glu Tyr Ile Glu His Pro Ala His Val
   65                  70                  75                  80
   Glu Phe Ala Lys
<210> 11
   <211> 98
   <212> PRT
   <213> Oryza sativa
<220>
   <221> misc_feature
   <222> (50)..(51)
   <223> any amino acid
<400> 11
   Val Val Lys His Ile Leu Leu Ala Arg Phe Lys Glu Asp Val Ala Pro
   1               5                   10                  15
   Glu Arg Leu Asp Gln Leu Ile Arg Gly Tyr Ala Gly Leu Val Asp Leu
               20                  25                  30
   Val Pro Ser Met Lys Ala Phe His Trp Gly Thr Asp Val Ser Ile Glu
           35                  40                  45
   Asn Xaa Xaa Met His Gln Gly Phe Thr His Val Phe Glu Ser Thr Phe
       50                  55                  60
   Glu Ser Thr Glu Gly Val Lys Glu Tyr Ile Glu His Pro Ala His Val
   65                  70                  75                  80
   Glu Phe Ala Asn Glu Phe Leu Pro Val Leu Glu Lys Thr Leu Ile Ile
                   85                  90                  95
   Asp Tyr
<210> 12
   <211> 98
   <212> PRT
   <213> Triticum aestivum
<220>
   <221> misc_feature
   <222> (21)..()
   <223> any amino acid
<220>
   <221> misc_feature
   <222> (48)..(49)
   <223> any amino acid
<400> 12
   Val Val Lys His Leu Val Leu Ala Arg Phe Lys Glu Glu Ala Thr Pro
   1               5                   10                  15
   Glu Ala Leu Asp Xaa Leu Ile Arg Arg Tyr Ala Gly Leu Val Asp Ala
   20 25 30
   Val Pro Ser Met Lys Ala Phe His Trp Gly Thr Asp Val Thr Val Xaa
   35 40 45
   Xaa Leu Asp Thr His Glu Gly Phe Thr His Val Phe Glu Ser Thr Phe
   50 55 60
   Glu Ser Ala Glu Gly Val Lys Glu Tyr Ile Ala His Pro Ser His Val
   65 70 75 80
   Glu Phe Val Asp Glu Phe Leu Ala Leu Ala Glu Lys Met Leu Ile Val
   85 90 95
   Asp Tyr
<210> 13
   <211> 109
   <212> PRT
   <213> Arabidopsis thaliana
<400> 13
   Met Glu Glu Ala Lys Gly Pro Val Lys His Val Leu Leu Ala Ser Phe
   1               5                   10                  15
   Lys Asp Gly Val Ser Pro Glu Lys Ile Glu Glu Leu Ile Lys Gly Tyr
               20                  25                  30
   Ala Asn Leu Val Asn Leu Ile Glu Pro Met Lys Ala Phe His Trp Gly
           35                  40                  45
   Lys Asp Val Ser Ile Glu Asn Leu His Gln Gly Tyr Thr His Ile Phe
       50                  55                  60
   Glu Ser Thr Phe Glu Ser Lys Glu Ala Val Ala Glu Tyr Ile Ala His
   65                  70                  75                  80
   Pro Ala His Val Glu Phe Ala Thr Ile Phe Leu Gly Ser Leu Asp Lys
                           85                  90                  95
   Val Leu Val Ile Asp Tyr Lys Pro Thr Ser Val Ser Leu
               100                 105
<210> 14
   <211> 47
   <212> PRT
   <213> Arabidopsis thaliana
<900> 14
<210> 15
   <211> 98
   <212> PRT
   <213> Glycine max
<220>
   <221> misc_feature
   <222> (50)..(51)
   <223> any amino acid
<400> 15
   Val Val Lys His Val Leu Leu Ala Lys Phe Lys Asp Asp Val Thr Pro
   1               5                   10                  15
   Glu Arg Ile Glu Glu Leu Ile Lys Asp Tyr Ala Asn Leu Val Asn Leu
               20                  25                  30
   Ile Pro Pro Met Lys Ser Phe His Trp Gly Lys Asp Val Ser Ala Glu
           35                  40                  45
   Asn Xaa Xaa Leu His Gln Gly Phe Thr His Val Phe Glu Ser Thr Phe
       50                  55                  60
   Glu Ser Pro Glu Gly Val Ala Glu Tyr Val Ala His Pro Ala His Val
   65                  70                  75                  80
   Glu Tyr Ala Asn Leu Phe Leu Ser Cys Leu Glu Lys Val Ile Val Ile
                   85                  90                  95
   Asp Tyr
<210> 16
   <211> 98
   <212> PRT
   <213> Lycopersicon esculentum
<220>
   <221> misc_feature
   <222> (50)..(51)
   <223> any amino acid
<400> 16
   Val Val Lys His Ile Leu Leu Ala Lys Phe Lys Asp Gly Ile Pro Pro
   1               5                   10                  15
   Glu Gln Ile Asp Gln Leu Ile Lys Gln Tyr Ala Asn Leu Val Asn Leu
               20                  25                  30
   Val Glu Pro Met Lys Ala Phe Gln Trp Gly Lys Asp Val Ser Ile Glu
           35                  40                  45
   Asn Xaa Xaa Leu His Gln Gly Phe Thr His Val Phe Glu Ser Thr Phe
       50                  55                  60
   Asp Ser Leu Glu Gly Val Ala Glu Tyr Ile Ala His Pro Val His Val
   65                  70                  75                  80
   Glu Tyr Ala Asn Thr Leu Leu Pro Gln Leu Glu Lys Phe Leu Ile Val
                   85                  90                  95
   Asp Tyr
<210> 17
   <211> 93
   <212> PRT
   <213> Glycine max
<220>
   <221> misc_feature
   <222> (49)..(50)
   <223> any amino acid
<400> 17
   His Val Leu Leu Pro Lys Leu Lys Asp Tyr Phe Thr Pro Glu Arg Ile
   1               5                   10                  15
   Glu Leu Met Val Asp Tyr Ala Asn Leu Val Asn Leu Met Pro Arg Met
               20                  25                  30
   Lys Ser Phe His Ser Gly Arg Asp Val Ser Ala Glu Tyr Leu His Leu
   35 40 45
   Xaa Xaa Gly Cys Thr His Val Tyr Glu Ser Thr Phe Asp Ser Pro Gly
   50 55 60
   Val Ala Glu Tyr Val Ala His Ala Ala His Val Glu Tyr Ala Asn Gln
   65 70 75 80
   Asp Leu Ser Cys Leu Glu Lys Val Ile Ala Ile Asp Tyr
   85 90
<210> 18
   <211> 108
   <212> PRT
   <213> Populus tremula x Populus tremuloides
<400> 18
   Met Ala Thr Arg Thr Pro Lys Leu Val Lys His Thr Leu Ala Thr Arg
   1 5 10 15
   Phe Lys Asp Glu Ile Thr Arg Glu Gln Ile Asp Asn Tyr Ile Asn Asp
   20 25 30
   Tyr Thr Asn Leu Leu Asp Leu Ile Pro Ser Met Lys Ser Phe Asn Trp
   35 40 45
   Gly Thr Asp Leu Gly Met Glu Ser Ala Glu Leu Asn Arg Gly Tyr Thr
   50 55 60
   His Ala Phe Glu Ser Thr Phe Glu Ser Lys Ser Gly Leu Gln Glu Tyr
   65 70 75 80
   Leu Asp Ser Ala Ala Leu Ala Ala Phe Ala Glu Gly Phe Leu Pro Thr
   85 90 95
   Leu Ser Gln Arg Leu Val Ile Asp Tyr Phe Leu Tyr
   100 105
<210> 19
   <211> 96
   <212> PRT
   <213> Triticum aestivum
<220>
   <221> misc_feature
   <222> (18)..(20)
   <223> any amino acid
<220>
   <221> misc_feature
   <222> (49) .. ()
   <223> any amino acid
<400> 19
   Lys His Leu Cys Leu Val Arg Phe Lys Glu Gly Val Val Val Glu Asp
   1               5                   10                  15
   Ile Xaa Xaa Xaa Ile Glu Glu Leu Thr Lys Leu Ala Ala Glu Leu Asp
               20                  25                  30
   Thr Val Lys Phe Phe Gly Trp Gly Lys Asp Val Leu Asn Gln Glu Ala
           35                  40                  45
   Xaa Leu Thr Gln Gly Phe Thr His Val Phe Ser Met Ser Phe Ala Ser
       50                  55                  60
   Ala Glu Asp Leu Ala Ala Tyr Met Gly His Glu Lys His Ser Ala Phe
   65                  70                  75                  80
   Ala Ala Thr Phe Met Ala Val Leu Asp Lys Val Val Val Leu Asp Phe
                   85                  90                  95
<210> 20
   <211> 96
   <212> PRT
   <213> Triticum aestivum
<220>
   <221> misc_feature
   <222> (18) .. (20)
   <223> any amino acid
<220>
   <221> misc_feature
   <222> (49) .. ()
   <223> any amino acid
<400> 20
   Lys His Leu Cys Leu Val Arg Phe Lys Glu Gly Val Val Val Glu Asp
   1               5                   10                  15
   Ile Xaa Xaa Xaa Ile Glu Glu Leu Thr Lys Leu Ala Ala Glu Leu Asp
               20                  25                  30
   Thr Val Lys Phe Phe Gly Trp Gly Lys Asp Val Leu Asn Gln Glu Ala
           35                  40                  45
   Xaa Leu Thr Gln Gly Phe Thr His Val Phe Ser Met Ser Phe Ala Ser
       50                  55                  60
   Ala Glu Asp Leu Ala Ala Cys Met Gly His Glu Lys His Ser Ala Phe
   65                  70                  75                  80
   Ala Ala Thr Phe Met Ala Val Leu Asp Lys Val Val Val Leu Asp Phe
                   85                  90                  95
<210> 21
   <211> 96
   <212> PRT
   <213> Triticum aestivum
<220>
   <221> misc_feature
   <222> (18)..(20)
   <223> any amino acid
<220>
   <221> misc_feature
   <222> (49) .. ()
   <223> any amino acid
<400> 21
   Lys His Leu Cys Met Ala Lys Phe Lys Glu Gly Val Val Val Glu Asp
   1 5 10 15
   Ile Xaa Xaa Xaa Ile Gln Glu Leu Thr Lys Leu Ala Ala Glu Leu Asp
   20 25 30
   Thr Val Lys Tyr Phe Gly Trp Gly Lys Asp Val Leu Asn Gln Glu Ala
   35 40 45
   Xaa Leu Thr Gln Gly Phe Thr His Val Phe Val Met Thr Phe Ala Ser
   50 55 60
   Ala Glu Asp Leu Ala Ala Cys Met Gly His Glu Lys His Thr Ala Phe
   65 70 75 80
   Ala Ala Thr Phe Met Ala Ala Leu Asp Lys Val Val Val Met Asp Phe
   85 90 95
<210> 22
   <211> 97
   <212> PRT
   <213> Oryza sativa
<220>
   <221> misc_feature
   <222> (15)..(17)
   <223> any amino acid
<220>
   <221> misc_feature
   <222> (45)..()
   <223> any amino acid
<400> 22
   Val Lys His Leu Cys Leu Val Lys Phe Lys Glu Glu Val Leu Xaa Xaa
   1 5 10 15
   Xaa Val Asp Asp Ile Leu Gln Gly Met Thr Lys Leu Val Ser Glu Met
   20 25 30
   Asp Met Val Lys Ser Phe Glu Trp Gly Lys Asp Val Xaa Leu Asn Gln
   35 40 45
   Glu Met Leu Thr Gln Gly Phe Thr His Val Phe Ser Leu Thr Phe Ala
   50 55 60
   Ser Ser Glu Asp Leu Thr Thr Tyr Met Ser His Glu Arg His Gln Glu
   65 70 75 80
   Phe Ala Gly Thr Phe Met Ala Ala Ile Asp Lys Val Val Val Val Asp
   85 90 95
   Phe
<210> 23
   <211> 104
   <212> PRT
   <213> Sorghum bicolor
<220>
   <221> misc_feature
   <222> (40)..(42)
   <223> any amino acid
<220>
   <221> misc_feature
   <222> (52)..()
   <223> any amino acid
<400> 23
   Arg Arg Pro Thr Met Gly Glu Val Lys His Leu Cys Leu Val Lys Phe
   1 5 10 15
   Lys Glu Gly Val Val Val Glu Asp Val Leu Lys Gly Met Thr Asp Leu
   20 25 30
   Val Ala Gly Met Asp Met Val Xaa Xaa Xaa Lys Ser Phe Glu Trp Gly
   35 40 45
   Gln Asp Val Xaa Leu Asn Gln Glu Met Leu Thr Gln Gly Phe Thr His
   50 55 60
   Val Phe Ser Leu Thr Phe Ala Phe Ala Asp Asp Leu Ala Thr Tyr Met
   65 70 75 80
   Gly His Asp Arg His Ala Ala Phe Ala Ala Thr Phe Met Ala Ala Leu
   85 90 95
   Asp Lys Val Val Val Ile Asp Phe
   100
<210> 24
   <211> 77
   <212> PRT
   <213> Zea mays
<220>
   <221> misc_feature
   <222> (25)..()
   <223> any amino acid
<400> 24
   Glu Ser Thr Phe Glu Ser Thr Glu Gly Ile Lys Glu Tyr Ile Glu His
   1 5 10 15
   Pro Ala His Val Glu Phe Ala Lys Xaa Leu Asn Gln Glu Met Leu Thr
   20 25 30
   Gln Gly Phe Thr His Val Phe Ser Leu Thr Phe Ala Thr Ala Ala Asp
   35 40 45
   Leu Ala Ala Tyr Met Ala His Asp Ser His Thr Ala Phe Ala Ala Thr
   50 55 60
   Phe Met Ala Ala Ile Asp Lys Val Leu Val Val Asp Phe
   65 70 75
<210> 25
   <211> 97
   <212> PRT
   <213> Lycopersicon esculentum
<220>
   <221> misc_feature
   <222> (32)..(34)
   <223> any amino acid
<220>
   <221> misc_feature
   <222> (44)..(45)
   <223> any amino acid
<400> 25
   Lys His Leu Val Leu Val Lys Phe Lys Glu Asp Val Val Val Glu Asp
   1               5                   10                  15
   Ile Leu Lys Glu Leu Glu Lys Leu Val Gln Glu Met Asp Ile Val Xaa
               20                  25                  30
   Xaa Xaa Lys Ser Phe Val Trp Gly Lys Asp Val Xaa Xaa Glu Ser His
           35                  40                  45
   Glu Met Leu Arg Gln Gly Phe Thr His Ala Ile Ile Met Thr Phe Asn
       50                  55                  60
   Ser Lys Glu Asp Tyr Gln Thr Phe Ala Asn His Pro Asn His Val Gly
   65                  70                  75                  80
   Phe Ser Ala Thr Phe Ala Thr Val Ile Asp Lys Ala Val Leu Leu Asp
                   85                  90                  95
   Phe
<210> 26
   <211> 94
   <212> PRT
   <213> Solanum tuberosum
<220>
   <221> misc_feature
   <222> (29)..(31)
   <223> any amino acid
<220>
   <221> misc_feature
   <222> (41)..(42)
   <223> any amino acid
<220>
   <221> misc_feature
   <222> (75)..(76)
   <223> any amino acid
<400> 26
   Leu Leu Val Lys Phe Lys Gln Asp Val Val Glu Glu Asp Val Leu Lys
   1               5                   10                  15
   Gln Ile Glu Gln Leu Val Asn Glu Ile Asp Leu Ile Xaa Xaa Xaa Lys
               20                  25                  30
   Ser Phe Val Trp Gly Lys Asp Thr Xaa Xaa Glu Ser Asn Glu Met Val
           35                  40                  45
   Thr Gln Gly Tyr Thr His Ala Met Ile Met Thr Phe Asn Ser Lys Glu
       50                  55                  60
   Asp Tyr Glu Ala Cys Val Val Lys Glu Val Xaa Xaa Glu Phe Ser Ala
   65                  70                  75                  80
   Ile Phe Val Thr Val Val Glu Lys Ile Leu Val Leu Asn Phe
                   85                  90
<210> 27
   <211> 96
   <212> PRT
   <213> Glycine max
<220>
   <221> misc_feature
   <222> (13)..(15)
   <223> any amino acid
<220>
   <221> misc_feature
   <222> (43)..(44)
   <223> any amino acid
<400> 27
   His Tyr Val Ile Val Lys Phe Lys Asp Gly Val Ala Xaa Xaa Xaa Val
   1               5                   10                  15
   Asp Asp Leu Ile Gln Gly Leu Glu Lys Met Val Phe Gly Ile Asp His
               20                  25                  30
   Val Lys Ser Phe Glu Trp Gly Lys Asp Ile Xaa Xaa Glu Ser His Asp
           35                  40                  45
   Met Leu Arg Gln Gly Phe Thr His Ala Phe Leu Met Thr Phe Asn Gly
       50                  55                  60
   Lys Glu Glu Phe Asn Ala Phe Gln Thr His Pro Asn His Leu Glu Phe
   65                  70                  75                  80
   Ser Gly Val Phe Ser Pro Ala Ile Glu Lys Ile Val Val Leu Asp Phe
                   85                  90                  95
<210> 28
   <211> 96
   <212> PRT
   <213> Glycine max
<220>
   <221> misc_feature
   <222> (13)..(15)
   <223> any amino acid
<220>
   <221> misc_feature
   <222> (43)..(44)
   <223> any amino acid
<400> 28
   His Tyr Val Ile Val Lys Phe Lys Asp Gly Val Ala Xaa Xaa Xaa Val
   1               5                   10                  15
   Asp Glu Leu Ile Gln Gly Leu Glu Lys Met Val Ser Gly Ile Asp His
               20                  25                  30
   Val Lys Ser Phe Glu Trp Gly Lys Asp Ile Xaa Xaa Glu Ser His Asp
           35                  40                  45
   Met Leu Arg Gln Gly Phe Thr His Val Phe Leu Met Ala Phe Asn Gly
       50                  55                  60
   Lys Glu Glu Phe Asn Ala Phe Gln Thr His Pro Asn His Leu Glu Phe
   65                  70                  75                  80
   Thr Gly Val Phe Ser Pro Ala Ile Glu Lys Ile Val Val Leu Asp Phe
                   85                  90                  95
<210> 29
   <211> 97
   <212> PRT
   <213> Glycine max
<220>
   <221> misc_feature
   <222> (14)..(16)
   <223> any amino acid
<220>
   <221> misc_feature
   <222> (44)..(45)
   <223> any amino acid
<400> 29
   Lys His Phe Val Ile Val Lys Phe Lys Glu Gly Val Ala Xaa Xaa Xaa
   1 5 10 15
   Val Asp Glu Leu Thr Lys Gly Met Glu Lys Leu Val Thr Glu Ile Gly
   20 25 30
   Ala Val Lys Ser Phe Glu Trp Gly Gln Asp Ile Xaa Xaa Glu Ser Leu
   35 40 45
   Asp Val Leu Arg Gln Gly Phe Thr His Ala Phe Leu Met Thr Phe Asn
   50 55 60
   Lys Lys Glu Asp Phe Val Ala Phe Gln Ser His Pro Asn His Val Glu
   65 70 75 80
   Phe Ser Thr Lys Phe Ser Ala Ala Ile Glu Asn Ile Val Leu Leu Asp
   85 90 95
   Phe
<210> 30
   <211> 43
   <212> PRT
   <213> Glycine max
<220>
   <221> misc_feature
   <222> (18)..(19)
   <223> any amino acid
<400> 30
   Leu Val Ser Glu Ile His Ala Val Lys Ser Phe Glu Trp Gly Gln Asp
   1 5 10 15
   Ile Xaa Xaa Glu Ser Leu Asp Val Leu Arg Gln Gly Phe Thr His Ala
   20 25 30
   Phe Leu Met Thr Phe Asn Lys Lys Arg Arg Leu
   35 40
<210> 31
   <211> 111
   <212> PRT
   <213> Arabidopsis thaliana
<400> 31
   Met Ala Thr Ser Gly Phe Lys His Leu Val Val Val Lys Phe Lys Glu
   1 5 10 15
   Asp Thr Lys Val Asp Glu Ile Leu Lys Gly Leu Glu Asn Leu Val Ser
   20 25 30
   Gln Ile Asp Thr Val Lys Ser Phe Glu Trp Gly Glu Asp Lys Glu Ser
   35 40 45
   His Asp Met Leu Arg Gln Gly Phe Thr His Ala Phe Ser Met Thr Phe
   50 55 60
   Glu Asn Lys Asp Gly Tyr Val Ala Phe Thr Ser His Pro Leu His Val
   65 70 75 80
   Glu Phe Ser Ala Ala Phe Thr Ala Val Ile Asp Lys Ile Val Leu Leu
   85 90 95
   Asp Phe Pro Val Ala Ala Val Lys Ser Ser Val Val Ala Thr Pro
   100 105 110
<210> 32
   <211> 100
   <212> PRT
   <213> Glycine max
<220>
   <221> misc_feature
   <222> (39)..()
   <223> any amino acid
<220>
   <221> misc_feature
   <222> (55)..(56)
   <223> any amino acid
<400> 32
   Lys Thr Val Glu His Ile Val Leu Phe Lys Val Lys Glu Glu Thr Glu
   1 5 10 15
   Pro Ser Lys Val Ser Asp Met Val Asn Gly Leu Gly Ser Leu Val Ser
   20 25 30
   Leu Asp Pro Val Leu His Xaa Leu Ser Val Gly Pro Leu Leu Arg Asn
           35                  40                  45
   Arg Ser Ser Ala Leu Thr Xaa Xaa Phe Thr His Met Leu His Ser Arg
       50                  55                  60
   Tyr Lys Ser Lys Glu Asp Leu Glu Ala Tyr Ser Ala His Pro Ser His
   65                  70                  75                  80
   Val Ser Val Val Lys Gly Tyr Val Leu Pro Ile Ile Asp Asp Ile Met
                   85                  90                  95
   Ser Val Asp Trp
               100
<210> 33
   <211> 102
   <212> PRT
   <213> Artificial
<220>
   <223> Consensus sequence
<220>
   <221> misc_feature
   <222> (12)..()
   <223> any amino acid
<220>
   <221> misc_feature
   <222> (14)..()
   <223> any amino acid
<220>
   <221> misc_feature
   <222> (17).. (18)
   <223> any amino acid
<220>
   <221> misc_feature
   <222> (20)..()
   <223> any amino acid
<220>
   <221> misc_feature
   <222> (23)..()
   <223> any amino acid
<220>
   <221> misc_feature
   <222> (27)..()
   <223> any amino acid
<220>
   <221> misc_feature
   <222> (30)..(32)
   <223> any amino acid
<220>
   <221> misc_feature
   <222> (34)..()
   <223> any amino acid
<220>
   <221> misc_feature
   <222> (36)..(37)
   <223> any amino acid
<220>
   <221> misc_feature
   <222> (42)..()
   <223> any amino acid
<220>
   <221> misc_feature
   <222> (48)..(49)
   <223> any amino acid
<220>
   <221> misc_feature
   <222> (51)..(54)
   <223> any amino acid
<220>
   <221> misc_feature
   <222> (77)..(78)
   <223> any amino acid
<220>
   <221> misc_feature
   <222> (87)..(88)
   <223> any amino acid
<220>
   <221> misc_feature
   <222> (90)..()
   <223> any amino acid
<220>
   <221> misc_feature
   <222> (92)..(93)
   <223> any amino acid
<400> 33
   Val Lys His Leu Val Leu Val Lys Phe Lys Glu Xaa Val Xaa Pro Glu
   1 5 10 15
   Xaa Xaa Asp Xaa Leu Ile Xaa Gly Tyr Ala Xaa Leu Val Xaa Xaa Xaa
   20 25 30
   Asp Xaa Val Xaa Xaa Met Lys Ser Phe Xaa Trp Gly Lys Asp Val Xaa
   35 40 45
   Xaa Glu Xaa Xaa Xaa Xaa Leu His Gln Gly Phe Thr His Val Phe Glu
   50 55 60
   Ser Thr Phe Glu Ser Lys Glu Gly Val Ala Glu Tyr Xaa Xaa His Pro
   65 70 75 80
   Ala His Val Glu Phe Ala Xaa Xaa Phe Xaa Leu Xaa Xaa Leu Glu Lys
   85 90 95
   Val Leu Val Ile Asp Phe
   100
<210> 34
   <211> 428
   <212> DNA
   <213> Populus deltoides
<400> 34
   atggcaacca gaactccaaa gcttgtgaag cacacattgt tgactcggtt caaggatgag 60
   atcacacgag aacaaatcga caactacatt aatgactata ccaatctgct cgatctcatt 120
   ccaaccatga agagtttcaa ttggggcacg gatttgggca tggagtctgc ggagctaaac 180
   cgaggataca ctcatgcctt tgaatctaca tttgagagca agtcaggttt gcaagagtac 240
   ctcgattctg ctgctcttgc tgcatttgca gaaggatttt tgcctacttt gtcacagcgt 300
   cttgtgatag actactttct ctactaaatg ctcaggagta acgacttcgg ccgggctatt 360
   tcatgggaat aaagtaatgt aatgtgcaat aaatgctggt tttgaaccac tgaatgttcg 420
   tgtcttga 428
<210> 35
   <211> 112
   <212> PRT
   <213> Populus deltoides
<400> 35
   Met Ala Thr Arg Thr Pro Lys Leu Val Lys His Thr Leu Leu Thr Arg
   1               5                   10                  15
   Phe Lys Asp Glu Ile Thr Arg Glu Gln Ile Asp Asn Tyr Ile Asn Asp
               20                  25                  30
   Tyr Thr Asn Leu Leu Asp Leu Ile Pro Thr Met Lys Ser Phe Thr Phe
           35                  40                  45
   Asn Trp Gly Thr Asp Leu Gly Met Glu Ser Ala Glu Leu Asn Arg Gly
       50                  55                  60
   Tyr Thr His Ala Phe Glu Ser Thr Phe Glu Ser Lys Ser Gly Leu Gln
   65                  70                  75                  80
   Glu Tyr Leu Asp Ser Ala Ala Leu Ala Ala Phe Ala Glu Gly Phe Leu
                   85                  90                  95
   Pro Thr Leu Ser Gln Arg Leu Val Ile Asp Phe Thr Tyr Phe Leu Tyr
               100                 105                 110
<210> 36
   <211> 9
   <212> PRT
   <213> Artificial
<220>
   <223> conserved consensus sequences
<400> 36
   His Ala Phe Glu Ser Thr Phe Glu Ser
   1               5
<210> 37
   <211> 3
   <212> PRT
   <213> Artificial
<220>
   <223> conserved consensus sequences
<400> 37
   Val Lys His
   1
<210> 38
   <211> 3
   <212> PRT
   <213> Artificial
<220>
   <223> conserved consensus sequences
<400> 38
   Lys Ser Phe
   1
<210> 39
   <211> 497
   <212> DNA
   <213> Populus tremula x Populus tremuloides
<220>
   <221> misc_feature
   <222> (21)..()
   <223> any nucleotide
<220>
   <221> misc_feature
   <222> (50)..()
   <223> any nucleotide
<220>
   <221> misc-feature
   <222> (75)..()
   <223> any nucleotide
<220>
   <221> misc_feature
   <222> (108)..()
   <223> any nucleotide
<220>
   <221> misc_feature
   <222> (128)..()
   <223> any nucleotide
<220>
   <221> misc_feature
   <222> (147)..()
   <223> any nucleotide
<220>
   <221> misc_feature
   <222> (182)..()
   <223> any nucleotide
<220>
   <221> misc_feature
   <222> (270).. ()
   <223> any nucleotide
<400> 39
   tcagagagag aaagagaaga ntggaacaga actccaaagc ttgtgaagan acattgtgac     60
   tcggtcaagg atgantcaca cagaacagat cgacaataca ttaatganat accaatctgc    120
   tcgatccntc attccaagct gaaganttca ttggggcacg gatctgggct ggagtctgcg    180
   gntaaaccga ggatacactc agcctttgat ctactttgag agcagtctgg tttgcaagag    240
   tactcgatct gctgctcttg ctgcattgcn gaagggtttg cctactttgt cacagcgtct    300
   tgtgatagac tactttctct actaaacgct cagggtaacg acttcggccg ggtattcatg    360
   gataagtatg tatgtccata atgctggttt gaccactgat gtccgtgtct gattctgttg    420
   tgctagtgag ggatgctgct atccttaaaa taagcctggg ttgagtgtgt ttccactttt    480
   cccgataatt cggtcag                                                   497
<210> 40
   <211> 366
   <212> DNA
   <213> Populus tremula x Populus tremuloides
<220>
   <221> misc_feature
   <222> (54)..()
   <223> any nucleotide
<220>
   <221> misc_feature
   <222> (59)..()
   <223> any nucleotide
<220>
   <221> misc_feature
   <222> (63)..()
   <223> any nucleotide
<220>
   <221> misc_feature
   <222> (72)..()
   <223> any nucleotide
<220>
   <221> misc_feature
   <222> (79)..()
   <223> any nucleotide
<220>
   <221> misc_feature
   <222> (83)..()
   <223> any nucleotide
<220>
   <221> misc_feature
   <222> (99)..()
   <223> any nucleotide
<220>
   <221> misc_feature
   <222> (114)..()
   <223> any nucleotide
<220>
   <221> misc_feature
   <222> (117)..()
   <223> any nucleotide
<220>
   <221> misc_feature
   <222> (121)..()
   <223> any nucleotide
<220>
   <221> misc_feature
   <222> (138)..()
   <223> any nucleotide
<220>
   <221> misc_feature
   <222> (141)..()
   <223> any nucleotide
<220>
   <221> misc_feature
   <222> (144)..()
   <223> any nucleotide
<220>
   <221> misc_feature
   <222> (153)..()
   <223> any nucleotide
<220>
   <221> misc_feature
   <222> (178)..()
   <223> any nucleotide
<220>
   <221> misc_feature
   <222> (200)..()
   <223> any nucleotide
<220>
   <221> misc_feature
   <222> (212)..()
   <223> any nucleotide
<220>
   <221> misc_feature
   <222> (221)..()
   <223> any nucleotide
<220>
   <221> misc_feature
   <222> (231)..()
   <223> any nucleotide
<220>
   <221> misc_feature
   <222> (245)..()
   <223> any nucleotide
<220>
   <221> misc_feature
   <222> (252)..()
   <223> any nucleotide
<220>
   <221> misc_feature
   <222> (266)..()
   <223> any nucleotide
<220>
   <221> misc_feature
   <222> (274)..()
   <223> any nucleotide
<220>
   <221> misc_feature
   <222> (281)..()
   <223> any nucleotide
<220>
   <221> misc_feature
   <222> (306)..()
   <223> any nucleotide
<220>
   <221> misc_feature
   <222> (323)..()
   <223> any nucleotide
<400> 40
   aacccagcaa ctcccaaagc ttgtgcaagc tacgacattg ttgcactcgg ttcnaaggna     60
   tgnagcatca cnacgtagna acnagcatcg cacgaactna cattcaatgc actnatnacc    120
   naatctgctc gcatctcnat nccnaagcga tgnaagcagt ttccaattgg ggcgaggnat    180
   ctgggcatgg agtctgcggn taacaccgag gnatacactc natgcctttg naatctcaca    240
   tttgnagcag cnaagtctgg tttgcnaagc agtnacctcg nattctgctg ctcttgctgc    300
   atttgnaaca gggtttttgc ctnactttgt ccacagcgtc ttgtgataga ttactttctc    360
   tactaa                                                               366